(19) Europäisches Patentamt
European Patent Office
Office européen des brevets

(11) **EP 1 397 135 B1**

(12) **EUROPEAN PATENT SPECIFICATION**

(45) Date of publication and mention of the grant of the patent: **06.12.2006 Bulletin 2006/49**

(21) Application number: **02740638.8**

(22) Date of filing: **23.05.2002**

(51) Int Cl.:
***A61K 31/46*** *(2006.01)* ***A61K 9/72*** *(2006.01)*
***A61P 11/06*** *(2006.01)* ***A61P 11/08*** *(2006.01)*
*A61K 31/437* *(2006.01)*

(86) International application number:
**PCT/EP2002/005643**

(87) International publication number:
**WO 2002/096423 (05.12.2002 Gazette 2002/49)**

(54) **COMBINATION OF A PDE4 INHIBITOR AND TIOTROPIUM OR DERIVATIVE THEREOF FOR TREATING OBSTRUCTIVE AIRWAYS**

KOMBINATION EINES PDE4-INHIBITORS MIT TIOTROPIUM ZUR BEHANDLUNG OBSTRUKTIVER ATEMWEGSERKRANKUNGEN

COMBINAISON D'UN INHIBITEUR DE PDE4 ET DE TIOTROPIUM OU D'UN DERIVE DE CE DERNIER DESTINEE AU TRAITEMENT DES MALADIES OBSTRUCTIVES DES VOIES AERIENNES ET D'AUTRES MALADIES INFLAMMATOIRES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.05.2001 US 293555 P**
**09.07.2001 US 303845 P**

(43) Date of publication of application:
**17.03.2004 Bulletin 2004/12**

(73) Proprietor: **Boehringer Ingelheim Pharma GmbH & Co.KG**
**55216 Ingelheim am Rhein (DE)**

(72) Inventors:
- **YEADON, Michael**
  **SANDWICH, Kent CT13 9NJ (GB)**
- **ARMSTRONG, Roisin, A.**
  **Mystic, Connecticut 06355 (US)**
- **WATSON, John, W.**
  **Waterford, Connecticut 06385 (US)**

(56) References cited:
**WO-A-96/39408** **WO-A-02/069945**

- **BARNES P J: "NOVEL APPROACHES AND TARGETS FOR TREATMENT OF CHRONIC OBSTRUCTIVE PULMONARY DISEASE" AMERICAN JOURNAL OF RESPIRATORY AND CRITICAL CARE MEDICINE, AMERICAN LUNG ASSOCIATION, NEW YORK, NY, US, vol. 160, no. 5, PART 2, November 1999 (1999-11), pages S72-S79, XP001098482 ISSN: 1073-449X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a combination of therapeutic agents useful in the treatment of obstructive airways and other inflammatory diseases comprising (I) a PDEIV inhibitor selected from a specific group of compounds that is therapeutically effective in the treatment of said diseases when administered by inhalation; together with (II) an anti-cholinergic agent comprising a member selected from the group consisting of tiotropium and derivatives thereof that is therapeutically effective in the treatment of said diseases when administered by inhalation.

The present invention further relates to the use of said combination for the manufacture of a medicament for the treatment of said obstructive airways and other inflammatory diseases comprising administering to said mammal by inhalation a therapeutically effective amount of said combination of therapeutic agents; and a pharmaceutical composition comprising a pharmaceutically acceptable carrier together with said combination of therapeutic agents; and a package containing a pharmaceutical composition for insertion into a device capable of simultaneous or sequential delivery of said pharmaceutical composition in the form of an aerosol or dry powder dispersion to said mammal, where said device is a metered dose inhaler or a dry powder inhaler. It is preferred that said anti-cholinergic agent component be tiotropium bromide.

## Background of the invention

The present invention is concerned with novel combinations of different classes of therapeutic agents that together are useful in the treatment of obstructive airways and other inflammatory diseases. Of particular importance as an object of these treatment combinations are the obstructive airways diseases asthma, chronic obstructive pulmonary disease (COPD) and other obstructive airways diseases exacerbated by heightened bronchial reflexes, inflammation, bronchial hyper-reactivity and bronchospasm, especially COPD.

Compounds for the treatment of COPD are known in the art. P.J. Barnes (Am. Respir.Crit Care Med 160, ppS72-S79, 1999) describes several classes of drugs that possess utility in the treatment of COPD. Among bronchodilators like tiotropium bromide Barnes discloses also antiinflammatory treatments as for instance PDE4 inhibitors to be useful in the treatment of COPD. However, Barnes fails to disclose that an improved therapy of COPD could be reached by combining an anticholinergic agent like tiotropium bromide with a PDE4 inhibitor.

In particular, the combinations of compounds of the present invention are useful in the treatment of respiratory diseases and conditions comprising: asthma, acute respiratory distress syndrome, chronic pulmonary inflammatory disease,bronchitis, chronic bronchitis, chronic obstructive pulmonary (airway) disease, and silicosis; or immune diseases and conditions comprising: allergic rhinitis and chronic sinusitis.

The novel combinations of therapeutic agents with which the present invention is concerned and which are used for the treatment of obstructive airways and other inflammatory diseases, especially asthma, COPD, and other obstructive airways diseases exacerbated by bronchial hyper-reactivity and bronchospasm, comprise the following: *(I)* a PDE4 inhibitor; together with *(II)* an anti-cholinergic agent comprising a member selected from the group consisting of tiotropium and derivatives thereof, especially tiotropium bromide.

## PDE4 Isozyme Inhibitors

The class of PDE4 isozyme inhibitors useful in the novel combinations of therapeutic agents of the present invention comprises compounds which are efficient substrates for, *i.e.,* exhibit an acceptably high level of binding to, the PDE4 isozyme.

The 3',5'-cyclic nucleotide phosphodiesterases (PDEs) comprise a large class of enzymes divided into at least eleven different families which are structurally, biochemically and pharmacologically distinct from one another. The enzymes within each family are commonly referred to as isoenzymes, or isozymes. A total of more than fifteen gene products is included within this class, and further diversity results from differential splicing and post-translational processing of those gene products. The present invention is primarily concerned with the four gene products of the fourth family of PDEs, *i.e.,* PDE4A, PDE4B, PDE4C, and PDE4D. These enzymes are collectively referred to as being isoforms or subtypes of the PDE4 isozyme family. Further below will be found a more detailed discussion of the genomic organization, molecular structure and enzymatic activity, differential splicing, transcriptional regulation and phosphorylation, distribution and expression, and selective inhibition of the PDE4 isozyme subtypes.

PDE4s are characterized by selective, high affinity hydrolytic degradation of the second messenger cyclic nucleotide, adenosine 3',5'-cyclic monophosphate (cAMP), and by sensitivity to inhibition by rolipram. A number of selective inhibitors of the PDE4s have been discovered in recent years, and beneficial pharmacological effects resulting from that inhibition have been shown in a variety of disease models. See, *e.g.,* Torphy *et al., Environ. Health Perspect.* **102** Suppl. 10, 79-84, 1994; Duplantier *et al., J. Med. Chem.* **39** 120-125, 1996; Schneider *et al., Pharmacol. Biochem. Behav.* **50** 211-217, 1995; Banner and Page, *Br. J. Pharmacol.* **114** 93-98, 1995; Barnette *et al., J. Pharmacol. Exp.*

*Ther.* **273** 674-679, 1995; Wright *et al.* "Differential *in vivo* and *in vitro* bronchorelaxant activities of CP-80633, a selective phosphodiesterase 4 inhibitor," *Can. J. Physiol. Pharmacol.* **75** 1001-1008, 1997; Manabe *et al.* "Anti-inflammatory and bronchodilator properties of KF19514, a phosphodiesterase 4 and 1 inhibitor," *Eur. J. Pharmacol.* **332** 97-107, 1997; and Ukita *et al.* "Novel, potent, and selective phosphodiesterase-4 inhibitors as antiasthmatic agents: synthesis and biological activities of a series of 1-pyridylnaphthalene derivatives," *J. Med. Chem.* **42** 1088-1099, 1999. Accordingly, there continues to be considerable interest in the art in the discovery of further and more selective inhibitors of PDE4s.

The present invention is also concerned with the use of selective PDE4 inhibitors in combination with anticholinergic agents for the improved therapeutic treatment of a number of inflammatory, respiratory and allergic diseases and conditions, but especially for the treatment of asthma; chronic obstructive pulmonary disease, including chronic bronchitis, emphysema, and bronchiectasis; chronic rhinitis; and chronic sinusitis. Heretofore in the art, however, the first-line therapy for treatment of asthma and other obstructive airway diseases has been the nonselective PDE inhibitor theophylline, as well as IBMX and pentoxifylline, which may be represented by Formulas (0.1.1), (0.1.2), and (0.1.3), respectively:

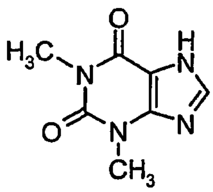

Theophylline
(0.1.1)

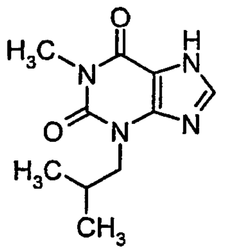

IBMX
(0.1.2)

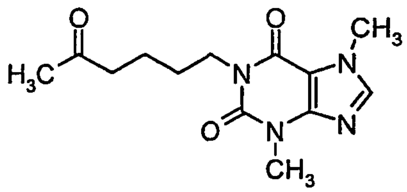

Pentoxifylline
(0.1.3)

Theophylline, which has the PDEs as one of its biochemical targets, in addition to its well characterized bronchodilatory activity, affects the vasculature of patients with increased pulmonary artery pressure, suppresses inflammatory cell responses, and induces apoptosis of eosinophils. Theophylline's adverse events, most commonly cardiac dysrhythmias and nausea, are also mediated by PDE inhibition, however, leading to the search for more selective inhibitors of PDEs that are able to suppress both immune cell functions *in vitro* and allergic pulmonary inflammation *in vivo,* while at the same time having improved side-effect profiles. Within the airways of patients suffering from asthma and other obstructive airway diseases, PDE4 is the most important of the PDE isozymes as a target for drug discovery because of its distribution in airway smooth muscle and inflammatory cells. Several "second-generation" PDE4 inhibitors introduced to the art thus far have been designed to have an improved therapeutic index as compared to the cardiovascular, gastrointestinal, and central nervous system side effects of the above-mentioned nonselective xanthines.

Airflow obstruction and airway inflammation are features of asthma as well as COPD. While bronchial asthma is predominantly characterized by an eosinophilic inflammation, neutrophils appear to play a major role in the pathogenesis of COPD. Thus, PDEs that are involved in smooth muscle relaxation and are also found in eosinophils as well as neutrophils probably constitute an essential element of the progress of both diseases. The PDEs involved include PDE3s as well as PDE4s, and bronchodilating inhibitors have been discovered which are selective PDE3 inhibitors and dual PDE3/4 selective inhibitors. Examples of these are milrinone, a selective PDE3 inhibitor, and benafentrine and zardaverine, both dual PDE3/4 selective inhibitors, which may be represented by Formulas (0.1.4), (0.1.5), and (0.1.6), respectively:

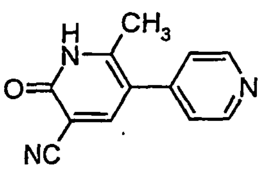

Milrinone
(0.1.4)

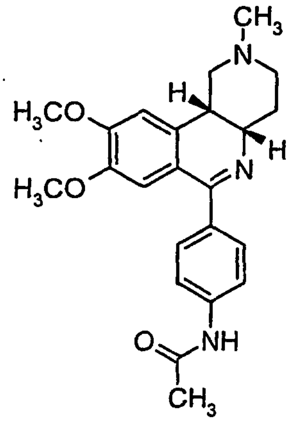

Benafentrine
(0.1.5)

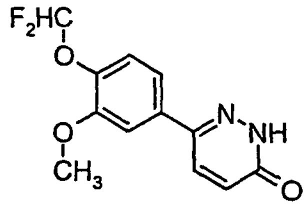

Zardaverine
(0.1.6)

However, benafentrine results in bronchodilation only when administered by inhalation, and zardaverine produces only a modest and short-lived bronchodilation. Milrinone, a cardiotonic agent, induces short-lived bronchodilation and a slight degree of protection against induced bronchoconstriction, but has marked adverse events, *e.g.*, tachycardia and hypotension. Unsatisfactory results have also been obtained with a weakly selective PDE4 inhibitor, tibenelast, and a selective PDE5 inhibitor, zaprinast, which may be represented by Formulas (0.1.7) and (0.1.8):

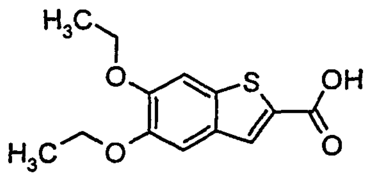

Tibenelast
(0.1.7)

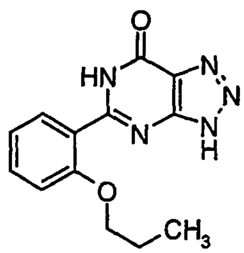

Zaprinast
(0.1.8)

More relative success has been obtained in the art with the discovery and development of selective PDE4 inhibitors.

*In vivo,* PDE4 inhibitors reduce the influx of eosinophils to the lungs of allergen-challenged animals while also reducing the bronchoconstriction and elevated bronchial responsiveness occurring after allergen challenge. PDE4 inhibitors also suppress the activity of immune cells, including $CD4^+$ T-lymphocytes, monocytes, mast cells, and basophils; reduce pulmonary edema; inhibit excitatory nonadrenergic noncholinergic neurotransmission (eNANC); potentiate inhibitory nonadrenergic noncholinergic neurotransmission (iNANC); reduce airway smooth muscle mitogenesis; and induce bronchodilation. PDE4 inhibitors also suppress the activity of a number of inflammatory cells associated with the pathophysiology of COPD, including monocytes/macrophages, $CD8^+$ T-lymphocytes, and neutrophils. PDE4 inhibitors also reduce vascular smooth muscle mitogenesis and, and potentially interfere with the ability of airway epithelial cells to generate pro-inflammatory mediators. Through the release of neutral proteases and acid hydrolases from their granules, and the generation of reactive oxygen species, neutrophils contribute to the tissue destruction associated with chronic inflammation, and are further implicated in the pathology of conditions such as emphysema.

Selective PDE4 inhibitors which have been discovered thus far that provide therapeutic advantages include SB-207,499, identified as ARIFLO®, which may be represented by Formula (0.1.9):

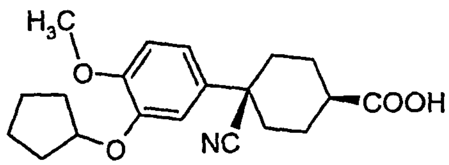

SB-207,499 (0.1.9)

SB-207,499, administered orally at dosages of 5, 10, and 15 mg *b.i.d.,* has produced significant increases in trough $FEV_1$ (forced expiratory volume in 1 second) from placebo at week 2 of a study involving a large number of patients. Another potent, selective PDE4 inhibitor, CDP840, has shown suppression of late reactions to inhaled allergen after 9.5 days of oral administration at doses of 15 and 30 mg in a group of patients with bronchial asthma. CDP840 may be represented by Formula (0.1.9):

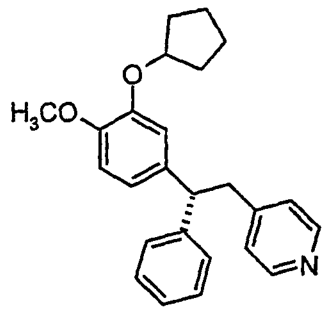

CDP840 (0.1.9)

PDEs have also been investigated as potential therapy for obstructive lung disease, including COPD. In a large study of SB-207,499 in patients with COPD, the group of patients receiving 15 mg *b.i.d.* has experienced a progressive improvement in trough $FEV_1$, reaching a maximum mean difference compared with placebo of 160 mL at week 6, which represents an 11 % improvement. See Compton *et al.,* "The efficacy of Ariflo (SB207499), a second generation, oral PDE4 inhibitor, in patients with COPD," *Am. J. Respir. Crit. Care Med.* **159**, 1999. Patients with severe COPD have been observed to have pulmonary hypertension, and decreases in mean pulmonary artery pressure under clinical conditions have been achieved by oral administration of the selective PDE3 inhibitors milrinone and enoximone. Enoximone has also been shown to reduce airway resistance in patients hospitalized with decompensated COPD. See Leeman *et al., Chest* **91** 662-6, 1987. Using selective PDE3 inhibition by motapizone and selective PDE5 inhibition by zaprinast, it has been shown that combined inhibition of PDE 3 and 5 exerts a relaxation of pulmonary artery rings which corresponds broadly to the pattern of PDE isozymes found in the pulmonary artery smooth muscle. See Rabe *et al., Am. J. Physiol.* **266** (LCMP 10): L536-L543, 1994. The structures of milrinone and zaprinast are shown above as Formulas (0.1.4) and (0.1.8), respectively. The structures of enoximone and motapizone may be represented by Formulas (0.1.10) and (0.1.11), respectively:

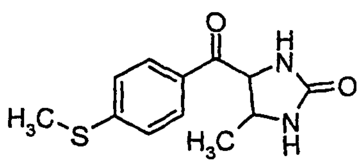

Enoximone
(0.1.10)

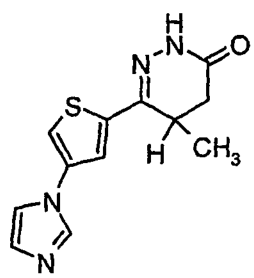

Motapizone (0.1.11)

The effects of PDE4 inhibitors on various inflammatory cell responses can be used as a basis for profiling and selecting inhibitors for further study. These effects include elevation of cAMP and inhibition of superoxide production,

degranulation, chemotaxis, and tumor necrosis factor alpha (TNFα) release in eosinophils, neutrophils and monocytes. PDE4 inhibitors may induce emesis, *i.e.,* nausea and vomiting, which, as expected, is an adverse effect. The emesis adverse effect became apparent when PDE4 inhibitors were first investigated for CNS indications such as depression, when rolipram and denbufylline were used in clinical trials. Rolipram and denbufylline may be represented by Formulas (0.1.12) and (0.1.13), respectively:

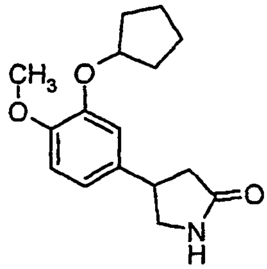

Rolipram
(0.1.12)

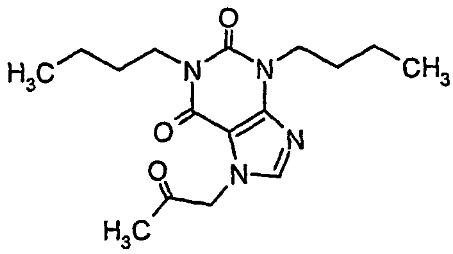

Denbufylline

(0.1.13)

The mechanism(s) by which PDE4 inhibitors may potentially induce emesis is/are uncertain, but a study of the PDE4 inhibitor Ro-20-1724 suggests that nausea and vomiting are at least partially mediated by the emesis centers in the brain. Gastrointestinal adverse events may be caused by local effects, *e.g.*, rolipram is a very potent stimulator of acid secretion from gastric parietal cells, and the resulting excess acid, by producing local irritation, may exacerbate gastrointestinal disturbances. Ro-20-1724 may be represented by Formula (0.1.14):

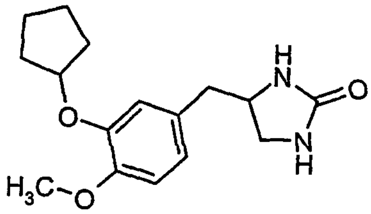

Ro-20-1724 (0.1.14)

Efforts to minimize or eliminate the above-mentioned adverse events sometimes associated with PDE4 inhibitors have included creating inhibitors which do not penetrate the central nervous system, and administering PDE4 inhibitors by inhalation, as in this invention, rather than orally.

With regard to the PDE4 subtypes, A, B, C, and D, it has been found that PDE4C is usually less sensitive to all inhibitors; whereas, with respect to the subtypes A, B, and D, there is as yet no clear evidence of inhibitor specificity, which is defined as a 10-fold difference in $IC_{50}$ values. While most inhibitors, especially RS-25,344, are more potent against PDE4D, this does not amount to selectivity. RS-25,344 may be represented by Formula (0.1.15):

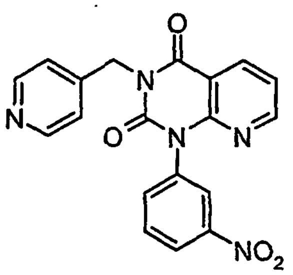

RS-25,344 (0.1.15)

On the other hand, there is a stereoselective effect on the elevation of cAMP in a range of cell types, which has been demonstrated with the results of an investigation of CDP840, shown above as Formula (0.1.9), and its less active enantiomer CT-1731, which is represented by Formula (0.1.16):

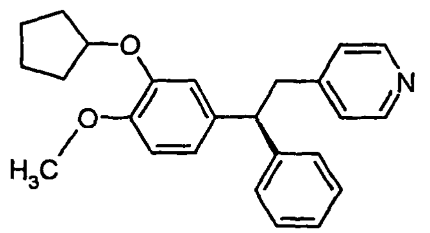

CT-1731 (0.1.16)

It has been known for some time that rolipram had the ability to interact with a high-affinity binding site on brain membranes, and it was later established in the art that this high-affinity rolipram binding site ($S_r$), which is distinct from the catalytic site ($S_c$), exists in a truncated recombinant PDE4A and a full-length recombinant PDE4B. More recently, $S_r$ has been identified on all four PDE4 subtypes. See Hughes *et al., Drug Discovery Today* **2**(3) 89-101, 1997. The presence of $S_r$ appears to have a profound effect on the ability of certain inhibitors such as rolipram and RS-25,344 to inhibit the catalytic activity of PDE4 isozymes.

The impact of residues on inhibitor binding is also significant. A single amino acid substitution (alanine for aspartate) in the catalytic region of PDE4B has been shown to be critical for inhibition by rolipram, and this appears to be a class effect because related inhibitors RP-73,401 and Ro-20-1724 also lose potency on the mutant enzyme. However, the role of binding of inhibitors to the $S_c$ or to the $S_r$, in terms of elevation of cAMP and inhibition of cell responses, is not fully understood at the present time.

RP-73,401, in guinea-pig studies, has been found to be active in (1) the inhibition of antigen-induced lung eosinophilia and eosinophil peroxidase (EPO), Banner, K.H., "The effect of selective phosphodiesterase inhibitors in comparison with other antiasthma drugs on allergen-induced eosinophilia in guinea-pig airways," *Pulm. Pharmacol.* **8** 37-42, 1995; (2) antigen-induced bronchoalveolar lavage (BAL) eosinophilia, Raeburn *et al.,* "Anti-inflammatory and bronchodilator properties of RP73401, a novel and selective phosphodiesterase Type IV inhibitor," *Br. J. Pharmacol.* **113** 1423-1431, 1994; (3) antigen-induced airway eosinophilia and platelet activating factor- (PAF)- and ozone-induced airway hyper-responsiveness (AHR), Karlsson *et al.,* "Anti-inflammatory effects of the novel phosphodiesterase IV inhibitor RP73401," *Int. Arch. Allergy Immunol.* **107** 425-426, 1995; and (4) IL-5 induced pleural eosinophila. Development of RP-73,401, piclamilast, has been discontinued. Piclamilast may be represented by Formula (0.1.17):

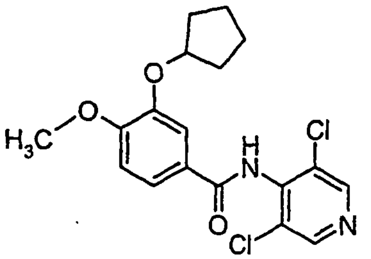

Piclamilast (RP-73,401) (0.1.17)

A related series of compounds is represented by RPR-132294 and RPR-132703, which have been demonstrated in rat studies to have activity in the inhibition of antigen-induced bronchospasm; Escott *et al.,* "Pharmacological profiling of phosphodiesterase 4 (PDE4) inhibitors and analysis of the therapeutic ratio in rats and dogs," *Br. J. Pharmacol.* **123**(Proc. Suppl.) 40P, 1998; and Thurairatnam *et al.,* "Biological activity and side effect profile of RPR-132294 and RPR-132703 - novel PDE4 inhibitors," *XV$^{th}$ EFMC Int. Symp. Med. Chem.,* 1998. The structure of RPR-132294 may be represented by Formula (0.1.18):

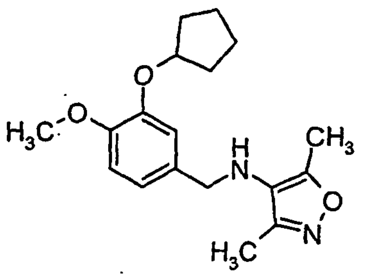

RPR-132294

(0.1.18)

Another compound whose development has been discontinued is WAY-PDA-641, filaminast, which in studies in the dog, has been found to be active in the inhibition of seratonin-induced bronchoconstriction. Filaminast may be represented by Formula (0.1.19):

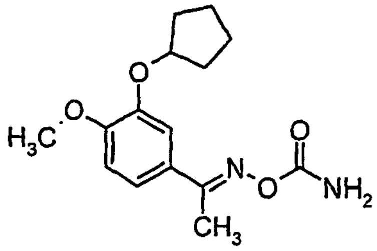

Filaminast (WAY-PDA-641)

(0.1.19)

It has been suggested in the art that PDE4 inhibitors that have a high affinity at the $S_r$ can be correlated with emesis and increased gastric acid secretion. RS-23,544, RP-73,401, and CP-80,633 elicit emesis and have a high affinity at the $S_r$. CDP840 and SB-207,499 have a comparatively low affinity at the $S_r$, but CDP840 has a significantly higher potency at the $S_c$ than does SB-207,499. CDP840 has been demonstrated to provide significant inhibition of late-phase response in the treatment of asthma without any adverse events of nausea or headache. Another PDE4 inhibitor that has been shown to have adverse events of nausea and vomiting is BRL-61,063, also referred to as cipamfylline, which is described further below. The development of CDP840 has been discontinued, while CP-80,633, atizoram, continues in development. CP-80,633 and BRL-61,063 may be represented by Formulas (0.1.20) and (0.1.12), respectively:

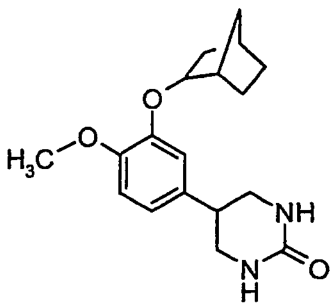

Atizoram (CP-80,633)
(0.1.20)

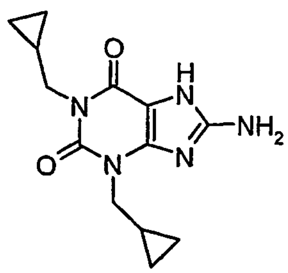

Cipamfylline (BRL-61,063)

(0.1.12)

Another compound which is in development is LAS-31025, arofylline, which in guinea-pig studies, has been found to be active in the inhibition of antigen-induced bronchoconstriction; Beleta, B. J., "Characterization of LAS31025: a new selective PDE IV inhibitor for bronchial asthma," *Third Int. Conf. On Cyclic Nucleotide Phosphodiesterase: From Genes to Therapies,* Glasgow, UK, 1996, Abstract 73. LAS-31025, arofylline, may be represented by Formula (0.1.21):

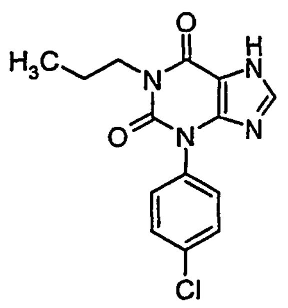

Arofylline (LAS-31025)

(0.1.21)

A number of PDE4 inhibitors have been advanced in development. For example, the effects of V-11294A on LPS-stimulated *ex vivo* TNF release and PHA induced lymphocyte proliferation have been determined in a randomized, double-blind placebo-controlled study which has found that an oral dose of 300 mg is effective in reducing TNF levels and lymphocyte proliferation; Landells *et al.,* "Oral administration of the phosphodiesterase (PDE) 4 inhibitor, V11294A inhibits ex-vivo agonist-induced cell activation," *Eur. Resp. J.* **12**(Suppl. 28) 362s, 1998; and Gale *et al.,* "Pharmacodynamic-pharmacokinetic (PD/PK) profile of the phosphodiesterase (PDE) 4 inhibitor, V11294A, in human volunteers," *Am. J. Respir. Crit. Care Med.* **159** A611, 1999.

The compound D4418 has been administered to healthy volunteers in a single escalating dose, randomized, placebo-controlled Phase I study; Montana *et al.,* "Activity of D4418, a novel phosphodiesterase 4 (PDE4) inhibitor, effects in cellular and animal models of asthma and early clinical studies," *Am. J. Respir. Crit. Care Med.* **159** A108, 1999. D4418 is a moderately potent PDE4 inhibitor with an $IC_{50}$ of 200 nM. It has good oral absorption; a 200 mg dose provides a plasma $C_{max}$ of 1.4 μg/ml. D4418 has been discontinued from development due to its moderate potency, and has been replaced by the preclinical development candidate D4396.

V-11294A and D4418 may be represented by Formulas (0.1.22) and (0.1.23), respectively:

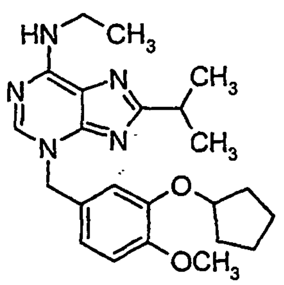

V-11294A

(0.1.22)

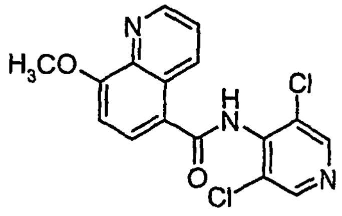

D4418

(0.1.23)

Another compound, CI-1018, has been evaluated in 54 subjects and no adverse events were reported at doses up to 400 mg; Pruniaux *et al.,* "The novel phosphodiesterase inhibitor CI-1018 inhibits antigen-induced lung eosinophilia in sensitized brown-norway rats - comparison with rolipram," *Inflammation* S-04-6, 1999. CI-1018 has been demonstrated to have good oral bioavailability (57% in the rat) and good oral potency of with an $ED_{50}$ of 5mg/kg in that same species. CI-1018 is a relatively weak PDE4 inhibitor with an $IC_{50}$ of 1.1μM in U937 cells. CI-1018 has also been identified as, or associated with as closely related in structure to, PD-168787, which in rat studies has been demonstrated to have activity in the inhibition of antigen-induced eosinophilia; Pascal *et al.,* "Synthesis and structure-activity relationships of 4-oxo-1-phenyl-3,4,6,7-tetrahydro-[1,4]-diazepino[6,7,1-hi] indolines: novel PDE4 inhibitors," *215$^{th}$ ACS,* Dallas, USA, MEDI 50, 1998. Inferred structures for CI-1018 and PD-168787 are represented by Formulas (0.1.24) and (0.1.25), respectively:

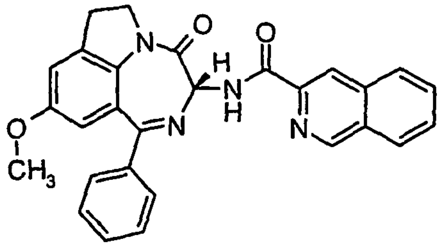

CI-1018

(0.1.24)

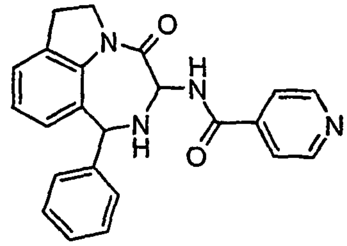

PD-168787

(0.1.25)

The above-mentioned compounds have also been evaluated in animal models which demonstrate their PDE4 inhibition activity. For example, V-11294A, in guinea-pig studies, has been found to be active in the inhibition of antigen-induced bronchoconstriction; Cavalla *et al.,* "Activity of V11294A, a novel phosphodiesterase 4 (PDE4) inhibitor, in cellular and animal models of asthma," *Amer. J. Respir. Crit. Care Med,* **155** A660, 1997. D4418, in guinea-pig studies, has been found to be active in the inhibition of antigen-induced early and late phase bronchoconstriction and BAL eosinophilia; Montana, *et al., Ibid.* CI-1018, in rat studies, has been found to be active in the inhibition of antigen-induced eosinophilia; Burnouf, *et al.,* "Pharmacology of the novel phosphodiesterase Type 4 inhibitor, CI-1018," *215th ACS Nat. Meeting,* MEDI 008, 1998.

Other compounds which have been advanced in development include CDC-3052; D-22888, YM-58997, and roflumilast, which may be represented by Formulas (0.1.27), (0.1.28), (0.1.29), and (0.1.30), respectively:

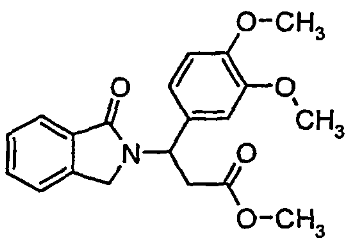

CDC-3052
(0.1.27)

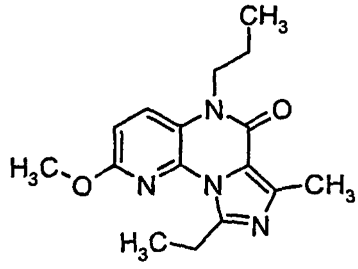

D-22888

(0.1.28)

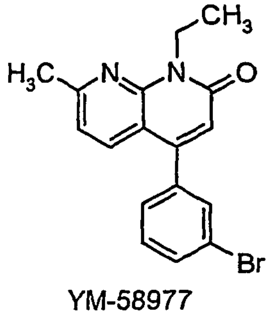

YM-58977
(0.1.29)

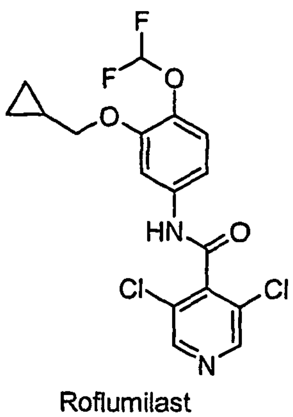

Roflumilast

(0.1.30)

CDC-3052 has been discontinued from development, but has been succeeded by very potent inhibitors of PDE4 such as the compound represented by Formula (0.1.31), and by the anti-inflammatory compound CDC-801 represented by Formula (0.1.32), respectively:

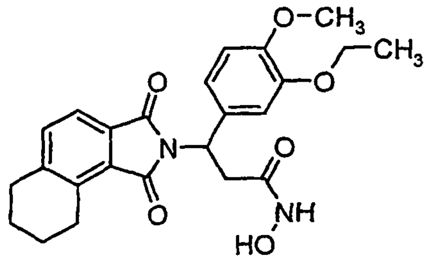

(0.1.31)

CDC-801

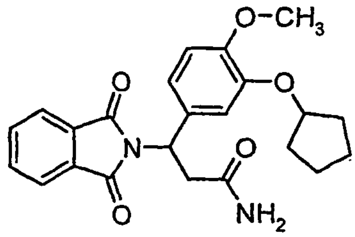

(0.1.32)

The compound of Formula (0.1.32) is reported to have $IC_{50}$ values of 42 pM and 130 nM as an inhibitor of PDE4 and TNFproduction, respectively; Muller *et al.,* "N-Phthaloyl beta-aryl-beta-amino derivatives: Potent TNF-alpha and PDE4 inhibitors," *217th American Chemical Society,* Annheim, Germany, MEDI 200, 1999; and Muller *et al.,* "Thalidomide analogs and PDE4 inhibition," *Bioorg. Med. Chem. Letts.* **8** 2669-2674, 1998.

CDC-801 is from a series of compounds based on thalidomide and has been developed primarily to improve the TNF-$\alpha$ inhibitory activity of thalidomide for the treatment of autoimmune diseases. Thalidomide may be represented by Formula (0.1.33):

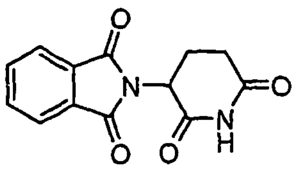

Thalidomide

(0.1.33)

CDC-801 has also been studied for the treatment of Crohn's disease, a chronic granulomatous inflammatory disease of unknown etiology commonly involving the terminal ileum, with scarring and thickening of the bowel wall which frequently leads to intestinal obstruction and fistula and abscess formation. Crohn's disease has a high rate of recurrence after treatment.

YM-58997 has an $IC_{50}$ value of 1.2 nM against PDE4; Takayama *et al.,* "Synthetic studies on selective Type IV phosphodiesterase (PDE IV) inhibitors," *214th American Chemical Society,* Las Vegas, USA, MEDI 245, 1997. YM-58997 has a 1,8-naphthyridin-2-one structure, as does YM-976.

Roflumilast has been studied for the treatment of both COPD and asthma, and has an $IC_{50}$ value of 3.5 nM in standard *in vitro* guinea-pig models of asthma. The use of roflumilast and a surfactant for the treatment of adult respiratory distress syndrome (ARDS) has also been described.

AWD-12,281, which is now designated as loteprednol, has been shown to be active in a rat model of allergic rhinitis, as described further below in a section which deals with allergic rhinitis and the use of PDE4 inhibitors to treat it. AWD-12,281 may be represented by Formula (0.1.34):

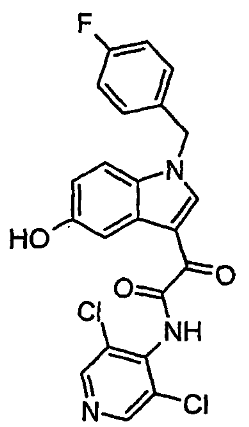

Lotepredinol (AWD-12,281)

(0.1.34)

Compounds related in structure to CDP840, shown further above as Formula (0.1.9), include L-826,141, which has been reported to have activity in a rat model of bronchitis; Gordon *et al.,* "Anti-inflammatory effects of a PDE4 inhibitor in a rat model of chronic bronchitis," Am. *J. Respir. Crit. Care Med.* **159** A33, 1999. Another such compound is related in structure to those reported in Perrier *et al.*, "Substituted furans as inhibitors of the PDE4 enzyme," *Bioorg. Med. Chem. Letts.* **9** 323-326, 1999, and is represented by Formula (0.1.35):

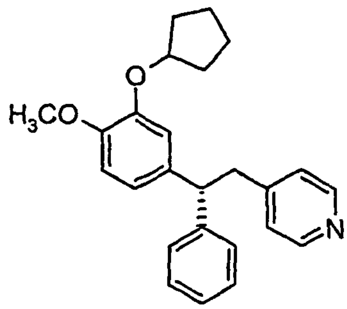

CDP840
(0.1.9)

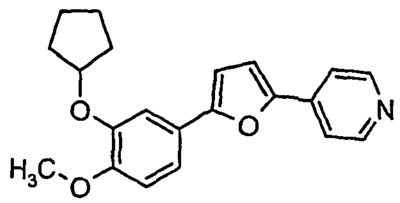

(0.1.35)

Other compounds which been found to be very potent PDE4 inhibitors are those represented by Formulas (0.1.36), (0.1.37), and (0.1.38):

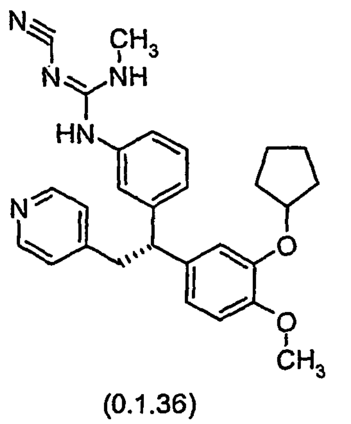

(0.1.36)

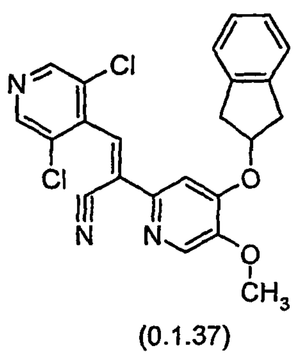

(0.1.37)

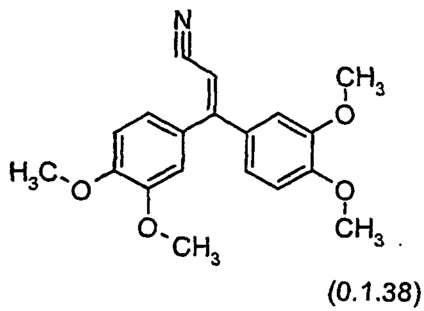

(0.1.38)

Compounds have been created, which combine PDE4 and matrix metalloproteinase (MMP) inhibitory activity in a single molecule; Groneberg *et al.,* "Dual inhibition of phosphodiesterase 4 and matrix metalloproteinases by an (arylsulfonyl)hydroxamic acid template," *J. Med. Chem.* **42**(4) 541-544, 1999. Two examples of such compounds are represented by Formulas (0.1.39) and (0.1.40):

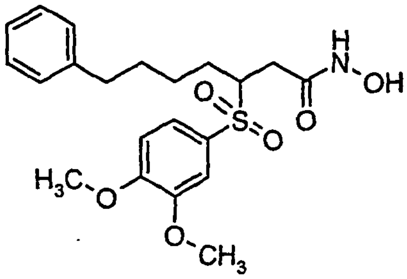

**(0.1.39)**

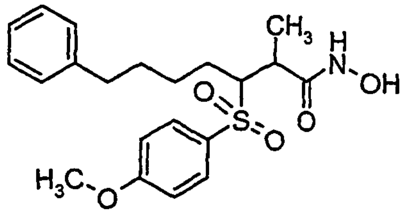

**(0.1.40)**

The compounds identified as KF19514 and KF17625 have been shown in guinea-pig studies to have activity in the inhibition of the following: histamine-induced and antigen-induced bronchoconstriction; PAF-induced lung eosinophilia and antigen-induced BAL eosinophilia; acetylcholine (ACh)-induced AHR; PAF-induced BAL eosinophilia and neutrophilia, and AHR; antigen-induced bronchospasm; and anaphylactic bronchoconstriction; Fujimura *et al.,* "Bronchoprotective effects of KF-19514 and cilostazol in guinea-pigs *in vivo," Eur. J. Pharmacol.* **327** 57-63, 1997; Manabe *et al., Ibid.;* Manabe *et al.,* "KF19514, a phosphodiesterase 4 and 1 inhibitor, inhibits PAF-induced lung inflammatory responses by inhaled administration in guinea-pigs," *Int. Arch. Allergy Immunol.* **114** 389-399, 1997; Suzuki *et al.,* "New bronchodilators. 3. Imidazo[4,5-*c*][1,8]naphthyridin-4(5H)-ones," *J. Med. Chem.* **35** 4866-4874, 1992; Matsuura *et al.,* "Substituted 1,8-naphthyridin-2(1H)-ones as selective phosphodiesterase IV inhibitors," *Biol. Pharm. Bull.* **17**(4) 498-503, 1994; and Manabe *et al.,* "Pharmacological properties of a new bronchodilator, KF17625," *Jpn. J. Pharmacol.* **58**(Suppl. 1) 238P, 1992. KF19514 and KF17625 may be represented by Formulas (0.1.41) and (0.1.42):

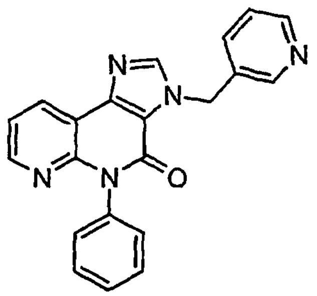

**KF19514**
**(0.1.41)**

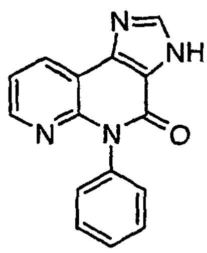

**KF17625**

**(0.1.42)**

The reported potency and lack of emesis in a series of indandiones suggests that the hypothesis that has related side-effects such as emesis to the ratio of affinity for the PDE4 enzyme relative to that for the high affinity rolipram binding site (HARBS) is erroneous. Such indandiones may be represented by Formulas (0.1.43) and (0.1.44):

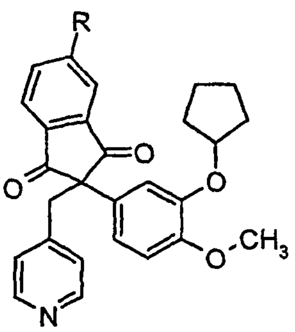

R = benzyloxy (0.1.43)

R = [1,4']-piperidinyl-1'-carbonyloxy (0.1.44)

The PDE4 inhibitors that have been created heretofore fall into a significant number of different classes in terms of their chemical structures. Such classes have been as diverse as phenanthridines and naphthyridines. One class of PDE4 inhibitors are lignans such as T-440, which has been demonstrated to have activity in the inhibition of the following: early phase bronchoconstriction induced by antigen, histamine, LTD4, U-46619, Ach, neurokinin A, and endothelin-1; allergen-induced early phase and late phase bronchoconstriction and BAL eosinophilia; and ozone-induced AHR and airway epithelial injury. Optimization of the PDE4 inhibitory potency of such compounds has led to the discovery of T-2585, one of the most potent PDE4 inhibitors described to date with an $IC_{50}$ value of 0.13 nM against guinea-pig lung PDE4. T-440 and T-2585 may be represented by Formulas (0.1.45) and (0.1.46):

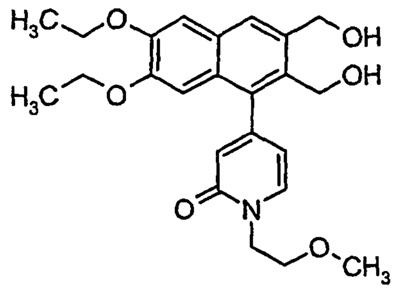

T-440
(0.1.45)

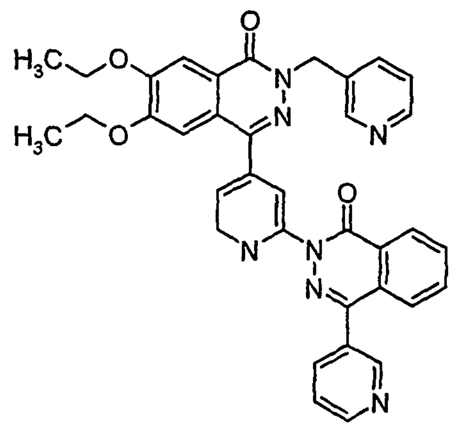

T-2585

(0.1.46)

Another class of PDE4 inhibitors consists of benzofurans and benzothiophenes. In particular, furan and chroman rings have been utilized as surrogates for the cyclopentylether of the rolipram pharmacophore. An example of such a compound is one that is apparently related in structure to BAY 19-8004, and which may be represented by Formula (0.1.47):

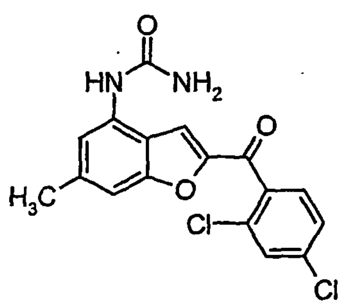

(0.1.47)

Another benzofuran-type compound has been reported to have an $IC_{50}$ value of 2.5 nM, and may be represented by Formula (0.1.48):

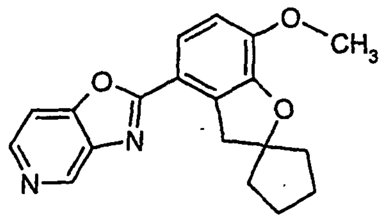

**(0.1.48)**

A compound with a related structure, which is not, however, a benzofuran, is characterized by a fused dioxicin ring and has been reported to produce almost complete inhibition of canine tracheal PDE4 at 100 nM. This compound may be represented by Formula (0.1.49):

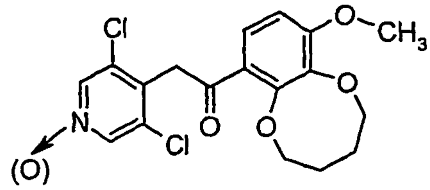

**(0.1.49)**

Quinolines and quinolones are a further class of PDE4 inhibitor structures, and they serve as surrogates for the catechol moiety of rolipram. This compound and two compounds of similar structure may be represented by Formulas (0.1.50), (0.1.51), and (0.1.52):

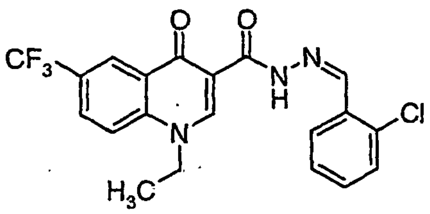

**(0.1.50)**

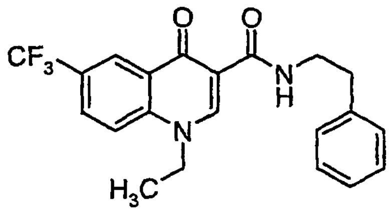

**(0.1.51)**

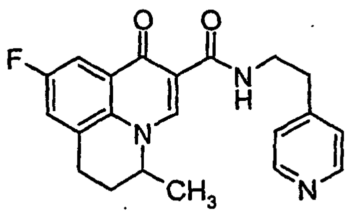

**(0.1.52)**

Diazepinoindoles represent another structural class of compound to which some PDE4 inhibitors described in the art belong. The PDE4 inhibitors CI-1018 and PD-168787, described further above, belong to this class of compounds. Another example of a diazepinoindole, one that has been reported to have an $IC_{50}$ of 3 nM against the PDE4 from U937 cells, may be represented by Formula (0.1.53):

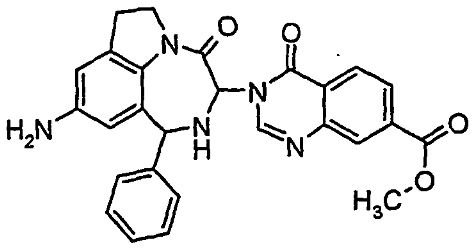

(0.1.53)

Purines, xanthines, and pteridines represent yet further classes of chemical compounds to which PDE4 inhibitors described heretofore in the art belong. The compound V-11294A described further above and represented by Formula (0.1.22), is a purine. A PDE4 inhibitor which is a xanthine compound, the class of compounds to which theophylline belongs, has been described in the art; Montana *et al.,* "PDE4 inhibitors, new xanthine analogues," *Bioorg. Med. Chem. Letts.* **8** 2925-2930, 1998. The xanthine compound may be represented by Formula (0.1.54):

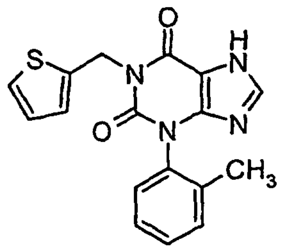

(0.1.54)

A potent PDE4 inhibitor belonging to the pteridine class of compounds has been demonstrated to have an $IC_{50}$ value of 16 nM against a PDE4 derived from tumor cells and to inhibit the growth of tumor cells at micromolar concentrations; Merz *et al.,* "Synthesis of 7-Benzylamino-6-chloro-2-piperazino-4-pyrrolidinopteridine and novel derivatives free of positional isomers. Potent inhibitors of cAMP-specific phosphodiesterase and of malignant tumor cell growth," *J. Med. Chem.* **41** (24) 4733-4743, 1998. The pteridine PDE4 inhibitor may be represented by Formula (0.1.55):

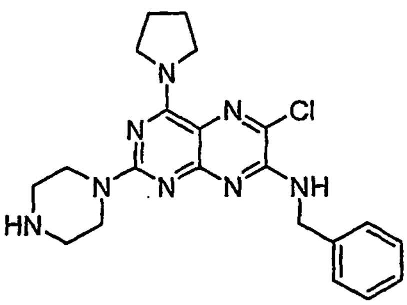

(0.1.55)

Triazines represent a still further class of chemical compounds to which PDE4 inhibitors belong that have been described in the art heretofore. Two such triazines have been described which display bronchodilator activity and are potent relaxant agents in a guinea-pig trachea model. These compounds, which may be represented by Formulas (0.1.56) and (0.1.57) below, are also moderately potent PDE4 inhibitors with $IC_{50}$ values of 150 and 140 nM, respectively:

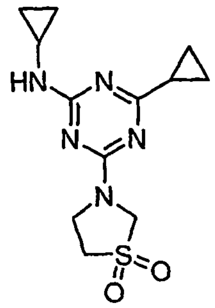

(0.1.56)

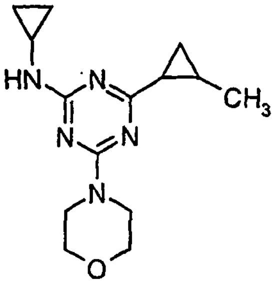

(0.1.57)

A triazine having a structure assumed to be closely related to that of the compounds of Formulas (0.1.56) and (0.1.57) is UCB-29936, which has been demonstrated to have activity in a murine model of septic shock; Danhaive *et al.,* "UCB29936, a selective phosphodiesterase Type IV inhibitor: therapeutic potential in endotoxic shock," *Am. J. Respir. Crit. Care. Med.* **159** A611, 1999.

Efforts have also been made in the art to improve the selectivity of PDE4 inhibitors with respect to the A through D subtypes described further above. There are presently four known isoforms (subtypes) of the PDE4 isozyme, encompassing seven splice variants, also described further above. The PDE4D isoform mRNA is expressed in inflammatory cells such as neutrophils and eosinophils, and it has been suggested in the art that D-selective inhibitors of PDE4 will provide good clinical efficacy with reduced side-effects. A nicotinamide derivative displaying 100-fold selectivity for inhibition of the PDE4D isoform has been described; WO 98/45268; as well as a naphthyridine derivative reported to be a PDE4D selective inhibitor; WO 98/18796. These compounds may be represented by Formulas (0.1.58) and (0.1.59), respectively:

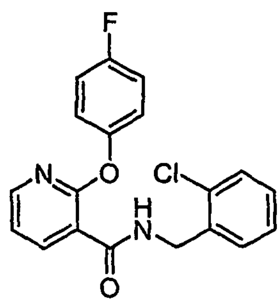

(0.1.58)

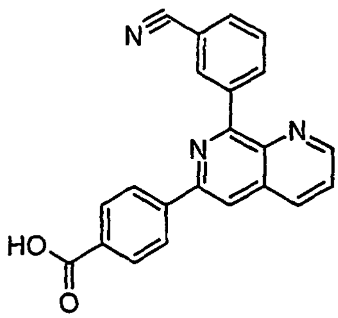

(0.1.59)

Another nicotinamide compound has been described in the art which may be useful in the treatment of CNS diseases such as multiple sclerosis; GB-2327675; and a rolipram derivative has been described in the art which is a PDE4 inhibitor which binds with equal affinity to both the catalytic and the HARB sites on human PDE4B2B; Tian *et al.,* "Dual inhibition of human Type 4 phosphodiesterase isostates by (*R,R*)-(+/-)-methyl-3-acetyl-4-[3-(cyclopentyloxy)-4-methoxyphenyl]-3-methyl-1-pyrrolidine carboxylate," *Biochemistry* **37**(19) 6894-6904, 1998. The nicotinamide derivative and the rolipram derivative may be represented by Formulas (0.1.60) and (0.1.61), respectively:

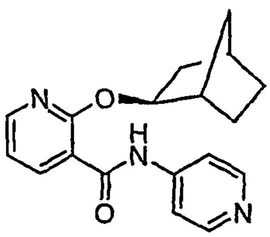

(0.1.60)

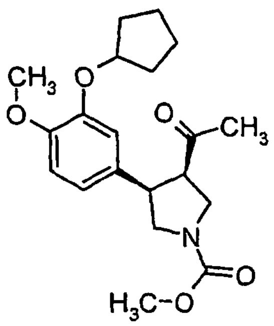

(0.1.61)

Further background information concerning selective PDE4 isozymes may be found in publications available in the art, *e.g.,* Norman, "PDE4 inhibitors 1999," *Exp. Opin. Ther. Patents* **9**(8) 1101-1118, 1999 (Ashley Publications Ltd.); and Dyke and Montana, "The therapeutic potential of PDE4 inhibitors," *Exp. Opin. Invest. Drugs* **8**(9) 1301-1325, 1999 (Ashley Publications Ltd.).

WO 98/45268 (Marfat *et al.*), published October 15, 1998, discloses nicotinamide derivatives having activity as selective inhibitors of PDE4D isozyme. These selective inhibitors are represented by Formula (0.1.62):

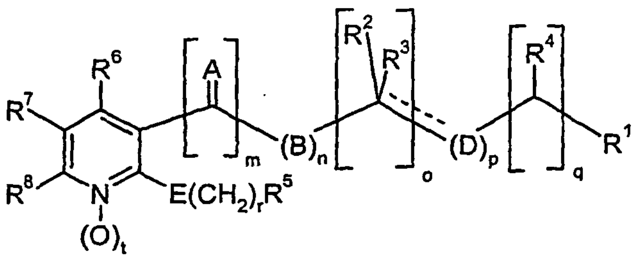

(0.1.62)

US 4,861,891 (Saccomano *et al.*), issued August 29, 1989, discloses nicotinamide compounds which function as calcium independent c-AMP phosphodiesterase inhibitors useful as antidepressants, of Formula (0.1.63):

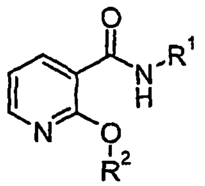

(0.1.63)

The nicotinamide nucleus of a typical compound disclosed in this patent is bonded directly to the **$R^1$** group, which is defined as 1-piperidyl, 1-(3-indolyl)ethyl, $C_1$-$C_4$ alkyl, phenyl, 1-(1-phenylethyl), or benzyl optionally mono-substituted by methyl, methoxy, chloro or fluoro. The **$R^2$** substituent is bicyclo[2.2.1]hept-2-yl or

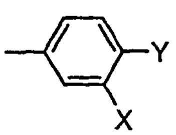

where **Y** is H, F or Cl; and **X** is H, F, Cl, $OCH_3$, $CF_3$, CN, COOH, -C(=O)($C_1$-$C_4$) alkoxy, $NH(CH_3)C(=O)$- (methylcarbamoyl) or $N(CH_3)_2C(=O)$- (dimethylcarbamoyl).

US 4,692,185 (Michaely *et al.*) discloses herbicides such as those of Formula (0.1.64):

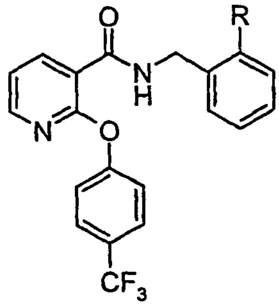

**(0.1.64)**

where **R** is ($C_1$-$C_4$) alkyl, ($C_1$-$C_4$) haloalkyl, or halo.

EP 550 900 (Jeschke *et al.)* discloses herbicides and plant nematicides of Formule (0.1.65);

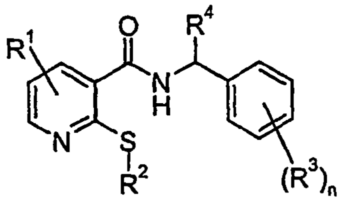

**(0.1.65)**

where n is 0-3; $\mathbf{R^1}$ is selected from numerous groups, but is usually H, 6-$CH_3$, or 5-Cl; $\mathbf{R^2}$ is alkyl, alkenyl, alkynyl, cycloalkyl, aryl or aralkyl; $\mathbf{R^3}$ is halo, CN, $NO_2$, alkyl, haloalkyl, alkoxy, haloalkoxy, alkylthio, haloalkylthio, alkylsulfonyl, haloalkylsulfonyl, aryl, aryloxy, or arylthio; and $\mathbf{R^4}$ is alkyl.

EP 500 989 (Mollner *et al.*) discloses ACE inhibitors of Formula (0.1.66):

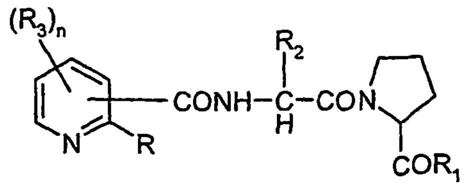

**(0.1.66)**

where n is 0-3; **R** is OH, SH, COOH, $NH_2$, halo, $OR_4$, $SR_4$, $COOR_4$, $NHR_4$ or $N(R_4)_2$, where $\mathbf{R_4}$ is lower alkyl, optionally substituted aryl, or acyl; $\mathbf{R_1}$ is OH, lower alkoxy, optionally substituted aryl lower alkoxy, aryloxy, or disubstituted amino; $\mathbf{R_2}$ is lower alkyl or amino lower alkyl; and $\mathbf{R_3}$ is halo, $NO_2$, lower alkyl, halo lower alkyl, aryl lower alkyl, or aryl. Specific embodiments disclosed include compounds such as that of Formula (0.1.67):

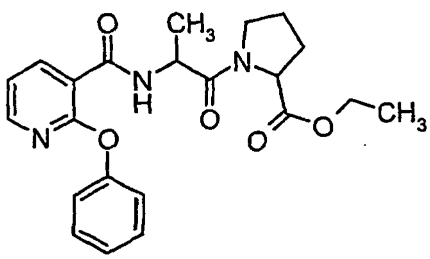

**(0.1.67)**

FR 2.140.772 (Aries) discloses compounds asserted to have utility as analgesics, tranquilizers, antipyretics, anti-inflammatories, and antirheumatics, of Formula (0.1.68):

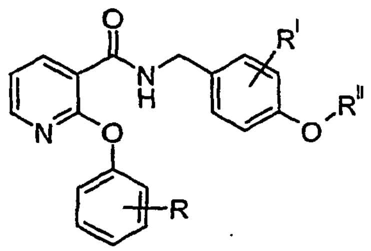

(0.1.68)

where **R** is 1 or 2 substituents chosen from lower alkyl, trihalomethyl, alkoxy, and halo; **R'** is H or alkyl; and **R"** is hydrogen or alkyl.

JP 07 304775 (Otsuka *et al.*) discloses naphthyridine and pyridopyrazine derivatives which have anti-inflammatory, immunomodulating, analgesic, antipyretic, antiallergic, and antidepressive action. Also disclosed are intermediates of Formula (0.1.69):

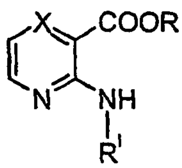

(0.1.69)

where **X** may be CH, and **R** and **R'** are each lower alkyl.

With regard to the disclosures of the above-identified patents and published patent applications, it will be appreciated that only the disclosure of WO 98/45268 (Marfat *et al.*) concerns the same biological activity of inhibition of the PDE4 isozyme. The state of the art also contains information regarding compounds wholly dissimilar in chemical structure to those used in the combinations of the present invention, but which, on the other hand, possess biological activity similar to that of the compounds used in the present invention. Representative patents and published patent applications disclosing said information are illustrated further below.

US 5,552,438; US 5,602,157; and US 5,614,540 (all to Christensen), which all share the same April 2, 1992 priority date, relate to a therapeutic agent identified as ARIFLO®, which is a compound of Formula (0.1.70) and named as indicated below:

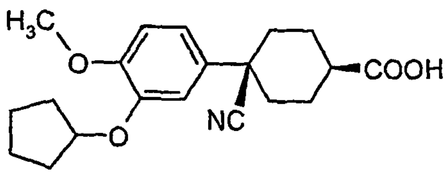

ARIFLO®

cis-[4-cyano-4-(3-cyclopentyl-oxy-4-methoxyphenyl)cyclo-hexane-1-carboxylic acid (0.1.70)

The compound of Formula (0.1.9) falls within the scope of US 5,552,438 which discloses a genus of compounds of Formula (0.1.71):

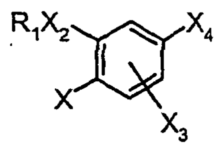

(0.1.71)

where $\mathbf{R_1}$ = $-(CR_4R_5)_rR_6$ where r = 0 and $\mathbf{R_6}$ = $C_{3-6}$ cycloalkyl; **X** = $YR_2$ where **Y** = O and $\mathbf{R_2}$ = $-CH_3$; $\mathbf{X_2}$ = O; $\mathbf{X_3}$ = H; and $\mathbf{X_4}$ = a moiety of partial Formula (0.1.72)

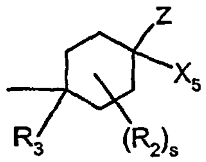

(0.1.72)

where $\mathbf{X_5}$ = H; **s** = 0; $\mathbf{R_3}$ = CN; and **Z** = $C(O)OR_{14}$ where $\mathbf{R_{14}}$ = H. The disclosures of US 5,602,157 and US 5,614,540 differ from that of US 5,552,438 and each other as to the definition of the $R_3$ group, which in the case of the ARIFLO® compound, is CN. A preferred salt form of the ARIFLO® compound is disclosed to be the tris(hydroxymethyl)ammonium methane salt.

US 5,863,926 (Christensen *et al.*) discloses analogs of the ARIFLO® compound, *e.g.,* that of Formula (0.1.73):

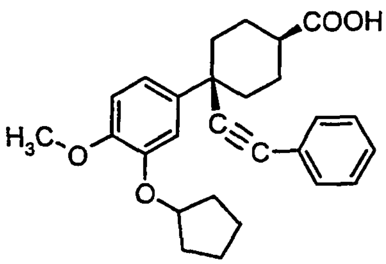

(0.1.73)

WO 99/18793 (Webb *et al.*) discloses a process of making the ARIFLO® and related compounds. WO 95/00139 (Bamette *et al.*) claims a compound which has an $IC_{50}$ ratio of about 0.1 or greater as regards the $IC_{50}$ for the PDE IV catalytic form which binds rolipram with a high affinity, divided by the $IC_{50}$ for the form which binds rolipram with a low affinity; but in a dependent claim restricts the scope thereof to a compound which was not known to be a PDE4 inhibitor prior to June 21, 1993.

WO 99/20625 (Eggleston) discloses crystalline polymorphic forms of cipamfylline for treatment of $PDE_4$ and TNF mediated diseases, of Formula (0.1.74):

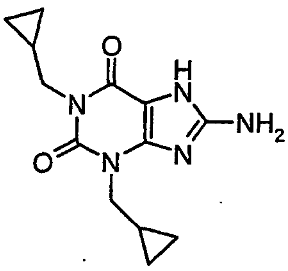

Cipamfylline

(0.1.74)

WO 99/20280 (Griswold *et al.*) discloses a method of treating pruritis by administering an effective amount of a PDE4 inhibitor, *e.g.*, a compound of Formula (0.1.75):

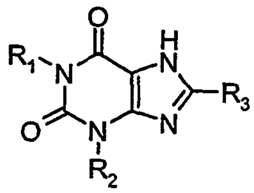

**(0.1.75)**

US 5,922,557 (Pon) discloses a CHO-K1 cell line which stably expresses high levels of a full length low-Km cAMP specific PDE4A enzyme, which has, in turn, been used to examine potent PDE4 enzyme inhibitors and compare the rank order of their potencies in elevating cAMP in a whole-cell preparation with their ability to inhibit phosphodiesterase activity in a broken-cell preparation. It is further said to be found that the soluble enzyme inhibition assay described in the prior art does not reflect behavior of the inhibitors acting *in vivo.* An improved soluble enzyme whole-cell assay is then disclosed which is said to reflect the behavior of inhibitors acting *in vivo.* It is further disclosed that there exist at least four distinct PDE4 isoforms or subtypes, and that each subtype has been shown to give rise to a number of splice variants, which in themselves can exhibit different cellular localization and affinities for inhibitors.

With regard to the disclosures of the above-identified patents and published patent applications, it will be appreciated that the compounds involved possess the same biological activity of inhibition of PDE4 as the compounds used in the combinations of the present invention. At the same time, however, the artisan will observe that the chemical structures of said compounds disclosed in the prior art are not only diverse from each other but dissimilar to that of the novel compounds of the present invention as well. The state of the art contains still further information regarding compounds which are dissimilar in chemical structure to those used in the present invention, and which, moreover, do not possess PDE4 inhibitory activity similar to said compounds of the present invention. Such compounds disclosed in the prior art do, nevertheless, often have therapeutic utility similar to that possessed by the compounds used in the present invention, *i.e.,* in the treatment of inflammatory, respiratory and allergic diseases and conditions. In particular this is applicable to certain inhibitors of enzymes and antagonists of receptors in the so-called leukotriene pathway. This is especially the case with regard to the leukotrienes $LTB_4$ and $LTD_4$. Accordingly, representative patents and published patent applications disclosing further information of this type are described below.

Arachidonic acid is metabolized by cyclooxygenase-1 and by 5-lipoxygenase. The 5-lipoxygenase pathway leads to the production of leukotrienes (LTs) which contribute to the inflammatory response through their effect on neutrophil aggregation, degranulation and chemotaxis; vascular permeability; smooth muscle contractility; and on lymphocytes. The cysteinyl leukotrienes, $LTC_4$, $LTD_4$, and $LTE_4$, play an important role in the pathogenesis of asthma. The components of the leukotriene pathway which afford targets for therapeutic intervention are illustrated in the following diagram:

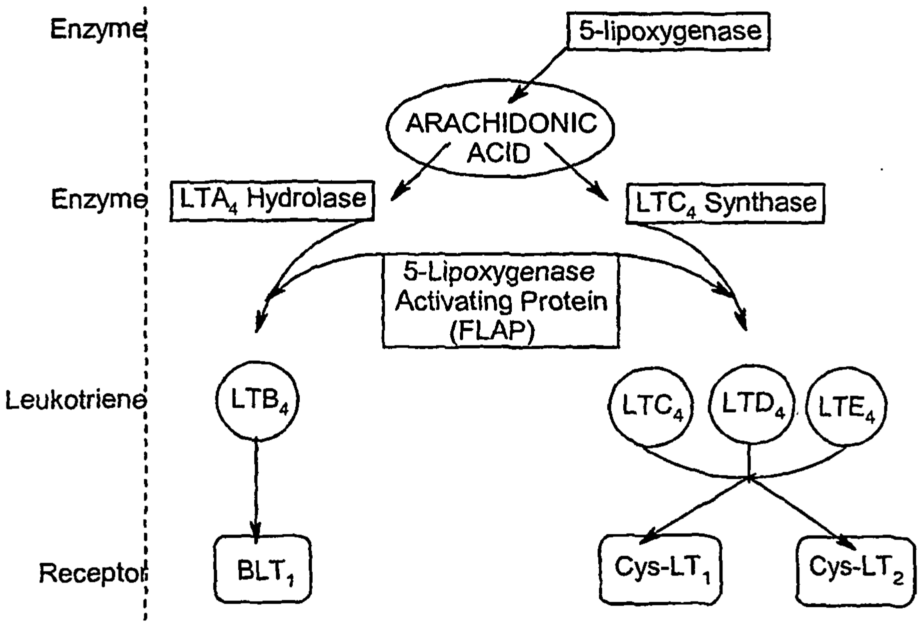


Accordingly, agents which are able to intervene in any of the steps of the 5-lipoxygenase pathway afford an opportunity for therapeutic treatment. An example of one such agent is the 5-lipoxygenase inhibitor, zileuton, a therapeutic agent identified as ZYFLO® which may be represented by Formula (0.1.76):

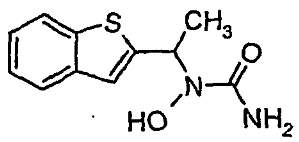

ZYFLO®
*Zileuton* (0.1.76)

Another such agent is the $LTD_4$ receptor antagonist zafirlukast, a therapeutic agent identified as ACCOLATE® which may be represented by Formula (0.1.77):

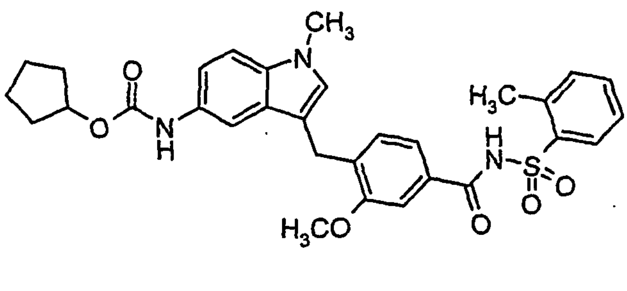

ACCOLATE®

*Zafirlukast* (0.1.77)

A further such $LTD_4$ receptor antagonist is montelukast, a therapeutic agent identified as SINGULAIR® which may be represented by Formula (0.1.78):

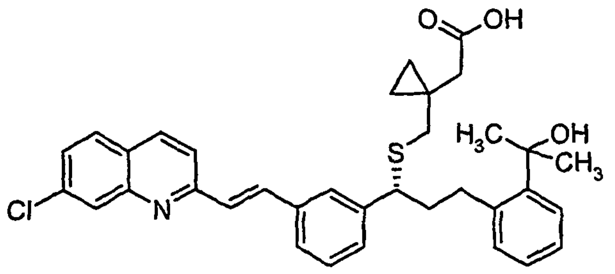

SINGULAIR®
Montelukast (0.1.78)

Another type of the above-mentioned therapeutic targets is the $LTB_4$ receptor, and an example of an antagonist for said receptor is BIIL-260, a therapeutic agent which may be represented by Formula (0.1.79):

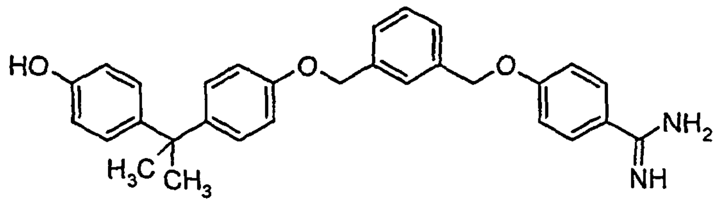

BIIL-260 (0.1.79)

Another example of a therapeutic agent which is an $LTB_4$ receptor antagonist is CGS-25019c which may be represented by Formula (0.1.80):

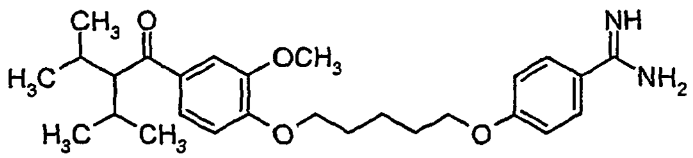

CGS-25019c (0.1.80)

Nothing in the above-described state of the art discloses or would suggest to the artisan the novel combinations of compounds of the present invention comprising PDE4 inhibitors and anti-cholinergic agents.

## Anti-Cholinergic Agents

Anti-cholinergic agents prevent the passage of, or effects resulting from passage of impulses through the parasympathetic nerves. This action results from their ability to inhibit the action of the neurotransmitter acetylcholine by blocking its binding to muscarinic cholinergic receptors. There are at least three types of muscarinic receptor subtypes. $M_1$ receptors is found primarily in brain and other tissue of the central nervous system, $M_2$ receptors are found in heart and other cardiovascular tissue, and $M_3$ receptors are found in smooth muscle and glandular tissues. The muscarinic receptors are located at neuroeffector sites on, *e.g.,* smooth muscle, and in particular $M_3$-muscarinic receptors are located in airway smooth muscle Consequently, anti-cholinergic agents may also be referred to as muscarinic receptor antagonists. Atropine and scopolamine are the best known members of this class of therapeutic agents.

The parasympathetic nervous system plays a major role in regulating bronchomotor tone, and bronchoconstriction is largely the result of reflex increases in parasympathetic activity caused in turn by a diverse set of stimuli. Anti-cholinergic agents have a long history of use in the treatment of asthma and were used as bronchodilators before the advent of epinephrine. They were thereafter supplanted by β-adrenergic agents and methylxanthines. However, the more recent introduction of ipratropium bromide has led to a revival in the use of anti-cholinergic therapy in the treatment of respiratory diseases.

However, there are muscarinic receptors on peripheral organ systems such as salivary glands and gut and therefore

systemically active muscarinic receptor antagonists are limited by dry mouth-and constipation. Thus the bronchodilatory and other beneficial actions of muscarinic receptor antagonists is ideally produced by an inhaled agent which has a high therapeutic index for activity in the lung compared with the peripheral compartment.

Anti-cholinergic agents also partially antagonize bronchoconstriction induced by histamine, bradykinin, or prostaglandin $F_{2\alpha}$, which is deemed to reflect the participation of parasympathetic efferents in the bronchial reflexes elicited by these agents.

The anti-cholinergic agents ipratropium and oxitropium are quaternary ammonium compounds in structure, and central effects from these agents are generally lacking because these agents do not readily cross the blood-brain barrier. When these agents are inhaled, their actions are confined almost entirely to the mouth and airways. Even when inhaled at several times the recommended dose, these agents produced little or no change in heart rate, blood pressure, bladder function, intraocular pressure, or pupillary diameter. This selectivity results from the very inefficient absorption of these agents from the lung or gastrointestinal tract. The preclinical and clinical profile of tiotropium is entirely in accord with these characteristics, with the profound difference that tiotropium has a prolonged duration of action resulting from its slow dissociation from the muscarinic $M_3$ receptor.
Ipratropium and oxitropium may be represented by Formulas (1.0.1) and (1.0.2), respectively:

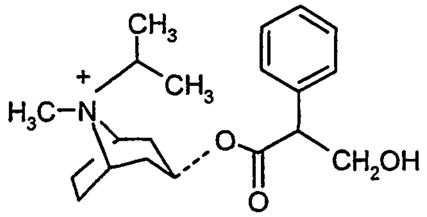

(1.0.1)

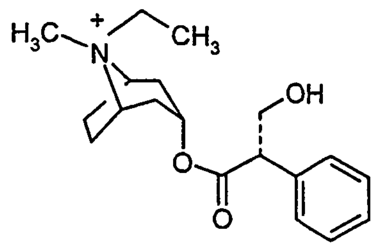

(1.0.2)

Anti-cholinergic agents having bronchodilator activity known in the art include ambutonium bromide; apoatropine; benzilonium bromide; benztropine mesylate; bevonium methylsulfate; butropium bromide; *N*-butylscopolammonium bromide; cimetropium bromide; clidinium bromide; cyclonium iodide; difemerine; diponium bromide; emepronium bromide; etomidoline; fenpiverinium bromide; fentonium bromide; flutropium bromide; heteronium bromide; hexocyclium methylsulfate; octamylamine; oxyphenonium bromide; pentapiperide; piperilate; poldine methylsulfate; prifinium bromide; propyromazine; sultroponium; tematropium methylsulfate; tiemonium iodide; tiquizium bromide; trimebutine; tropenzile; trospium chloride; and xenytropium bromide.

Further anti-cholinergic agents are disclosed and described in detail in the published applications and issued patents set out in the paragraphs that follow.

US 5,605,908 and US 5,998,404 assigned to Eli Lilly and Company discloses azacycloalkoxy-substituted pyrazines, oxadiazoles, and related compounds as muscarinic and nicotinic cholinergic agents useful as stimulants of cognitive function and the treatment of Alzheimer's disease, wherein said compounds are of Formulas (1.0.3) and (1.0.4), including a species compound of Formula (1.0.5):

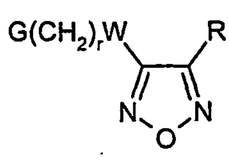

(1.0.3)

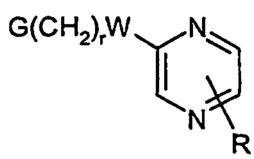

(1.0.4)

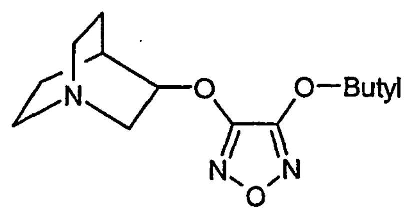

(1.0.5)

wherein **W** is O or S; **R** is H; amino; halo; $R^4$, $OR^4$, $SR^4$, $SOR^4$, or $SO_2R^4$ where $\mathbf{R^4}$ is optionally substituted alkyl, alkenyl, or alkynyl; cycloalkyl; optionally substituted phenyl; phenyl-$CH_2$-O(=O)C-; **G** is optionally substituted alkyl, cycloalkyl, azetidinyl, pyrrolidinyl, piperidinyl, azabicyclo[2.2.2]octyl; and r is 0 to 2.

US 5,821,249 assigned to the University of Rochester discloses methylecgonidine and anti-cholinergically

active derivatives or analogs thereof that are useful in the prevention or treatment of a disease or disorder treatable by antimuscarinic anti-cholinergic agent, an anti-histaminic agent or a spasmolytic agent, in particular bronchoconstriction in a number of pulmonary diseases such as asthma. The above-mentioned methylecgonidine and its derivatives and epoxide analogs may be represented by Formulas (1.0.6) and (1.0.7), respectively:

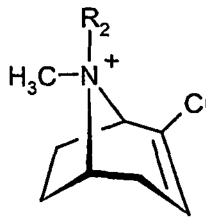

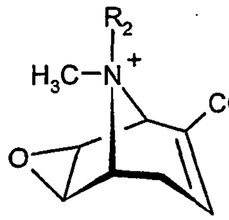

**(1.0.6)** **(1.0.7)**

wherein $\mathbf{R_2}$ is -H, $(C_1\text{-}C_{10})$ alkyl, or an amidine; and $\mathbf{R_1}$ is $(C_1\text{-}C_{10})$ alkyl, or an aryl substituted $(C_1\text{-}C_{10})$ alkyl.

US 5,861,423 assigned to R. J. Reynolds Tobacco Co. discloses pyridinylbutenylamine nicotinic cholinertic agents comprising a compound of Formula (1.0.8):

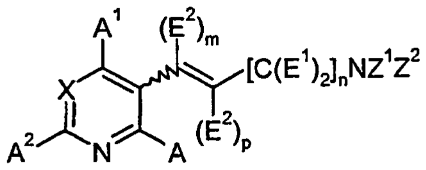

**(1.0.8)**

wherein **X** is CR', COR', or $CCH_2OR'$ where **R'** is H, alkyl, or an optionally substituted aromatic group-containing moiety; $\mathbf{E^1}$ is H, alkyl, or haloalkyl; $\mathbf{E^2}$ is alkyl, or haloalkyl; $\mathbf{Z^1}$ and $\mathbf{Z^2}$ are H, alkyl, or aryl; $\mathbf{Z^1Z^2N}$ is heterocyclyl; **A**, $\mathbf{A^1}$, and $\mathbf{A^2}$ are H, alkyl, or halo; **m** is 0 or 1; **n** is 1 to 8; and **p** is 0 or 1.

US 6,017,927 assigned to Yamanouchi Pharmaceutical Co. discloses quinuclidine derivatives that have a selective antagonistic effect on muscarinic $M_3$ receptors and are useful as a preventive treatment or remedy for urologic diseases, respiratory diseases, or digestive diseases. The above-mentioned derivatives may be represented by Formula (1.0.9):

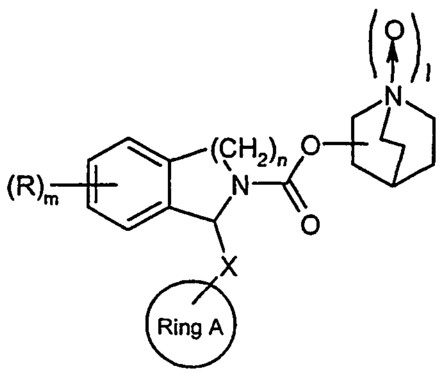

**(1.0.9)**

wherein **Ring A** is aryl, cycloalkyl, cycloalkenyl, heteroaryl of 1-4 heteroatoms N, O, or S, or optionally substituted 5-7-membered saturated heterocyclic; **X** is a single bond or methylene; **R** is halo, hydroxy, lower alkoxy, carboxyl, lower

alkoxycarbonyl, lower acyl, mercapto, lower alkylthio, sulfonyl, lower alkylsulfonyl, sulfinyl, lower alkylsulfinyl, sufonamido, lower alkylsufonamido, carbamoyl, thiocarbamoyl, mono- or di-lower alkylcarbamoyl, nitro, cyano, amino, mono- or di-lower alkylamino, methylenedioxy, ethylenedioxy, or loweralkyl optionally substituted by halo, hydroxy, lower alkoxy, amino, or mono- or di-lower alkylamino; **l** is 0 or 1; **m** is 0 or 1-3; and **n** is 1 or 2. Preferred compounds of the type described include, *e.g.*, those represented by Formulas (1.0.10) and (1.0.11):

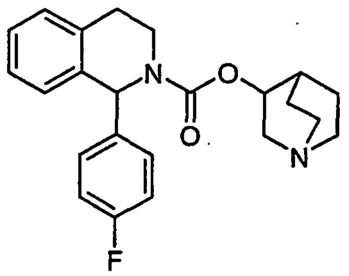

(1.0.10)

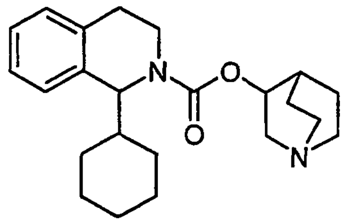

(1.0.11)

WO 97/08146 (Rachaman *et al.*) discloses carbamate derivatives of pyridostigmine useful in the treatment of cognitive impairments associated with cholinergic perturbances such as Alzheimer's disease comprising a compound of Formula (1.0.12), including a species compound of Formula (1.0.13):

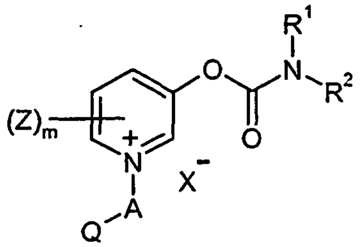

(1.0.12)

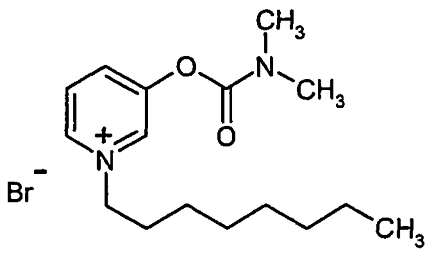

(1.0.13)

wherein $\mathbf{R^1}$ is H, alkyl, alkenyl, aryl, aralkyl, cycloalkyl, or cycloalkylalkyl; $\mathbf{R^2}$ is H, alkyl, alkenyl, aryl, aralkyl, cycloalkyl, or cycloalkylalkyl; **A** is alk(en/yn)ylene; **Z** is dialkylcarbamoyl or alkyl; **m** is 0 or 1; **Q** is a transporter recognition moiety for biological membranes, optionally coupled to a physiologically active acceptable moiety; and **X** is an anion.

WO 97/11072 assigned to Novo Nordisk A/s discloses azacyclic and azabicyclic nicotinic cholinergic agents useful in the treatment of Alzheimer's disease, Parkinson's disease, obesity, severe pain, tobacco withdrawal, and anxiety comprising a compound of Formula (1.0.14); (1.0.15); or (1.0.16); including a species compound of Formula (1.0.17):

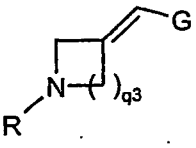

(1.0.14)

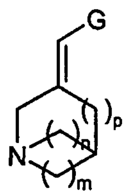

(1.0.15)

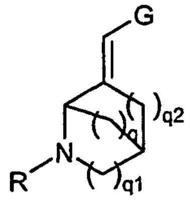

(1.0.16)

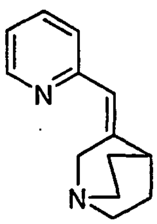

(1.0.17)

wherein m and **n** are 1 to 3; **p, q, q1,** and **q2** are 0 to 2; **q3** is 1 to 5; **R** is H, or alkyl; and **G** is selected from optionally substituted, 6-membered, N-heterocycles containing 1 to 4 N atoms.

WO 00/51970 assigned to Fujisawa Pharmaceutical Co., Ltd. discloses aryl and heteroayl amide potentiators of cholinergic activity useful as anti-amnesia or anti-dementia agents comprising a compound of Formula (1.0.18), including a species compound of Formula (1.0.19):

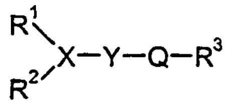

(1.0.18)

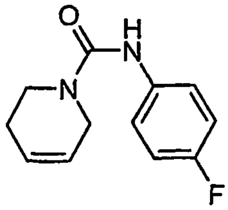

(1.0.19)

wherein $\mathbf{R^1}$ and $\mathbf{R^2}$ are aryl or ar(lower)alkyl, or together form lower alkylene, each of which is optionally substituted with aryl or condensed with a cyclic hydrocarbon optionally substituted by lower alkyl, lower alkoxy, aryl, arylamino, or aryloxy, each of which is optionally substituted by lower alkoxy or halogen, pyridyl, or pyridylamino; **X** is CH or N; **Y** is a single bond or -NH-; and **Q** is -C(=O)-.

## Brief description of the invention

The present invention is concerned with novel combinations of therapeutic agents which are useful in the treatment of obstructive airways and other inflammatory diseases, especially asthma, COPD, and other obstructive airways diseases exacerbated by bronchial hyper-reactivity and bronchospasm. Said novel combinations comprise the following: *(I)* a PDE4 inhibitor that is therapeutically effective in the treatment of the above-mentioned diseases when administered by inhalation and that is seleceted from the compounds recited below; together with *(II)* an anti-cholinergic agent comprising a member selected from the group consisting of tiotropium and derivatives thereof that is therapeutically effective in the treatment of the above-mentioned diseases when administered by inhalation.

The present invention is further concerned with the above-recited combination of therapeutic agents. The advantage of the combination is to provide optimal control of airway calibre through the mechanism most appropriate to the disease pathology, namely muscarinic receptor antagonism, together with effective suppression of inappropriate inflammation. By combining both antimuscarinic and PDE4 inhibitor compounds via the inhaled route, the benefits of each class are realised without the unwanted peripheral effects. Further, the combination results in unexpected synergy, producing greater efficacy than maximally tolerated doses of either class of agent used alone acting as they do on distinct disease processes important to the signs and symptoms suffered by the patients.

The present invention is concerned with the above-recited combination of therapeutic agents wherein said PDE4 inhbitor is selected from the group consisting of the following:

9-cyclopentyl-5,6-dihydro-7-ethyl-3-phenyl-9*H*-pyrazolo[3,4-*c*]-1,2,4-triazolo[4,3-α]pyridine;

9-cyclopenyl-5,6-dihydro-7-ethyl-3-(furan-2-yl)-9*H*-pyrazolo[3,4-*c*]-1,2,4-triazolo[4,3-α]pyridine;

9-cyclopentyl-5,6-dihydro-7-ethyl-3-(2-pyridyl)-9*H*-pyrazolo[3,4-*c*]-1,2,4-triazolo[4,3-α]pyridine;

9-cyclopentyl-5,6-dihydro-7-ethyl-3-(4-pyridyl)-9*H*-pyrazolo[3,4-*c*]-1,2,4-triazolo[4,3-α]pyridine;

9-cyclopentyl-5,6-dihydro-7-ethyl-3-(3-thienyl)-9*H*-pyrazolo[3,4-*c*]-1,2,4-triazolo[4,3-α]pyridine;

3-benzyl-9-cyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-propyl-9*H*-pyrazolo[3,4-*c*]-1,2,4-triazolo[4,3-α]pyridine;

3,9-dicyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-(1-methylcyclohex-1-yl)-9*H*-pyrazolo[3,4-*c*]-1,2,4-triazolo[4,3-α]pyridine;

3-(*tert*-butyl)-9-cyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-(2-methylphenyl)-9*H*-pyrazolo[3,4-*c*]-1,2,4-triazolo[4,3-α]pyridine;

9-cyclopentyl-5,6-dihydro-7-ethyl-3-(2-methoxyphenyl)-9*H*-pyrazolo[3,4-*c*]-1,2,4-triazolo[4,3-α]pyridine;

9-cyclopentyl-5,6-dihydro-7-ethyl-3-(thien-2-yl)-9*H*-pyrazolo[3,4-*c*]1,2,4-triazolo[4,3-α]pyridine;

3-(2-chlorophenyl)-9-cyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-(2-iodophenyl)-9*H*-pyrazolo[3,4-*c*]-1,2,4-triazolo[4,3-α]pyridine;

9-cyclopentyl-5,6-dihydro-7-ethyl-3-(2-trifluoromethylphenyl)-9*H*-pyrazolo[3,4-*c*]-1,2,4-triazolo[4,3-α]pyridine; and

5,6-dihydro-7-ethyl-9-(4-fluorophenyl)-3-(1-methylcyclohex-1-yl)-9*H*-pyrazolo[3,4-*c*]-1,2,4-triazolo[4,3-α]pyridine.

More in particular, the present invention relates to the above-mentioned combination of therapeutic agents

where said PDE4 inhibitor of Formula (1.1.1) is 9-cyclopentyl-5,6-dihydro-7-ethyl-3-(2-thienyl)-9*H*-pyrazolo[3,4-*c*]-1,2,4-triazolo[4,3-a]pyridine; or 9-cyclopentyl-5,6-dihydro-7-ethyl-3-(*tert*-butyl)-9*H*-pyrazolo[3,4-c]-1,2,4-triazolo[4,3-a]pyridine.

The present invention is further concerned with the above-recited combination of therapeutic agents wherein said anti-cholinergic agent consisting of a member selected from the group consisting of tiotropium and derivatives thereof is a compound of Formula (2.1.1):

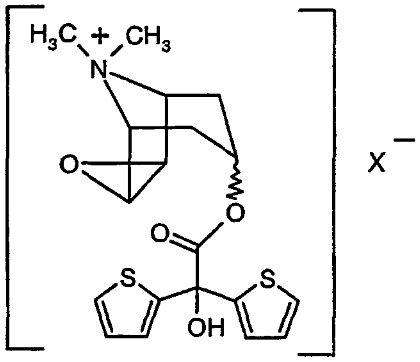

(2.1.1)

wherein $X^-$ is a physiologically acceptable anion selected from the group consisting of fluoride, $F^-$; chloride, $Cl^-$; bromide, $Br^-$; iodide, $I^-$; methanesulfonate, $CH_3S(=O)_2O^-$; ethanesulfonate, $CH_3CH_2S(=O)_2O^-$; methylsulfate, $CH_3OS(=O)_2O^-$; benzene sulfonate, $C_6H_5S(=O)_2O^-$; *p*-toluenesulfonate, and $4\text{-}CH_3\text{-}C_6H_5S(=O)_2O^-$.

The present invention is concerned in particular with the above-recited anti-cholinergic agent comprising a member selected from the group consisting of tiotropium and derivatives thereof, wherein said physiologically acceptable anion, $X^-$, is bromide, $Br^-$; and further wherein said tiotropium and derivatives thereof are 3-$\alpha$ compounds.

The present invention is further concerned in particular with the above-recited anti-cholinergic agent comprising a member selected from the group consisting of tiotropium and derivatives thereof, wherein said member thereof is tiotropium bromide, (1$\alpha$, 2$\beta$, 4$\beta$, 5$\alpha$, 7$\beta$)-7-[(hydroxydi-2-thienylacetyl)oxy]-9,9-dimethyl-3-oxa-9-azoniatricyclo[3.3.1.0$^{2,4}$]nonane bromide, represented by Formula (2.1.2) or Formula (2.1.3):

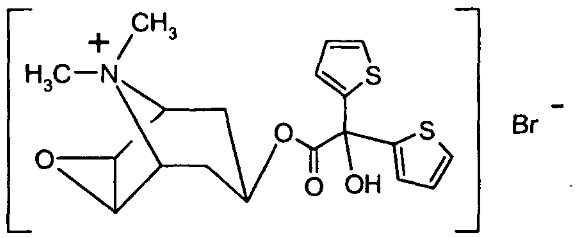

(2.1.2)

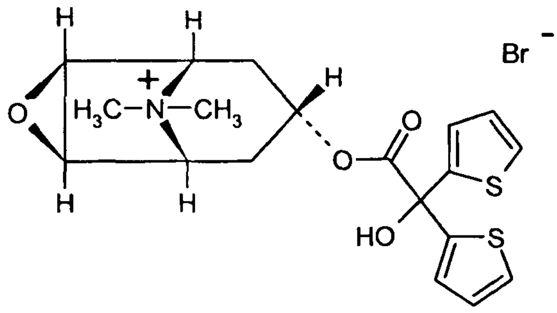

(2.1.3)

The present invention is also concerned with the use of a combination of therapeutic agents comprising (*I*) a PDE4 inhibitor selected from the group mentioned hereinbefore that is therapeutically effective in the treatment of the above-mentioned diseases when administered by inhalation; together with *(II)* an anti-cholinergic agent comprising a member selected from the group consisting of tiotropium and derivatives thereof that is therapeutically effective in the treatment of the above-mentioned diseases when administered by inhalation, for the manufacture of a medicament for the treatment of obstructive airways and other inflammatory diseases in a mammal in need of such treatment.

The present invention is concerned with the above-described use wherein the obstructive airways or other inflammatory disease comprises asthma, chronic obstructive pulmonary disease (COPD), and other obstructive airways diseases exacerbated by bronchial hyper-reactivity and bronchospasm.

The present invention is still further concerned with the above-described uses wherein said administration by inhalation comprises simultaneous or sequential delivery of the combination of therapeutic agents of the present invention in the form of an aerosol or dry powder dispersion.

The present invention is concerned with pharmaceutical compositions suitable for administration by inhalation comprising a pharmaceutically acceptable carrier together with a combination of therapeutic agents comprising *(I)* a PDE4 inhibitor selected from the group mentioned hereinbefore that is therapeutically effective when administered by inhalation; together with *(II)* an anti-cholinergic agent comprising a member selected from the group consisting of tiotropium and derivatives thereof that is therapeutically effective when administered by inhalation.

The present invention is further concerned with the above-described pharmaceutical compositions suitable for administration by inhalation comprising a package containing said pharmaceutical compositions for insertion into a device capable of simultaneous or sequential delivery of said pharmaceutical compositions in the form of an aerosol or dry powder dispersion, to a mammal in need of treatment.

The present invention is still further concerned with the combination of said above-mentioned device and said package inserted therein, wherein said device is a metered dose inhaler, or a dry powder inhaler.

## Detailed description of the invention

In its broadest terms, the present invention relates to a combination of two different groups of compounds. Each group of compounds is drawn from a different source, known in the art to have a different mechanism of action and a different therapeutic usefulness. The members of the first group of compounds are PDE4 inhibitors that possess anti-inflammatory activity involving a variety of immune and inflammatory cells selected from the group of compounds mentioned hereinbefore. PDE4 inhibitor activity can result in the elevation of cAMP and inhibition of superoxide production, degranulation, chemotaxis, and tumor necrosis factor alpha (TNF$\alpha$) release in eosinophils, neutrophils and monocytes including neutrophils and eosinophils.

The members of the second group of compounds consist of tiotropium and derivatives thereof known in the art to be anti-cholinergic agents that selectively antagonize $M_3$ muscarinic receptors and to be useful as respiratory agents for treating bronchoconstriction associated with obstructive airways diseases.

Once a component candidate for prospective use in the combination of therapeutic agents of the present invention has been selected from each source consisting of the above-described group of compounds, it must satisfy one further test. It will be appreciated that members of each said group of compounds selected for use in said combination must satisfy the criterion that they be therapeutically effective in the treatment of obstructive airways and other inflammatory diseases as described herein when administered by inhalation. Procedures and assays for determining such therapeutic effectiveness are well known in the art, and some of these are described in detail further herein.

## The PDE4 Inhibitor Component

The present invention concerns combinations of therapeutic agents in which one of the agents is a PDE4 inhibitor, which is broadly defined herein to be one which has therapeutic activity in treating obstructive airways and other inflammatory diseases, especially COPD and asthma, when administered to a patient by means of inhalation. Within the scope of this wide selection of PDE4 inhibitory agents that are suitable for use in the combinations of compounds of the present invention, there is of particular interest PDE4 inhibitors that comprise a compound selected from the group consisting of:

9-cyclopentyl-5,6-dihydro-7-ethyl-3-phenyl-9*H*-pyrazolo[3,4-*c*]-1,2,4-triazolo[4,3-α]pyridine;

9-cyclopenyl-5,6-dihydro-7-ethyl-3-(furan-2-yl)-9*H*-pyrazolo[3,4-*c*]-1,2,4-triazolo[4,3-α]pyridine;

9-cyclopentyl-5,6-dihydro-7-ethyl-3-(2-pyridyl)-9*H*-pyrazolo[3,4-*c*]-1,2,4-triazolo[4,3-α]pyridine;

9-cyclopentyl-5,6-dihydro-7-ethyl-3-(4-pyridyl)-9*H*-pyrazolo[3,4-*c*]-1,2,4-triazolo[4,3-α]pyridine;

9-cyclopentyl-5,6-dihydro-7-ethyl-3-(3-thienyl)-9*H*-pyrazolo[3,4-*c*]-1,2,4-triazolo[4,3-α]pyridine;

3-benzyl-9-cyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-propyl-9*H*-pyrazolo[3,4-*c*]-1,2,4-triazolo[4,3-α]pyridine;

3,9-dicyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-(1-methylcyclohex-1-yl)-9*H*-pyrazolo[3,4-*c*]-1,2,4-triazolo[4,3-α]pyridine;

3-(*tert*-butyl)-9-cyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-(2-methylphenyl)-9*H*-pyrazolo[3,4-*c*]-1,2,4-triazolo[4,3-α]pyridine;

9-cyclopentyl-5,6-dihydro-7-ethyl-3-(2-methoxyphenyl)-9*H*-pyrazolo[3,4-*c*]-1,2,4-triazolo[4,3-α]pyridine;

9-cydopentyl-5,6-dihydro-7-ethyl-3-(thien-2-yl)-9*H*-pyrazolo[3,4-*c*]1,2,4-triazolo[4,3-α]pyridine;

3-(2-chlorophenyl)-9-cyclopentyl-5,6-dihydro-7-ethyl-9*H*-pyrazolo[3,4-*c*]-1,2,4-triazolo[4,3-α]pyridine;

9-cyclopentyl-5,6-dihydro-7-ethyl-3-(2-iodophenyl)-9*H*-pyrazolo[3,4-*c*]-1,2,4-triazolo[4,3-α]pyridine;

9-cyclopentyl-5,6-dihydro-7-ethyl-3-(2-trifluoromethylphenyl)-9*H*-pyrazolo[3,4-*c*]-1,2,4-triazolo[4,3-α]pyridine; and

5,6-dihydro-7-ethyl-9-(4-fluorophenyl)-3-(1-methylcyclohex-1-yl)-9*H*-pyrazolo[3,4-*c*]-1,2,4-triazolo[4,3-α]pyridine.

The above-recited and other preferred embodiments of the PDE4 inhibitor component of the combinations of therapeutic agents of the present invention may be represented by Formulas (1.1.3) through (1.1.33):

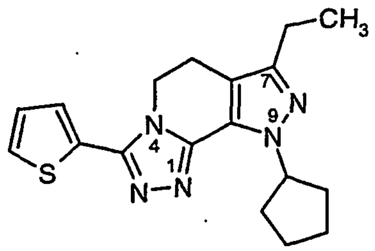

(1.1.3)

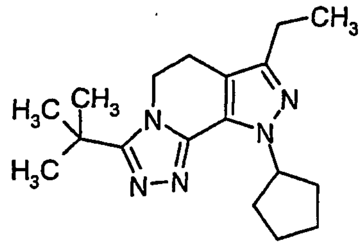

(1.1.4)

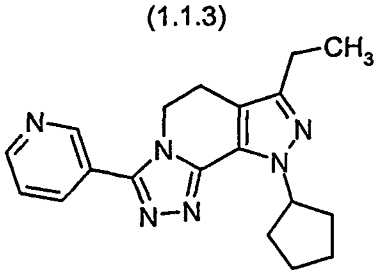

(1.1.5)

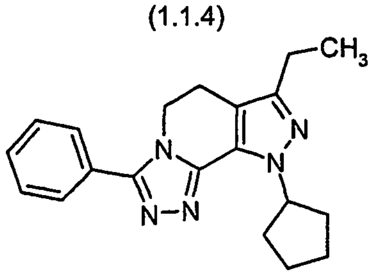

(1.1.6)

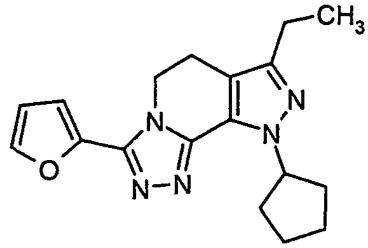

(1.1.7)

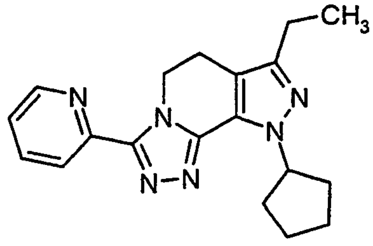

(1.1.8)

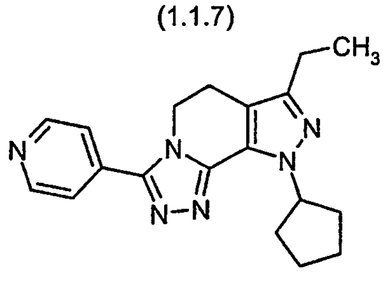

(1.1.9)

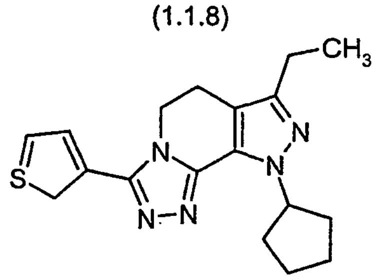

(1.1.10)

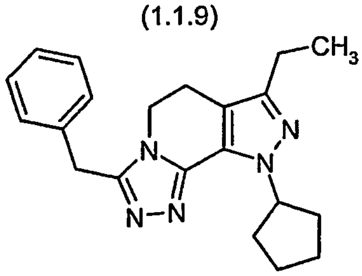

(1.1.11)

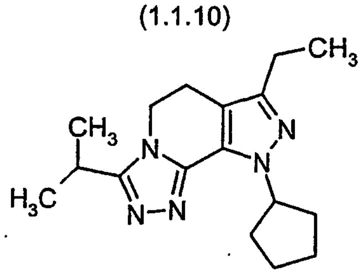

(1.1.12)

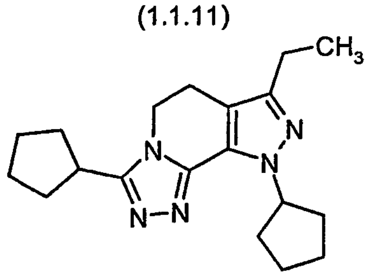

(1.1.13)

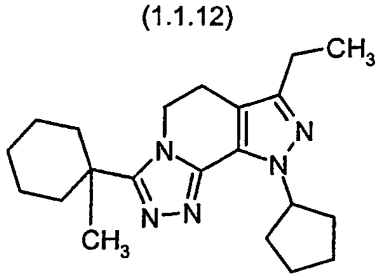

(1.1.14)

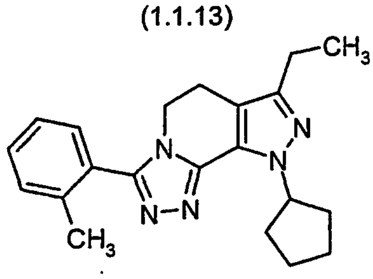

(1.1.15)

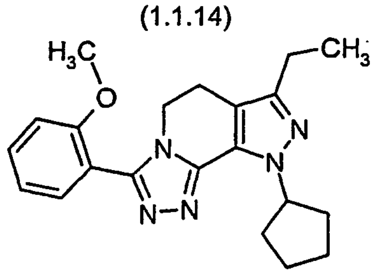

(1.1.16)

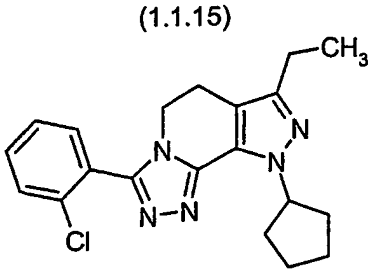

(1.1.17)

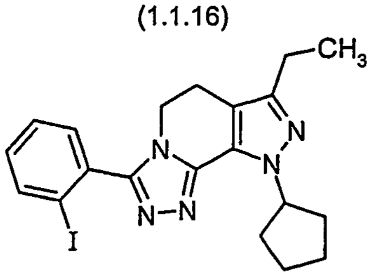

(1.1.18)

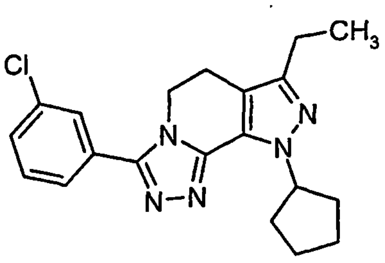

(1.1.29)

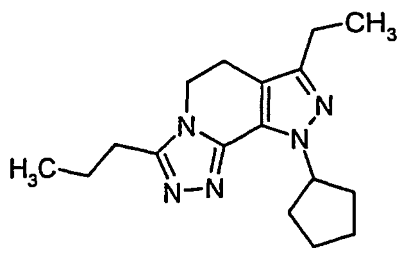

(1.1.30)

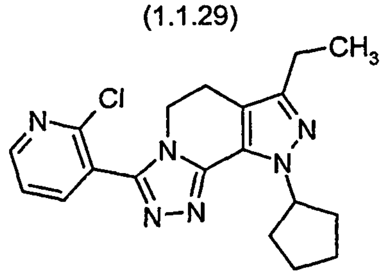

(1.1.31)

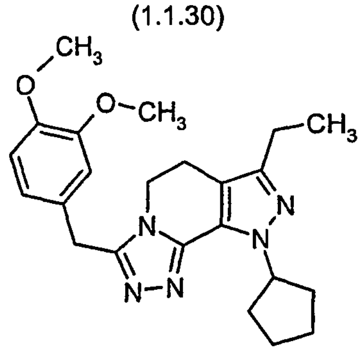

(1.1.32)

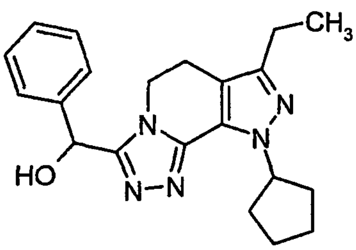

(1.1.33)

**The Anti-Cholinergic Agent Component**

The second component of the combination of therapeutic agents of the present invention comprises an anti-cholinergic agent comprising a member selected from the group consisting of tiotropium and derivatives thereof that is therapeutically effective in the treatment of obstructive airways and other inflammatory diseases as described herein when administered by inhalation. Said anti-cholinergic agent comprising a member selected from the group consisting of tiotropium and derivatives thereof is a compound of Formula (2.1.1):

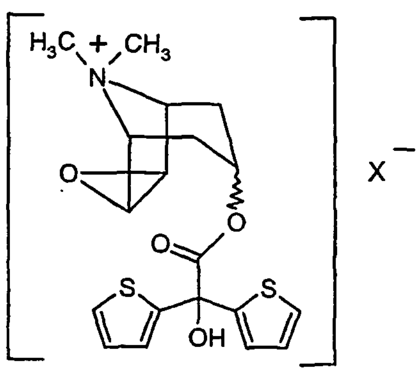

(2.1.1)

wherein $X^-$ is a physiologically acceptable anion. Most commonly, such a phsiologically acceptable anion will

be a halogen anion, but a number of other suitable physiologically acceptable anions would suggest themselves to the medicinal chemist of orginary skill in the art of preparing such therapeutic agents. In preferred embodiments of the subgenus of tiotropium-based anti-cholinergic agents the physiologically acceptable anion is selected from the group consisting of fluoride, $F^-$; chloride, $Cl^-$; bromide, $Br^-$; iodide, $I^-$; methanesulfonate, $CH_3S(=O)_2O^-$; ethanesulfonate, $CH_3CH_2S(=O)_2O^-$; methylsulfate, $CH_3OS(=O)_2O^-$; benzene sulfonate, $C_6H_5S(=O)_2O^-$; *p*-toluenesulfonate, and 4-$CH_3$-$C_6H_5S(=O)_2O^-$. In more preferred embodiments the physiologically acceptable anion is selected from the group consisting of chloride, $Cl^-$; and bromide, $Br^-$. In the most preferred embodiments of the present invention, the physiologically acceptable anion is bromide, $Br^-$.

In addition to the choice of physiologically acceptable anion, it will be appreciated that the anti-cholinergic agent comprising a member selected from the group consisting of tiotropium and derivatives thereof represented by Formula (2.1.1) presents a choice with respect to whether the compounds are 3α or 3β compounds. This choice is represented by the non-specific bond (~) in Formula (2.1.1). The members of the subgenus having an α-configuration are preferred. It is also preferred that the epoxy group have a 6β, 7β-configuration.

Taking into consideration all of the above-described preferred aspects of members of said group consisting of tiotropium and derivatives thereof comprising one of the components of the combination of the present invention, the most preferred species member of said group is tiotropium bromide.. Of particular importance is tiotropium bromide in form of its crystalline monohydrate as disclosed and described in detail in WO 02/30928. Tiotropium bromide may be named as (1α, 2β, 4β, 5α, 7β)-7-[(hydroxydi-2-thienylacetyl)oxy]-9,9-dimethyl-3-oxa-9-azoniatricyclo[3.3.1.$0^{2,4}$]-nonane bromide, or as 6β,7β-epoxy-3β-hydroxy-8-methyl-1α*H*,5α*H*-tropanium bromide, di-2-thienylglycolate. These names are based on

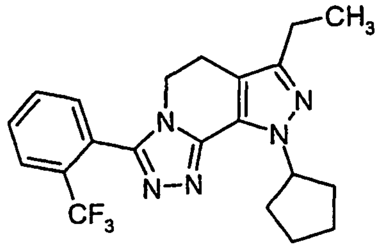

(1.1.19)

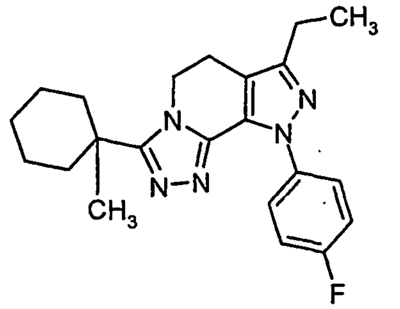

(1.1.20)

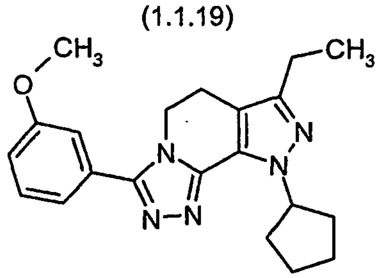

(1.1.21)

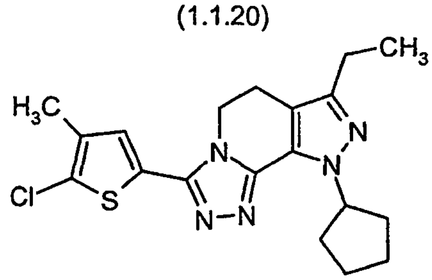

(1.1.22)

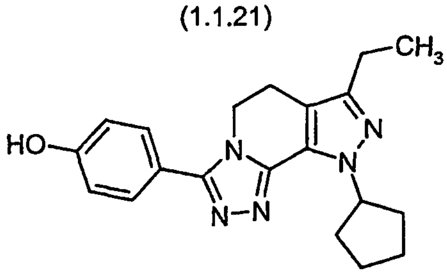

(1.1.23)

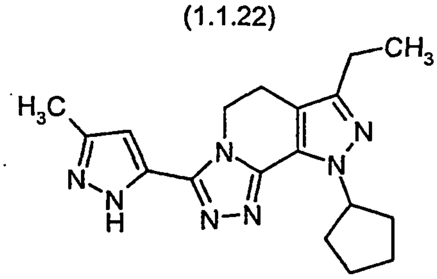

(1.1.24)

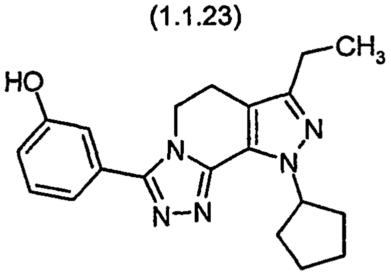

(1.1.25)

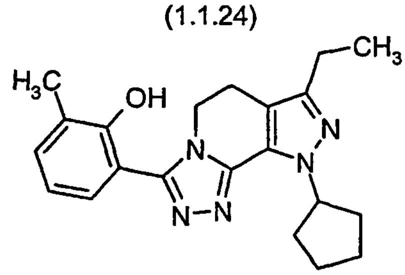

(1.1.26)

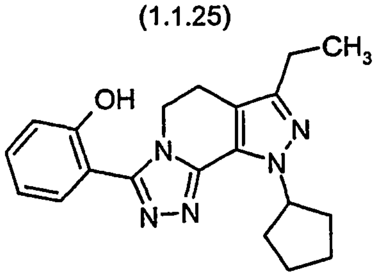

(1.1.27)

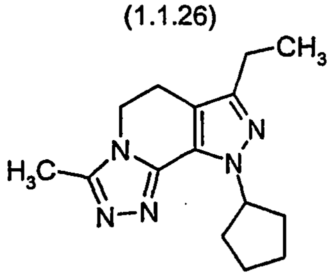

(1.1.28)

different nomenclature systems, but identify the same compound, which is referred to herein as tiotropium bromide. Tiotropium bromide may be represented by either Formula (2.1.2) or by Formula (2.1.3):

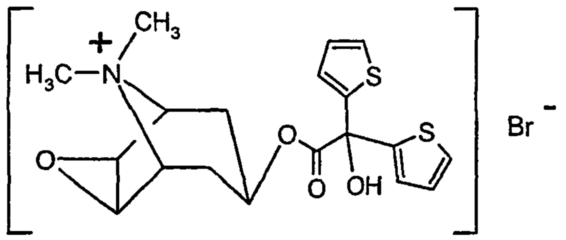

(2.1.2)

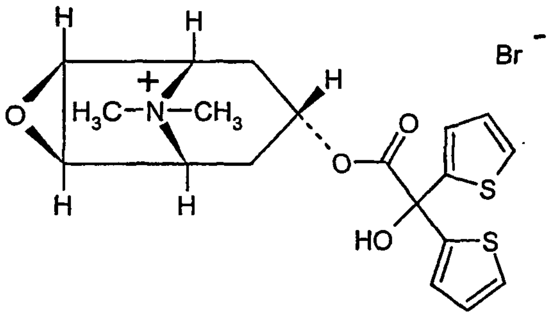

(2.1.3)

The relative stereochemistry of tiotropium bromide may also be shown by Formula (2.1.4):

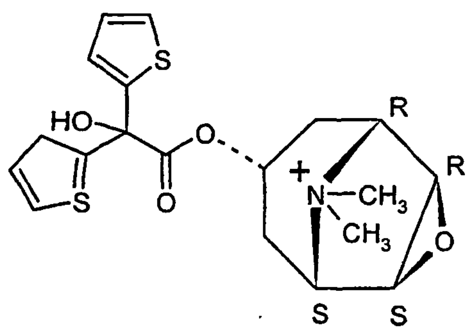

(2.1.4)

**Pharmaceutical Salts and Other Forms**

The individual components of the above-described combinations of compounds of the present invention may be utilized in their final, non-salt form. On the other hand, it is also within the scope of the present invention to utilize those component compounds in the form of their pharmaceutically acceptable salts derived from various organic and inorganic acids and bases in accordance with procedures well known in the art.

Pharmaceutically acceptable salt forms of the combinations of compounds of the present invention are prepared for the most part by conventional means. Where the component compound contains a carboxylic acid group, a suitable salt thereof may be formed by reacting the compound with an appropriate base to provide the corresponding base addition salt. Examples of such bases are alkali metal hydroxides including potassium hydroxide, sodium hydroxide, and lithium hydroxide; alkaline earth metal hydroxides such as barium hydroxide and calcium hydroxide; alkali metal alkoxides, *e.g.*, potassium ethanolate and sodium propanolate; and various organic bases such as piperidine, diethanolamine, and N methylglutamine. Also included are the aluminum salts of the component compounds of the present

invention.

For certain component compounds acid addition salts may be formed by treating said compounds with pharmaceutically acceptable organic and inorganic acids, *e.g.,* hydrohalides such as hydrochloride, hydrobromide, hydroiodide; other mineral acids and their corresponding salts such as sulfate, nitrate, phosphate, *etc.*; and alkyl- and monoarylsulfonates such as ethanesulfonate, toluenesulfonate, and benzenesulfonate; and other organic acids and their corresponding salts such as acetate, tartrate, maleate, succinate, citrate, benzoate, salicylate, ascorbate, *etc.*

Accordingly, the pharmaceutically acceptable acid addition salts of the component compounds of the present invention include, but are not limited to: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, *iso*-butyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate, phthalate.

Further, base salts of the component compounds of the present invention include, but are not limited to aluminum, ammonium, calcium, copper, ferric, ferrous, lithium, magnesium, manganic, manganous, potassium, sodium, and zinc salts. Preferred among the above-recited salts are ammonium; the alkali metal salts sodium and potassium; and the alkaline earth metal salts calcium and magnesium. Salts of the component compounds of the present invention derived from pharmaceutically acceptable organic non-toxic bases include, but are not limited to salts of primary, secondary, and tertiary amines, substituted amines including naturally occurring substituted amines, cyclic amines, and basic ion exchange resins, *e.g.*, arginine, betaine, caffeine, chloroprocaine, choline, *N,N'*-dibenzylethylenediamine (benzathine), dicyclohexylamine, diethanolamine, diethylamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, *iso*-propylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine, and tris-(hydroxymethyl)-methylamine (tromethamine).

Component ompounds of the present invention which comprise basic nitrogen-containing groups may be quaternized with such agents as ($C_1$-$C_4$) alkyl halides, *e.g.,* methyl, ethyl, iso-propyl and *tert*-butyl chlorides, bromides and iodides; di($C_1$-$C_4$) alkyl sulfate, *e.g.,* dimethyl, diethyl and diamyl sulfates; ($C_{10}$-$C_{18}$) alkyl halides, *e.g.,* decyl, dodecyl, lauryl, myristyl and stearyl chlorides, bromides and iodides; and aryl-($C_1$-$C_4$) alkyl halides, *e.g.,* benzyl chloride and phenethyl bromide. Such salts permit,the preparation of both watersoluble and oil-soluble compounds of the present invention.

Among the above-recited pharmaceutical salts those which are preferred include, but are not limited to acetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate, and tromethamine.

The acid addition salts of basic component compounds of the present invention are prepared by contacting the free base form with a sufficient amount of the desired acid to produce the salt in the conventional manner. The free base may be regenerated by contacting the salt form with a base and isolating the free base in the conventional manner. The free base forms differ from their respective salt forms somewhat in certain physical properties such as solubility in polar solvents, but otherwise the salts are equivalent to their respective free base forms for purposes of the present invention.

As indicated, the pharmaceutically acceptable base addition salts of the component compounds of the present invention are formed with metals or amines, such as alkali metals and alkaline earth metals, or organic amines. Preferred metals are sodium, potassium, magnesium, and calcium. Preferred organic amines are *N,N'*-dibenzylethylenediamine, chloroprocaine, choline, diethanolamine, ethylenediamine, N-methyl-D-glucamine, and procaine.

The base addition salts of acidic component compounds of the present invention are prepared by contacting the free acid form with a sufficient amount of the desired base to produce the salt in the conventional manner. The free acid form may be regenerated by contacting the salt form with an acid and isolating the free acid form in the conventional manner. The free acid forms differ from their respective salt forms somewhat in physical properties such as solubility in polar solvents, but otherwise the salts are equivalent to their respective free acid forms for purposes of the present invention.

Multiple salts forms are included within the scope of the present invention where a component compound of the present invention contains more than one group capable of forming such pharmaceutically acceptable salts. Examples of typical multiple salt forms include, but are not limited to bitartrate, diacetate, difumarate, dimeglumine, diphosphate, disodium, and trihydrochloride.

In light of the above, it can be seen that the expression "pharmaceutically acceptable salt" as used herein is

intended to mean an active ingredient comprising component compounds of the present invention utilized in the form of a salt thereof, especially where said salt form confers on said active ingredient improved pharmacokinetic properties as compared to the free form of said active ingredient or some other salt form of said active ingredient utilized previously. The pharmaceutically acceptable salt form of said active ingredient may also initially confer a desirable pharmacokinetic property on said active ingredient which it did not previously possess, and may even positively affect the pharmacodynamics of said active ingredient with respect to its therapeutic activity in the body.

The pharmacokinetic properties of said active ingredient which may be favorably affected include, *e.g.*, the manner in which said active ingredient is transported across cell membranes, which in turn may directly and positively affect the absorption, distribution, biotransformation and excretion of said active ingredient.

A component compound prepared in accordance with the methods described herein can be separated from the reaction mixture in which it is finally produced by any ordinary means known to the chemist skilled in the preparation of organic compounds. Once separated said compound can be purified by known methods. Various methods and techniques can be used as the means for separation and purification, and include, *e.g.*, distillation; recrystallization; column chromatography; ion-exchange chromatography; gel chromatography; affinity chromatography; preparative thin-layer chromatography; and solvent extraction.

**Stereoisomers**

In many cases, a PDEIV-inhibitor or an anti-cholinergic agent that comprises a component part of the combinations of the present invention may be such that its constituent atoms are capable of being arranged in space in two or more different ways, despite having identical connectivities. As a consequence, such an active agent exists in the form of stereoisomers. *Sys-trans* isomerism is but one type of stereoisomerism. Where the stereoisomers are nonsuperimposable mirror images of each other, they are enantiomers which have chirality or handedness, because of the presence of one or more asymmetric carbon atoms in their constituent structure. Enantiomers are optically active and therefore distinguishable because they rotate the plane of polarized light by equal amounts, but in opposite directions.

Where two or more asymmetric carbon atoms are present in an active agent forming a part of a combination of the present invention, there are two possible configurations at each said carbon atom. Where two asymmetric carbon atoms are present, for example, there are four possible stereoisomers. Further, these four possible stereoisomers may be arranged into six possible pairs of stereoisomers that are different from each other. In order for a pair of molecules with more than one asymmetric carbon to be enantiomers, they must have different configurations at every asymmetric carbon. Those pairs that are not related as enantiomers have a different stereochemical relationship referred to as a diastereomeric relationship. Stereoisomers that are not enantiomers are called diastereoisomers, or more commonly, diastereomers.

All of these well known aspects of the stereochemistry of the active agents that form a part of a combination of the present invention are contemplated to be a part of the present invention. Within the scope of the present invention there is thus included active agents that are stereoisomers, and where these are enantiomers, the individual enantiomers, racemic mixtures of said enantiomers, and artificial, *i.e.,* manufactured mixtures containing proportions of said enantiomers that are different from the proportions of said enantiomers found in a racemic mixture. Where an active agent forming part of a combination of the present invention comprises stereoisomers that are diastereomers, there is included within the scope of said active agent the individual diastereomers as well as mixtures of any two or more of said diastereomers in any proportions thereof.

By way of illustration, in the case where there is a single asymmetric carbon atom in an active agent of a combination of the present invention, resulting in the (-)(*R*) and (+)(*S*) enantiomers thereof; there is included within the scope of said active agent all pharmaceutically acceptable salt forms, prodrugs and metabolites thereof which are therapeutically active and useful in treating or preventing the diseases and conditions described further herein. Where an active agent of a combination of the present invention exists in the form of (-)(*R*) and (+)(*S*) enantiomers, there is also included within the scope of said active agent the (+)(*S*) enantiomer alone, or the (-)(*R*) enantiomer alone, in the case where all, substantially all, or a predominant share of the therapeutic activity resides in only one of said enantiomers, and/or unwanted side effects reside in only one of said enantiomers. In the case where there is substantially no difference between the biological activities of both enantiomers, there is further included within the scope of said active agent of a combination of the present invention the (+)(*S*) enantiomer and the (-)(*R*) enantiomer present together as a racemic mixture or as a non-racemic mixture in any ratio of proportionate amounts thereof.

For example, the particular biological activities and/or physical and chemical properties of a pair or set of enantiomers of an active agent of a combination of the present invention, where such exist, may suggest use of said enantiomers in certain ratios to constitute a final therapeutic product. By way of illustration, in the case where there is a pair of enantiomers, they may be employed in ratios such as 90% (R) - 10% (S); 80% (R) - 20% (S); 70% (R) - 30% (S); 60% (R) - 40% (S); 50% (R) - 50% (S); 40% (R) - 60% (S); 30% (R) - 70% (S); 20% (R) - 80% (S); and 10% (R) - 90% (S). After evaluating the properties of the various enantiomers of an active agent of a combination of the present

invention, where such exist, the proportionate amount of one or more of said enantiomers with certain desired properties that will constitute the final therapeutic product can be determined in a straightforward manner.

**Isotopes**

An isotopically-labelled form of an active agent of a combination of the present invention is identical to said active agent but for the fact that one or more atoms of said active agent have been replaced by an atom or atoms having an atomic mass or mass number different from the atomic mass or mass number of said atom which is usually found in nature. Examples of isotopes which are readily available commercially and which can be incorporated into an active agent of a combination of the present invention in accordance with well established procedures, include isotopes of hydrogen, carbon, nitrogen, oxygen, phosphorous, fluorine and chlorine, *e.g.,* $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$, respectively. An active agent of a combination of the present invention, a prodrug thereof, or a pharmaceutically acceptable salt of either which contains one or more of the above-mentioned isotopes and/or other isotopes of other atoms is contemplated to be within the scope of the present invention.

An isotopically-labelled active agent of a combination of the present invention may be used in a number of beneficial ways. For example, an isotopically-labelled active agent of a combination of the present invention, *e.g.*, one in which a radioactive isotope such as $^{3}H$ or $^{14}C$ has been incorporated, will be useful in drug and/or substrate tissue distribution assays. These radioactive isotopes, *i.e.,* tritium, $^{3}H$, and carbon-14, $^{14}C$, are especially preferred for their ease of preparation and eminent detectability. Incorporation of heavier isotopes, *e.g.,* deuterium, $^{2}H$, into an active agent of a combination of the present invention will provide therapeutic advantages based on the greater metabolic stability of said isotopically-labelled compound. Greater metabolic stability translates directly into increased *in vivo* half-life or reduced dosage requirements, which under most circumstances would constitute a preferred embodiment of the present invention. An isotopically-labelled active agent of a combination of the present invention can usually be prepared by carrying out the procedures disclosed in the Synthesis Schemes and related description, Examples, and Preparations herein, substituting a readily available isotopically-labelled reagent for its corresponding non-isotopically-labelled reagent.

Deuterium, $^{2}H$, can also be incorporated into an active agent of a combination of the present invention for the purpose of manipulating the oxidative metabolism of said active agent by way of the primary kinetic isotope effect. The primary kinetic isotope effect is a change of rate for a chemical reaction that results from substitution of isotopic nuclei, which in turn is caused by the change in ground state energies required for covalent bond formation subsequent to said isotopic substitution. Substitution of a heavier isotope will usually result in a lowering of the ground state energy for a chemical bond, thereby causing a reduction in rate for a rate-limiting bond breaking step. If the bond-breaking event occurs on or near a saddle-point region along the coordinate of a multi-product reaction, the product distribution ratios can be altered substantially. By way of illustration, when deuterium is bound to a carbon atom at a non-exchangeable site, rate differences of $k_M/k_D$ = 2-7 are typical. This difference in rate, applied successfully to an oxidatively labile active agent of a combination of the present invention, can dramatically affect the profile of said active agent *in vivo* and result in improved pharmacokinetic properties.

In discovering and developing therapeutic agents, the skilled artisan seeks to optimize pharmacokinetic parameters while retaining desirable *in vitro* properties. It is a reasonable surmise that many compounds with poor pharmacokinetic profiles suffer from a lability to oxidative metabolism. *In vitro* liver microsomal assays now available provide valuable information about the course of this oxidative metabolism, which in turn permits the rational design of deuterated active agents used in a combination of the present invention with improved stability through resistance to such oxidative metabolism. Significant improvements in the pharmacokinetic profiles of an active agent of a combination of the present invention are thereby obtained, and can be expressed quantitatively in terms of increases in *in vivo* half-life (t/2), concentration at maximum therapeutic effect ($C_{max}$), area under the dose response curve (AUC), and F; and in terms of decreases in clearance, dose, and cost-of-goods.

By way of illustration of the above, an active agent of a combination of the present invention which has multiple potential sites for oxidative metabolism, *e.g.*, benzylic hydrogen atoms and hydrogen atoms $\alpha$ to a nitrogen atom, is prepared as a series of analogs in which various combinations of hydrogen atoms are replaced by deuterium atoms so that some, most or all of said hydrogen atoms are replaced with deuterium atoms. Half-life determinations provide an expedient and accurate determination of the extent of improvement in resistance to oxidative metabolism. In this manner it is determined that the half-life of the parent compound can be extended by as much as 100% as the result of such deuterium-for-hydrogen substitution.

Deuterium-for-hydrogen substitution in an active agent of a combination of the present invention can also be used to achieve a favorable alteration in the metabolite profile of the parent compound as a way of diminishing or eliminating unwanted toxic metabolites. For example, where a toxic metabolite arises through an oxidative carbon-hydrogen, C-H, bond scission, the deuterated analog is reasonably expected to greatly diminish or eliminate production of the unwanted metabolite, even in the case where the particular oxidation is not a rate-determining step.

Further information concerning the state of the art with respect to deuterium-for-hydrogen substitution may be

found, *e.g.,* in Hanzlik *et al., J. Org. Chem.* **55** 3992-3997, 1990; Reider *et al., J. Org. Chem.* **52** 3326-3334, 1987; Foster, *Adv. Drug Res.* **14** 1-40, 1985; Gillette *et al.* , *Biochemistry* **33**(10) 2927-2937, 1994; and Jarman *et al.* , *Carcinogenesis* **16**(4) 683-688, 1993.

## Therapeutic Applications and Clinical Endpoints

The description which follows concerns the therapeutic applications to which the combinations of compounds of the present invention may be put, and where applicable an explanation of the clinical endpoints associated with such therapeutic applications. There is also set forth a disclosure of various *in vitro* assays and animal model experiments, which are capable of providing data sufficient to define and demonstrate the therapeutic utility of the combinations of compounds of the present invention.

The therapeutic utility of the combinations of compounds of the present invention is applicable to a patient or subject afflicted with a disease or condition as herein set forth and therefore in need of such treatment. The beneficial results are therapeutic whether administered to animals or humans. As used herein the terms "animal" and "animals" is used merely for the purpose of pointing out human beings as opposed to other members of the animal kingdom. The combinations of compounds of the present invention have therapeutic applicability in the treatment of mammals, and in particular of humans. All of the major subdivisions of the class of mammals (*Mammalia*) are included within the scope of the present invention with regard to being recipients of therapeutic treatment as described herein. Mammals have value as pets to humans and are therefore likely to be subjects of treatment. This applies especially to the canine and feline groups of mammals. Other mammals are valued as domesticated animals and their treatment in accordance with the present invention is likely in view of the adverse economic impact of not treating the diseases and conditions described herein. This applies especially to the equine, bovine, porcine, and ovine groups of mammals.

The types of diseases that may be treated using the novel combinations of compounds of the present invention include but are not limited to asthma; chronic or acute bronchoconstriction; chronic bronchitis; small airways obstruction; emphysema; chronic obstructive pulmonary disease (COPD); COPD that has chronic bronchitis, pulmonary emphysema or dyspnea associated therewith; COPD that is characterized by irreversible, progressive airways obstruction; adult respiratory distress syndrome (ARDS); exacerbation of airways hyper-reactivity consequent to drug therapy; pneumoconiosis; acute bronchitis; acute laryngotracheal bronchitis; arachidic bronchitis; catarrhal bronchitis; croupus bronchitis; dry bronchitis; infectious asthmatic bronchitis; productive bronchitis; staphylococcus or streptococcal bronchitis; vesicular bronchitis; cylindric bronchiectasis; sacculated bronchiectasis; fusiform bronchiectasis; capillary bronchiectasis; cystic bronchiectasis; dry bronchiectasis; follicular bronchiectasis; seasonal allergic rhinitis; perennial allergic rhinitis; purulent or nonpurulent sinusitis; acute or chronic sinusitis; ethmoid, frontal, maxillary, or sphenoid sinusitis; eosinophilia; pulmonary infiltration eosinophilia; Loffler's syndrome; chronic eosinophilic pneumonia; tropical pulmonary eosinophilia; bronchopneumonic aspergillosis; aspergilloma; granulomas containing eosinophils; allergic granulomatous angiitis or Churg-Strauss syndrome; sarcoidosis; alveolitis; chronic hypersensitivity pneumonitis; diffuse interstitial pulmonary fibrosis or interstitial lung fibrosis; and idiopathic pulmonary fibrosis.

## Asthma

One of the most important respiratory diseases treatable with the combinations of therapeutic agents of the present invention is asthma, a chronic, increasingly common disorder encountered worldwide and characterized by intermittent reversible airway obstruction, airway hyper-responsiveness and inflammation. The cause of asthma has yet to be determined, but the most common pathological expression of asthma is inflammation of the airways, which may be significant even in the airways of patients with mild asthma. Based on bronchial biopsy and lavage studies it has been clearly shown that asthma involves infiltration by mast cells, eosinophils, and T-lymphocytes into a patient's airways. Bronchoalveolar lavage (BAL) in atopic asthmatics shows activation of interleukin (IL)-3, IL-4, IL-5 and granulocyte/ macrophage-colony stimulating factor (GM-CSF) that suggests the presence of a T-helper 2 (Th-2)-like T-cell population.

The combinations of therapeutic agents of the present invention are useful in the treatment of atopic and non-atopic asthma. The term "atopy" refers to a genetic predisposition toward the development of type I (immediate) hypersensitivity reactions against common environmental antigens. The most common clinical manifestation is allergic rhinitis, while bronchial asthma, atopic dermatitis, and food allergy occur less frequently. Accordingly, the expression "atopic asthma" as used herein is intended to be synonymous with "allergic asthma", *i.e.,* bronchial asthma which is an allergic manifestation in a sensitized person. The term "non-atopic asthma" as used herein is intended to refer to all other asthmas, especially essential or "true" asthma, which is provoked by a variety of factors, including vigorous exercise, irritant particles, psychologic stresses, *etc.*

The use of the combinations of therapeutic agents of the present invention to treat atopic asthma or non-atopic asthma may be established and demonstrated by models of inhibition of eosinophil activation, and the bronchodilator models described below.

Bronchodilator Activity - cAMP is involved not only in smooth muscle relaxation, but also exerts an overall inhibitory influence on airway smooth muscle proliferation. Airway smooth muscle hypertrophy and hyperplasia can be modulated by cAMP, and these conditions are common morphological features of chronic asthma.

Relaxation of Human Bronchus - Samples of human lungs dissected during surgery for cancer are obtained within 3 days after removal. Small bronchi (inner diameter ≈ 2 to 5 mm) are excised, cut into segments and placed in 2 ml liquid nitrogen storage ampoules filled with fetal calf serum (FCS) containing 1.8M dimethylsulfoxide (DMSO) and 0.1M sucrose as cryoprotecting agents. The ampoules are placed in a polystyrol box (11 x 11 x 22 cm) and slowly frozen at a mean cooling rate of about 0.6°C/m in a freezer maintained at -70°C. After 3-15h the ampoules are transferred into liquid nitrogen (-196°C) where they are stored until use. Before use the tissues are exposed for 30-60m to -70°C before being thawed within 2.5m by placing the ampoules in a 37°C water bath. Thereafter the bronchial segments are rinsed by placing them in a dish containing Krebs-Henseleit solution (μM: NaCl 118, KCl 4.7. $MgSO_4$ 1.2, $CaCl_2$ 1.2, $KH_2P0_4$ 1.2, $NaHCO_3$ 25, glucose 11, EDTA 0.03) at 37°C, cut into rings and suspended in 10 ml organ baths for isometric tension recording under a preload of about 1g. Further increases in tension are induced *via* the application of field stimulation, which is known to induce activation of nerves in the airway sample and generate tension *via* release of acetylcholine and other neurally derived mediators. Concentration-response curves are produced by cumulative additions, each concentration being added when the maximum effect has been produced by the previous concentration. Papaverine (300 μM) is added at the end of the concentration response curve to induce complete relaxation of the bronchial rings. This effect is taken as 100% relaxation.

In the above test model the combinations of therapeutic agents of the present invention produce concentration-related relaxation of human bronchus ring preparations at concentrations in the range of from 0.001 to 1.0 μM with preferred embodiments being active at concentrations in the range of from 5.0 nM to 50 nM.

Suppression of Capsaicin-induced Bronchoconstriction - Male Dunkin-Hartley guinea- pigs (400-800g) having free access to food and water prior to the experiment, are anaesthetized with sodium phenobarbital (100 mg/kg i.p.) and sodium pentobarbital (30 mg/kg i.p.), then paralyzed with gallamine (10 mg/kg i.m.). Animals, maintained at 37°C with a heated pad, controlled by a rectal thermometer, are ventilated via a tracheal cannula (about 8 ml/kg, 1 Hz) with a mixture of air and oxygen (45:55 v/v). Ventilation is monitored at the trachea by a pneumotachograph connected to a differential pressure transducer in line with the respiratory pump. Pressure changes within the thorax are monitored directly *via* an intrathoracic cannula, using a differential pressure transducer so that the pressure difference between the trachea and thorax can be measured and displayed. From these measurements of air-flow and transpulmonary pressure, both airway resistance ($R_1$ $cmH_20$/l/s) and compliance ($Cd_{dyn}$) are calculated with a digital electronic respiratory analyzer for each respiratory cycle. Blood pressure and heart rate are recorded from the carotid artery using a pressure transducer.

When values for basal resistance and compliance are stable, sustained bronchoconstriction is induced by a intravenous infusion of capsaicin. Capsaicin is dissolved in 100% ethanol and diluted with phosphate buffered saline. Test combinations of therapeutic agents of the present invention are administered when the response to capsaicin is stable, which is calculated to be after 2-3 such administrations at 10 min intervals. Reversal of bronchoconstriction is assessed over 1-8 h following either intratracheal or intraduodenal instillation or intravenous bolus injection. Bronchospasmolytic activity is expressed as a % inhibition of the initial, maximal resistance ($R_D$) following the infusion of capsaicin. $ED_{50}$ values represent the dose which causes a 50% reduction of the increase in resistance induced by capsaicin. Duration of action is defined as the time in minutes where bronchoconstriction is reduced by 50% or more. Effects on blood pressure (BP) and heart rate (HR) are characterized by $ED_{20}$ values; *i.e.,* the doses which reduce BP or HR by 20% measured 5m after administration.

In the above test model the combinations of therapeutic agents of the present invention exhibit bronchodilator activity at dosages in the range of from 0.001 to 0.1 mg/kg *i.v.* or 0.1 to 5.0 mg/kg *i.d* or 0.0001 to 0.01 mg/kg *i.t.*

Asthmatic Rat Assay - A test for evaluating the therapeutic impact of the 32combinations of therapeutic agents of the present invention on the symptom of dyspnea, *i.e.,* difficult or labored breathing, utilizes rats obtained from an inbred line of asthmatic rats. Both female (190-250 g) and male (260-400 g) rats are used.

The egg albumin (EA), grade V, crystallized and lyophilized, aluminum hydroxide, and methysergide bimaleate used in this test are commercially available. The challenge and subsequent respiratory readings are carried out in a clear plastic box with internal dimensions of 10x6x4 inches. The top of the box is removable. In use the top is held firmly in place by four clamps, and an airtight seal is maintained by a soft rubber gasket. Through the center of each end of the chamber a nebulizer is inserted *via* an airtight seal and each end of the boxalso has an outlet. A pneumotachograph is inserted into one end of the box and is coupled to a volumetric pressure transducer which is then connected to a dynograph through appropriate couplers. While aerosolizing the antigen, the outlets are open and the pneumotachograph is isolated from the chamber. The outlets are then closed and the pneumotachograph and the chamber are connected during the recording of the respiratory patterns. For challenge, 2 ml of a 3% solution of antigen in saline is placed in each nebulizer and the aerosol is generated with air from a small diaphragm pump operating at 10 psi and a flow rate of 8 l/m.

Rats are sensitized by injecting subcutaneously 1 ml of a suspension containing 1 mg EA and 200 mg aluminum hydroxide in saline. They are used between days 12 and 24 post-sensitization. In order to eliminate the serotonin component of the response, rats are pretreated intravenously 5m prior to aerosol challenge with 3.0 mg/kg of methysergide. Rats are then exposed to an aerosol of 3% EA in saline for exactly 1 m, then respiratory profiles are recorded for a further 30m. The duration of continuous dyspnea is measured from the respiratory recordings.

Test combinations of therapeutic agents of the present invention are generally administered either orally 1-4h prior to challenge or intravenously 2m prior to challenge. The combinations of compounds are either dissolved in saline or 1% methocel, or suspended in 1 % methocel. The volume of test compound injected is 1 ml/kg (intravenously) or 10 ml/kg (orally). Prior to oral treatment rats are starved overnight. The activity of the rats is determined on the basis of their ability to decrease the duration of symptoms of dyspnea in comparison to a group of vehicle-treated controls. Test the combinations of therapeutic agents of the present invention are evaluated over a series of doses and an $ED_{50}$ is derived that is defined as the dose (mg/kg) which will inhibit the duration of symptoms by 50%.

Pulmonary Mechanics in Trained, Conscious Squirrel Monkeys - The ability of the combinations of therapeutic agents of the present invention to inhibit *Ascaris* antigen induced changes in the respiratory parameters, *e.g.*, airway resistance, of squirrel monkey test subjects is evaluated in this method. This test procedure involves placing trained squirrel monkeys in chairs in aerosol exposure chambers. For control purposes, pulmonary mechanics measurements of respiratory parameters are recorded for a period of about 30m to establish each monkey's normal control values for that day. For oral administration, combinations of compounds of the present invention are dissolved or suspended in a 1% methocel solution (methylcellulose, 65HG, 400 cps) and given in a volume of 1 ml/kg of body weight.

Following challenge, each minute of data is calculated as a percent change from control values for each respiratory parameter including airway resistance ($R_L$) and dynamic compliance ($C_{dyn}$). The results for each test compound are subsequently obtained for a minimum period of 60m post-challenge, which are then compared to previously obtained historical baseline control values for the particular monkey involved. Further, the overall values for 60m post-challenge for each monkey, *i.e.,* historical baseline values and test values, are averaged separately and are used to calculate the overall percent inhibition of *Ascaris* antigen response by the test compound. For statistical analysis of the results, the paired t-test is used.

Prevention of Induced Bronchoconstriction in Allergic Sheep - A procedure for testing the therapeutic activity of the combinations of therapeutic agents of the present invention in preventing bronchoconstriction is described below. It is based on the discovery of a certain breed of allergic sheep with a known sensitivity to a specific antigen, *Ascaris suum,* that responds to inhalation challenge with acute as well as late bronchial responses. The progress of both the acute and the late bronchial responses over time approximates the time course observed in humans with asthma; moreover, the pharmacological modification of both the acute and late responses is similar to that found in man. The responses of these sheep to the antigen challenge is observed for the most part in their large airways, which makes it possible to monitor the effects as changes in lung resistance, *i.e.,* specific lung resistance

Adult sheep with a mean weight of 35 kg (range: 18-50 kg) are used. All animals used meet two criteria: 1) they have a natural cutaneous reaction to 1:1000 or 1:10000 dilutions of *Ascaris suum* extract, and 2) they have previously responded to inhalation challenge with *Ascaris suum* with both an acute bronchoconstriction and a late bronchial obstruction. See Abraham *et al., Am. Rev. Resp. Dis.* **128** 839-844,1983.

The unsedated sheep are restrained in a cart in the prone position with their heads immobilized. After topical anesthesia of the nasal passages with 2% lidocaine solution, a balloon catheter is advanced through one nostril into the lower esophagus. The animals are then intubated with a cuffed endotracheal tube through the other nostril using a flexible fiberoptic bronchoscope as a guide. Pleural pressure is estimated with the esophageal balloon catheter (filled with 1 ml of air), which is positioned such that inspiration produces a negative pressure deflection with clearly discernible cardiogenic oscillations. Lateral pressure in the trachea is measured with a sidehole catheter (inner dimensions: 2.5 mm) advanced through and positioned distal to the tip of the nasotracheal tube. Transpulmonary pressure, *i.e.*, the difference between tracheal pressure and pleural pressure, is measured with a differential pressure transducer. Testing of the pressure transducer catheter system reveals no phase shift between pressure and flow to a frequency of 9 Hz. For the measurement of pulmonary resistance ($R_L$), the maximal end of the nasotracheal tube is connected to a pneumotachograph. The signals of flow and transpulmonary pressure are recorded on an oscilloscope which is linked to a computer for on-line calculation of $R_L$ from transpulmonary pressure, respiratory volume obtained by integration, and flow. Analysis of 10-15 breaths is used for the determination of $R_L$. Thoracic gas volume ($V_{tg}$) is measured in a body plethysmograph, to obtain pulmonary resistance ($SR_L = R_L \cdot V_{tg}$).

Aerosols of *Ascaris suum* extract (1:20) are generated using a disposable medical nebulizer which produces an aerosol with a mass median aerodynamic diameter of 6.2 μm (geometric standard deviation, 2.1) as determined by an electric size analyzer. The output from the nebulizer is directed into a plastic T-piece, one end of which is attached to the nasotracheal tube, and the other end of which is connected to the inspiratory part of a conventional respirator. The aerosol is delivered at a total volume of 500 ml at a rate of 20 ml per minute. Thus, each sheep receives an equivalent dose of antigen in both placebo and drug trials

Prior to antigen challenge, baseline measurements of $SR_L$ are obtained, infusion of the test compound is started 1h prior to challenge, the measurement of $SR_L$ is repeated, and the sheep then undergoes inhalation challenge with *Ascaris suum* antigen. Measurements of $SR_L$ are obtained immediately after antigen challenge and at 1, 2, 3, 4, 5, 6, 6.5, 7, 7.5, and 8h after antigen challenge. Placebo and drug tests are separated by at least 14 days. In a further study, sheep are given a bolus dose of the test compound followed by an infusion of the test compound for 0.5-1 h prior to *Ascaris* challenge and for 8h after *Ascaris* challenge as described above. A Kruskal-Wallis one way ANOVA test is used to compare the acute immediate responses to antigen and the peak late response in the controls and the drug treated animals.

Another useful assay, based on the use of primates, is that described in Turner *et al.,* "Characterization of a primate model of asthma using anti-allergy/anti-asthma agents," *Inflammation Research* **45** 239-245, 1996.

Anti-inflammatory Activity - The anti-inflammatory activity of the combinations of therapeutic agents of the present invention is demonstrated by the inhibition of eosinophil activation. In this assay blood samples (50ml) are collected from non-atopic volunteers with eosinophil numbers ranging between 0.06 and 0.47 x $10^9$ $L^{-1}$. Venous blood is collected into centrifuge tubes containing 5 ml trisodium citrate (3.8%, pH 7.4).

The anticoagulated blood is diluted (1:1, v:v) with phosphate-buffered saline (PBS, containing neither calcium nor magnesium) and is layered onto 15 ml isotonic Percoll (density 1.082 - 1.085 g/ml, pH 7.4), in a 50 ml centrifuge tube. Following centrifugation (30 minutes, 1000 x g, 20°C), mononuclear cells at the plasma/Percoll interface are aspirated carefully and discarded.

The neutrophil/eosinophil/erythrocyte pellet (*ca*. 5 ml by volume) is gently resuspended in 35 ml of isotonic ammonium chloride solution ($NH_4Cl$, 155mM; $KHC0_3$, 10mM; EDTA. 0.1mM; 0-4°C). After 15 min, cells are washed twice (10 min, 400 x g, 4°C) in PBS containing fetal calf serum (2%, FCS).

A magnetic cell separation system is used to separate eosinophils and neutrophils. This system is able to separate cells in suspension according to surface markers, and comprises a permanent magnet, into which is placed a column that includes a magnetizable steel matrix. Prior to use, the column is equilibrated with PBS/FCS for 1 hour and then flushed with ice-cold PBS/FCS on a retrograde basis *via* a 20 ml syringe. A 21 G hypodermic needle is attached to the base of the column and 1-2 ml of ice cold buffer are allowed to efflux through the needle.

Following centrifugation of granulocytes, supernatant is aspirated and cells are gently resuspended with 100μl magnetic particles (anti-CD16 monoclonal antibody, conjugated to superparamagnetic particles). The eosinophil/neutrophil/anti-CD16 magnetic particle mixture is incubated on ice for 40 minutes and then diluted to 5 ml with ice-cold PBS/FCS. The cell suspension is slowly introduced into the top of the column and the tap is opened to allow the cells to move slowly into the steel matrix. The column is then washed with PBS/FCS (35ml), which is carefully added to the top of the column so as not to disturb the magnetically labeled neutrophils already trapped in the steel matrix. Non-labeled eosinophils are collected in a 50ml centrifuge tube and washed (10 minutes, 400 x g, 4°C). The resulting pellet is resuspended in 5 ml Hank's balanced salt solution (HBSS) so that cell numbers and purity can be assessed prior to use. The separation column is removed from the magnet and the neutrophil fraction is eluted. The column is then washed with PBS (50ml) and ethanol (absolute), and stored at 4°C.

Total cells are counted with a micro cell counter. One drop of lysogenic solution is added to the sample, which after 30s is recounted to assess contamination with erythrocytes. Cytospin smears are prepared on a Shandon Cytospin 2 cytospinner (100 μl samples, 3 minutes, 500 rpm). These preparations are stained and differential cell counts are determined by light microscopy, examining at least 500 cells. Cell viability is assessed by exclusion of trypan blue.

Eosinophils are diluted in HBSS and pipetted into 96 well microtiter plates (MTP) at 1-10 x $10^3$ cells/well. Each well contains a 200 μl sample comprising: 100 μl eosinophil suspension; 50 μl HBSS; 10 μl lucigenin; 20 μl activation stimulus; and 20 μl test compound.

The samples are incubated with test compound or vehicle for 10m prior to addition of an activation stimulus fMLP (10 μM) dissolved in dimethylsulfoxide and thereafter diluted in buffer, such that the highest solvent concentration used is 1% (at 100 μM test compound). MTPs are agitated to facilitate mixing of the cells and medium, and the MTP is placed into a luminometer. Total chemiluminescence and the temporal profile of each well is measured simultaneously over 20m and the results expressed as arbitrary units, or as a percentage of fMLP-induced chemiluminescence in the absence of test compound. Results are fitted to the Hill equation and $IC_{50}$ values are calculated automatically.

The combinations of therapeutic agents of the present invention are active in the above test method at concentrations in the range of from 0.0001 μM to 0.5 μM, with preferred embodiments being active at concentrations in the range of from 0.5 nM to 20 nM.

From the above it may be seen that the combinations of therapeutic agents of the present invention are useful for the treatment of inflammatory or obstructive airways diseases or other conditions involving airways obstruction. In particular they are useful for the treatment of bronchial asthma.

In view of their anti-inflammatory activity and their influence on airways hyper-reactivity, the combinations of therapeutic agents of the present invention are useful for the treatment, in particular prophylactic treatment, of obstructive or inflammatory airways diseases. Thus, by continued and regular administration over prolonged periods of time the

combinations of compounds of the present invention are useful in providing advance protection against the recurrence of bronchoconstriction or other symptomatic attack consequential to obstructive or inflammatory airways diseases. The combinations of compounds of the present invention are also useful for the control, amelioration or reversal of the basal status of such diseases.

Having regard to their bronchodilator activity the combinations of therapeutic agents of the present invention are useful as bronchodilators, *e.g.*, in the treatment of chronic or acute bronchoconstriction, and for the symptomatic treatment of obstructive or inflammatory airways diseases.

The words "treatment" and "treating" as used throughout the present specification and claims in relation to obstructive or inflammatory airways diseases are to be understood, accordingly, as embracing both prophylactic and symptomatic modes of therapy.

In light of the above description, it may be seen that the present invention also relates to a method for the treatment of airways hyper-reactivity in mammals; to a method of effecting bronchodilation in mammals; and in particular, to a method of treating obstructive or inflammatory airways diseases, especially asthma, in a mammal subject in need thereof, which method comprises administering to said subject mammal an effective amount of a combination of therapeutic agents of the present invention.

Obstructive or inflammatory airways diseases to which the present invention applies include asthma; pneumoconiosis; chronic eosinophilic pneumonia; chronic obstructive airways or pulmonary disease (COAD or COPD); and adult respiratory distress syndrome (ARDS), as well as exacerbation of airways hyper-reactivity consequent to other drug therapy, *e.g.,* aspirin or β-agonist therapy.

The combinations of therapeutic agents of the present invention are useful in the treatment of asthma of whatever type, etiology, or pathogenesis; including intrinsic asthma attributed to pathophysiologic disturbances, extrinsic asthma caused by some factor in the environment, and essential asthma of unknown or inapparent cause. The combinations of therapeutic agents of the present invention are useful in the treatment of allergic (atopic/bronchial/IgE-mediated) asthma; and they are useful as well in the treatment of non-atopic asthma, including *e.g.* bronchitic, emphysematous, exercise-induced, and occupational asthma; infective asthma that is a sequela to microbial, especially bacterial, fungal, protozoal, or viral infection; and other non-allergic asthmas, *e.g.,* incipient asthma (wheezy infant syndrome).

The combinations of therapeutic agents of the present invention are further useful in the treatment of pneumoconiosis of whatever type, etiology, or pathogenesis; including, *e.g.,* aluminosis (bauxite workers' disease); anthracosis (miners' asthma); asbestosis (steam-fitters' asthma); chalicosis (flint disease); ptilosis caused by inhaling the dust from ostrich feathers; siderosis caused by the inhalation of iron particles; silicosis (grinders' disease); byssinosis (cotton-dust asthma); and talc pneumoconiosis.

## Chronic Obstructive Pulmonary Disease (COPD)

The combinations of therapeutic agents of the present invention are still further useful in the treatment of COPD or COAD including chronic bronchitis, pulmonary emphysema or dyspnea associated therewith. COPD is characterized by irreversible, progressive airways obstruction. Chronic bronchitis is associated with hyperplasia and hypertrophy of the mucus secreting glands of the submucosa in the large cartilaginous airways. Goblet cell hyperplasia, mucosal and submucosal inflammatory cell infiltration, edema, fibrosis, mucus plugs and increased smooth muscle are all found in the terminal and respiratory bronchioles. The small airways are known to be a major site of airway obstruction. Emphysema is characterized by destruction of the alveolar wall and loss of lung elasticity. A number of risk factors have also been identified as linked to the incidence of COPD. The link between tobacco smoking and COPD is well established. Other risk factors include exposure to coal dust and various genetic factors. See Sandford *et al.,* "Genetic risk factors for chronic obstructive pulmonary disease," *Eur. Respir. J.* **10** 1380-1391, 1997. The incidence of COPD is increasing and it represents a significant economic burden on the populations of the industrialized nations. COPD also presents itself clinically with a wide range of variation from simple chronic bronchitis without disability to patients in a severely disabled state with chronic respiratory failure.

COPD is characterized by inflammation of the airways, as is the case with asthma, but the inflammatory cells that have been found in the bronchoalveolar lavage fluid and sputum of patients neutrophils rather than eosinophils. Elevated levels of inflammatory mediators are also found in COPD patients, including IL-8, $LTB_4$, and TNF-α, and the surface epithelium and sub-epithelium of the bronchi of such patients has been found to be infiltrated by T-lymphocytes and macrophages. Symptomatic relief for COPD patients can be provided by the use of β-agonist and anticholinergic bronchodilators, but the progress of the disease remains unaltered. COPD has been treated using theophylline, but without much success, even though it reduces neutrophil counts in the sputum of COPD patients. Steroids have also failed to hold out much promise as satisfactory treatment agents in COPD.

Accordingly, the use of the combinations of therapeutic agents of the present invention to treat COPD and its related and included obstructed airways diseases, represents a significant advance in the art. The present invention is not limited to any particular mode of action or any hypothesis as to the way in which the desired therapeutic objectives

have been obtained by utilizing the combinations of therapeutic agents of the present invention.

**Bronchitis and Bronchiectasis**

In accordance with the particular and diverse inhibitory activities described above that are possessed by the combinations of therapeutic agents of the present invention, they are useful in the treatment of bronchitis of whatever type, etiology, or pathogenesis, including, *e.g.*, acute bronchitis which has a short but severe course and is caused by exposure to cold, breathing of irritant substances, or an acute infection; acute laryngotracheal bronchitis which is a form of nondiphtheritic croup; arachidic bronchitis which is caused by the presence of a peanut kernel in a bronchus; catarrhal bronchitis which is a form of acute bronchitis with a profuse mucopurulent discharge; chronic bronchitis which is a long-continued form of bronchitis with a more or less marked tendency to recurrence after stages of quiescence, due to repeated attacks of acute bronchitis or chronic general diseases, characterized by attacks of coughing, by expectoration either scanty or profuse, and by secondary changes in the lung tissue; croupus bronchitis which is characterized by violent cough and paroxysms of dyspnea; dry bronchitis which is characterized by a scanty secretion of tough sputum; infectious asthmatic bronchitis which is a syndrome marked by the development of symptoms of bronchospasm following respiratory tract infections in persons with asthma; productive bronchitis which is bronchitis associated with a productive cough; staphylococcus or streptococcal bronchitis which are caused by staphylococci or streptococci; and vesicular bronchitis in which the inflammation extends into the alveoli, which are sometimes visible under the pleura as whitish-yellow granulations like millet seeds.

Bronchiectasis is a chronic dilatation of the bronchi marked by fetid breath and paroxysmal coughing with the expectoration of mucopurulent matter. It may affect the tube uniformly, in which case it is referred to as cylindric bronchiectasis, or it may occur in irregular pockets, in which case it is called sacculated bronchiectasis. When the dilated bronchial tubes have terminal bulbous enlargements, the term fusiform bronchiectasis is used. In those cases where the condition of dilatation extends to the bronchioles, it is referred to as capillary bronchiectasis. If the dilatation of the bronchi is spherical in shape, the condition is referred to as cystic bronchiectasis. Dry bronchiectasis occurs where the infection involved is episodic and it may be accompanied by hemoptysis, the expectoration of blood or of blood-stained sputum. During quiescent periods of dry bronchiectasis, the coughing which occurs is nonproductive. Follicular bronchiectasis is a type of bronchiectasis in which the lymphoid tissue in the affected regions becomes greatly enlarged, and by projection into the bronchial lumen, may seriously distort and partially obstruct the bronchus. Accordingly, the combinations of therapeutic agents of the present invention are useful in the beneficial treatment of the various above-described types of bronchiectasis as a direct result of their inhibition of PDE4 isozymes.

The utility of the combinations of therapeutic agents of the present invention as bronchodilaors or bronchospasmolytic agents for treating bronchial asthma, chronic bronchitis and related diseases and disorder described herein, is demonstrable through the use of a number of different *in vivo* animal models known in the art, including those described in the paragraphs below..

Bronchospasmolytic Activity *In Vitro* - The ability of the combinations of therapeutic agents of the present invention to cause relaxation of guinea-pig tracheal smooth muscle is demonstrated in the following test procedure. Guinea-pigs (350-500 g) are killed with sodium pentothal (100 mg/kg i.p.). The trachea is dissected and a section 2-3 cm in length is excised. The trachea is transected in the transverse plane at alternate cartilage plates so as to give rings of tissue 3-5 mm in depth. The proximal and distal rings are discarded. Individual rings are mounted vertically on stainless steel supports, one of which is fixed at the base of an organ bath, while the other is attached to an isometric transducer. The rings are bathed in Krebs solution (composition μM: $NaHCO_3$ 25; NaCl 113; KCl 4.7; $MgSO_4 \cdot 7H_2O$ 1.2; $KH_2PO_4$ 1.2; $CaCl_2$ 2.5; glucose 11.7) at 37°C and gassed with $O_2/CO_2$ (95:5, v/v). Rings prepared in this manner, preloaded to 1 g, generate spontaneous tone and, after a period of equilibration (45-60m), relax consistently on addition of spasmolytic drugs. To ascertain spasmolytic activity, test combinations of therapeutic agents of the present invention are dissolved in physiological saline and added in increasing quantities to the organ bath at 5m intervals to provide a cumulative concentration-effect curve.

In the above test model, combinations of therapeutic agents of the present invention produce concentration-related relaxation of guinea-pig tracheal ring preparations at concentrations in the range of from 0.001 to 1.0 μM.

Suppression of Airways Hyper-reactivity in PAF-treated Animals - guinea-pigs are anesthetized and prepared for recording of lung function as described under "Suppression of bombesin-induced bronchoconstriction" further above. Intravenous injection of low dose histamine (1.0-1.8 μg/kg) establishes airways sensitivity to spasmogens. Following infusion of PAF (platelet activating factor) over 1h (total dose = 600 ng/kg), injection of low dose bombesin 20m after cessation of infusion reveals development of airways hyper-reactivity, which is expressed as the paired difference between the maximal response amplitude before and after PAF exposure. Upon administration of the combinations of therapeutic agents of the present invention by infusion during PAF exposure at dosages in the range of from 0.01 to 0.1 mg/kg, suppression of PAF-induced hyper-reactivity is obtained.

## Allergic and Other Types of Rhinitis; Sinusitis

Allergic rhinitis is characterized by nasal obstruction, itching, watery rhinorrhea, sneezing and occasional anosmia. Allergic rhinitis is divided into two disease categories, seasonal and perennial, in which the former is attributed to pollen or outdoor mould spores, while the latter is attributed to common allergens such as house dust mites, animal danders, and mould spores. Allergic rhinitis generally exhibits an early phase response and a late phase response. The early phase response is associated with mast cell degranulation, while the late phase response is characterized by infiltration of eosinophils, basophils, monocytes, and T-lymphocytes. A variety of inflammatory mediators is also released by these cells, all of which may contribute to the inflammation exhibited in the late phase response.

A particularly prevalent form of seasonal allergic rhinitis is hay fever, which is marked by acute conjunctivitis with lacrimation and itching, swelling of the nasal mucosa, nasal catarrh, sudden attacks of sneezing, and often with asthmatic symptoms. The combinations of compounds of the present invention are especially useful in the beneficial treatment of hay fever.

Other types of rhinitis for which the combinations of therapeutic agents of the present invention may be used as therapeutic agents include acute catarrhal rhinitis which is a cold in the head involving acute congestion of the mucous membrane of the nose, marked by dryness and followed by increased mucous secretion from the membrane, impeded respiration through the nose, and some pain; atrophic rhinitis which is a chronic form marked by wasting of the mucous membrane and the glands; purulent rhinitis which is chronic rhinitis with the formation of pus; and vasomotor rhinitis which is a non-allergic rhinitis in which transient changes in vascular tone and permeability with the same symptoms as allergic rhinitis, are brought on by such stimuli as mild chilling, fatigue, anger, and anxiety.

There is a recognized link between allergic rhinitis and asthma. Allergic rhinitis is a frequent accompaniment to asthma, and it has been demonstrated that treating allergic rhinitis will improve asthma. Epidemiologic data has also been used to show a link between severe rhinitis and more severe asthma. For example, the compound D-22888, under preclinical development for the treatment of allergic rhinitis, has been shown to exhibit a strong anti-allergic affect and to inhibit rhinorrhea in the antigen-challenged pig. See, Marx *et* 30 *al* "D-22888 - a new PDE4 inhibitor for the treatment of allergic rhinitis and other allergic disorders," *J. Allergy Clin. ImmunoL* 99 S444, 1997.

Sinusitis is related to rhinitis in terms of anatomical proximity as well as a shared etiology and pathogenesis in some cases. Sinusitis is the inflammation of a sinus and this condition may be purulent or nonpurulent, as well as acute or chronic. Depending upon the sinus where the inflammation is located, the condition is known as ethmoid, frontal, maxillary, or sphenoid sinusitis. The ethmoidal sinus is one type of paranasal sinus, located in the ethmoid bone. The frontal sinus is one of the paired paranasal sinuses located in the frontal bone. The maxillary sinus is one of the paired paranasal sinuses located in the body of the maxilla. Accordingly, the combinations of therapeutic agents of the present invention are useful in the beneficial treatment of acute or chronic sinusitis, but especially of chronic sinusitis.

## Eosinophil-related Disorders

The ability of the combinations of compounds of the present invention to inhibit eosinophil activation as part of their overall anti-inflammatory activity has been described above. Accordingly, the combinations of compounds of the present invention are useful in the therapeutic treatment of eosinophil-related disorders. Such disorders include eosinophilia, which is the formation and accumulation of an abnormally large number of eosinophils in the blood. The name of the disorder derives from "eosin", a rose-colored stain or dye comprising a bromine derivative of fiuorescein which readily stains "eosinophilic leukocytes" in the blood of patients who are thus readily identified. A particular eosinophilic disorder that can be treated in accordance with the present invention is pulmonary infiltration eosinophilia, which is characterized by the infiltration of the pulmonary parenchyma by eosinophils. This disorder includes especially Loffler's syndrome, which is a condition characterized by transient infiltrations of the lungs, accompanied by cough, fever, dyspnea, and eosinophilia.

Other eosinophilic disorders include chronic eosinophilic pneumonia, which is a chronic interstitial lung disease characterized by cough, dyspnea, malaise, fever, night sweats, weight loss, eosinophilia, and a chest film revealing non-segmental, non-migratory infiltrates in the lung periphery; tropical pulmonary eosinophilia, which is a subacute or chronic form of occult filariasis, usually involving *Brugia malayi, Wuchereria bancrofti,* or filariae that infect animals, occurs in the tropics, and is characterized by episodic nocturnal wheezing and coughing, strikingly elevated eosinophilia, and diffuse reticulonodular infiltrations of the lungs; bronchopneumonic aspergillosis, which is an infection of the bronchi and lungs by *Aspergillus* fungi resulting in a diseased condition marked by inflammatory granulomatous lesions in the nasal sinuses and lungs, but also in the skin, ear, orbit, and sometimes in the bones and meninges, and leading to aspergilloma, the most common type of fungus ball formed by colonization of *Aspergillus* in a bronchus or lung cavity.

The term "granulomatous" means containing granulomas, and the term "granuloma" refers to any small nodular delimited aggregation of mononuclear inflammatory cells or such a collection of modified macrophages resembling epithelial cells, usually surrounded by a rim of lymphocytes, with fibrosis commonly seen around the lesion. Some

granulomas contain eosinophils. Granuloma formation represents a chronic inflammatory response initiated by various infectious and noninfectious agents. A number of such granulomatous conditions are treatable using combinations of compounds of the present invention, e.g., allergic granulomatous angiitis, also called Churg-Strauss syndrome, which is a form of systemic necrotizing vasculitis in which there is prominent lung involvement, generally manifested by eosinophilia, granulomatous reactions, and usually severe asthma. A related disorder is polyarteritis nodosa (PAN), which is marked by multiple inflammatory and destructive arterial lesions and is a form of systemic necrotizing vasculitis involving the small and medium-sized arteries with signs and symptoms resulting from infarction and scarring of the affected organ system, in particular the lungs. Other eosinophil-related disorders which may be treated in accordance with the present invention are those affecting the airways which are induced or occasioned by a reaction to a therapeutic agent unrelated to any combinations of compounds of the present invention.

## Pharmaceutical Compositions, Formulations, and Delivery Devices

The description which follows concerns the manner in which the combinations of compounds of the present invention, together with other therapeutic agents or non-therapeutic agents where these are desired, are combined with what are for the most part conventional pharmaceutically acceptable carriers to form dosage forms suitable for administration by inhalation to any given patient, as well as appropriate to the disease, disorder, or condition for which any given patient is being treated.

The pharmaceutical compositions of the present invention comprise any one or more of the above-described combinations of compounds of the present invention, or a pharmaceutically acceptable salt thereof as also above-described, together with a pharmaceutically acceptable carrier in accordance with the properties and expected performance of such carriers for administration by inhalation, which are well-known in the pertinent art.

The amount of active ingredient that may be combined with the carrier materials will vary depending upon the host and disease or condition being treated. It should be understood, however, that a specific dosage and treatment regimen for any particular patient will depend upon a variety of factors, including the activity of the specific component compounds employed, the age, body weight, general health, sex, diet, time of administration, rate of excretion, and the judgment of the treating physician and the severity of the particular disease being treated.

The above-described component compounds of the present invention may be utilized in the form of acids, esters, or other chemical classes of compounds to which the components described belong. It is also within the scope of the present invention to utilize those component compounds in the form of pharmaceutically acceptable salts derived from various organic and inorganic acids and bases in accordance with procedures described in detail above and well known in the art. An active ingredient comprising a component compound of the present invention is often utilized in the form of a salt thereof, especially where said salt form confers on said active ingredient improved pharmacokinetic properties as compared to the free form of said active ingredient or some other salt form of said active ingredient utilized previously. The pharmaceutically acceptable salt form of said active ingredient may also initially confer a desirable pharmacokinetic property on said active ingredient which it did not previously possess, and may even positively affect the pharmacodynamics of said active ingredient with respect to its therapeutic activity in the body.

Specific preferred salt forms of specific preferred component compounds of the present invention have already been described above. In more general terms, of the pharmaceutical salts recited further above, those which are preferred include, but are not limited to acetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate, and tromethamine.

Multiple salts forms are included within the scope of the present invention where a component compound of the present invention contains more than one group capable of forming such pharmaceutically acceptable salts. Examples of typical multiple salt forms include, but are not limited to bitartrate, diacetate, difumarate, dimeglumine, diphosphate, disodium, and trihydrochloride.

The pharmaceutical compositions of the present invention comprise any one or more of the above-described component compounds of the present invention, or a pharmaceutically acceptable salt thereof as also above-described, together with a pharmaceutically acceptable carrier suitable for administration by inhalation, in accordance with the properties and expected performance of such carriers which are well-known in the pertinent art.

The term "carrier" as used herein includes acceptable diluents, excipients, adjuvants, vehicles, solubilization aids, viscosity modifiers, preservatives and other agents well known to the artisan for providing favorable properties in the final pharmaceutical composition to be administered by inhalation. In order to illustrate such carriers, there follows a brief survey of pharmaceutically acceptable carriers that may be used in the pharmaceutical compositions of the present invention, and thereafter a more detailed description of the various types of ingredients. Typical carriers include but are by no means limited to, ion exchange compositions; alumina; aluminum stearate; lecithin; serum proteins, *e.g.*, human serum albumin; phosphates; glycine; sorbic acid; potassium sorbate; partial glyceride mixtures of saturated vegetable fatty acids; hydrogenated palm oils; water; salts or electrolytes, *e.g.,* prolamine sulfate, disodium hydrogen

phosphate, potassium hydrogen phosphate, sodium chloride, and zinc salts; colloidal silica; magnesium trisilicate; polyvinyl pyrrolidone; cellulose-based substances; *e.g.,* sodium carboxymethylcellulose; polyethylene glycol; polyacrylates; waxes; polyethylene-polyoxypropylene-block polymers; and wool fat.

More particularly, the carriers used in the pharmaceutical compositions of the present invention comprise various classes and species of additives which are members independently selected from the groups consisting essentially of those recited in the following paragraphs.

Acidifying and alkalizing agents are added to obtain a desired or predetermined pH and comprise acidifying agents, *e.g.*, acetic acid, glacial acetic acid, malic acid, and propionic acid. Stronger acids such as hydrochloric acid, nitric acid and sulfuric acid may be used but are less preferred. Alkalizing agents include, *e.g.*, edetol, potassium carbonate, potassium hydroxide, sodium borate, sodium carbonate, and sodium hydroxide. Alkalizing agents which contain active amine groups, such as diethanolamine and trolamine, may also be used.

Aerosol propellants that are required to deliver the pharmaceutical composition as an aerosol under significant pressure are described in more detail further below.

Antimicrobial agents including antibacterial, antifungal and antiprotozoal agents are added where the pharmaceutical composition is topically applied to areas of the skin which are likely to have suffered adverse conditions or sustained abrasions or cuts which expose the skin to infection by bacteria, fungi or protozoa. Antimicrobial agents include such compounds as benzyl alcohol, chlorobutanol, phenylethyl alcohol, phenylmercuric acetate, potassium sorbate, and sorbic acid. Antifungal agents include such compounds as benzoic acid, butylparaben, ethylparaben, methylparaben, propylparaben, and sodium benzoate.

Antimicrobial preservatives are added to the pharmaceutical compositions of the present invention in order to protect them against the growth of potentially harmful microorganisms, which usually invade the aqueous phase, but in some cases can also grow in the oil phase of a composition. Thus, preservatives with both aqueous and lipid solubility are desirable. Suitable antimicrobial preservatives include, *e.g.*, alkyl esters of *p*-hydroxybenzoic acid, propionate salts, phenoxyethanol, methylparaben sodium, propylparaben sodium, sodium dehydroacetate, benzalkonium chloride, benzethonium chloride, benzyl alcohol, hydantoin derivatives, quaternary ammonium compounds and cationic polymers, imidazolidinyl urea, diazolidinyl urea, and trisodium ethylenediamine tetracetate (EDTA). Preservatives are preferably employed in amounts ranging from about 0.01 % to about 2.0% by weight of the total composition.

Antioxidants are added to protect all of the ingredients of the pharmaceutical composition from damage or degradation by oxidizing agents present in the composition itself or the use environment, *e.g.,* anoxomer, ascorbyl palmitate, butylated hydroxyanisole, butylated hydroxytoluene, hypophosphorous acid, potassium metabisulfite, propyl octyl and dodecyl gallate, sodium metabisulfite, sulfur dioxide, and tocopherols.

Buffering agents are used to maintain a desired pH of a composition once established, from the effects of outside agents and shifting equilibria of components of the composition. The buffering may be selected from among those familiar to the artisan skilled in the preparation of pharmaceutical compositions, *e.g.*, calcium acetate, potassium metaphosphate, potassium phosphate monobasic, and tartaric acid.

Chelating agents are used to help maintain the ionic strength of the pharmaceutical composition and bind to and effectively remove destructive compounds and metals, and include, *e.g.,* edetate dipotassium, edetate disodium, and edetic acid.

Dispersing and suspending agents are used as aids for the preparation of stable formulations and include, *e.g.*, poligeenan, povidone, and silicon dioxide.

Emulsifying agents, including emulsifying and stiffening agents and emulsion adjuncts, are used for preparing oil-in-water emulsions when these form the basis of the pharmaceutical compositions of the present invention. Such emulsifying agents include, *e.g.,* non-ionic emulsifiers such as $C_{10}$ -$C_{20}$ fatty alcohols and said fatty alcohols condensed with from 2 to 20 moles of ethylene oxide or propylene oxide, ($C_6$ -$C_{12}$)alkyl phenols condensed with from 2 to 20 moles of ethylene oxide, mono- and di- $C_{10}$ -$C_{20}$ fatty acid esters of ethylene glycol, $C_{10}$ -$C_{20}$ fatty acid monoglyceride, diethylene glycol, polyethylene glycols of MW 200-6000, polypropylene glycols of MW 200-3000, and particularly sorbitol, sorbitan, polyoxyethylene sorbitol, polyoxyethylene sorbitan, hydrophilic wax esters, cetostearyl alcohol, oleyl alcohol, lanolin alcohols, cholesterol, mono- and di-glycerides, glyceryl monostearate, polyethylene glycol monostearate, mixed mono- and distearic esters of ethylene glycol and polyoxyethylene glycol, propylene glycol monostearate, and hydroxypropyl cellulose. Emulsifying agents which contain active amine groups may also be used and typically include anionic emulsifiers such as fatty acid soaps, *e.g.*, sodium, potassium and triethanolamine soaps of $C_{10}$ -$C_{20}$ fatty acids; alkali metal, ammonium or substituted ammonium ($C_{10}$ -$C_{30}$)alkyl sulfates, ($C_{10}$ -$C_{30}$)alkyl sulfonates, and ($C_{10}$ -$C_{50}$)alkyl ethoxy ether sulfonates. Other suitable emulsifying agents include castor oil and hydrogenated castor oil; lecithin; and polymers of 2-propenoic acid together with polymers of acrylic acid, both cross-linked with allyl ethers of sucrose and/or pentaerythritol, having varying viscosities and identified by product names carbomer 910, 934, 934P, 940, 941, and 1342. Cationic emulsifiers having active amine groups may also be used, including those based on quaternary ammonium, morpholinium and pyridinium compounds. Similarly, amphoteric emulsifiers having active amine groups, such as cocobetaines, lauryl dimethylamine oxide and cocoylimidazoline, may be used. Useful emulsifying and stiffening agents also include cetyl

alcohol and sodium stearate; and emulsion adjuncts such as oleic acid, stearic acid, and stearyl alcohol.

Excipients include, *e.g.*, laurocapram and polyethylene glycol monomethyl ether.

Preservatives are used to protect pharmaceu6cal compositions of the present invention from degradative attack by ambient microorganisms, and include, *e.g.*, benzalkonium chloride, benzethonium chloride, alkyl esters of p-hydroxybenzoic acid, hydantoin derivatives, cetylpyridinium chloride, monothioglycerol, phenol, phenoxyethanol, methylparagen, imidazolidinyl urea, sodium dehydroacetate, propylparaben, quaternary ammonium compounds, especially polymers such as polixetonium chloride, potassium benzoate, sodium formaldehyde sulfoxylate, sodium propionate, and thimerosal.

Sequestering agents are used to improve the stability of the pharmaceutical compositions of the present invention and include, *e.g.*, the cyclodextrins which are a family of natural cyclic oligosaccharides capable of forming inclusion complexes with a variety of materials, and are of varying ring sizes, those having 6-, 7- and 8-glucose residues in a ring being commonly referred to as α-cyclodextrins, β-cyclodextrins, and γ-cydodextrins, respectively. Suitable cyclodextrins include, *e.g.*, α-cyclodextrin, β-cyclodextrin, γ-cyclodextrin, δ-cyclodextrin and cationized cyclodextrins.

Solvents which may be used in preparing the pharmaceutical compositions of the present invention include, *e.g.,* acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water.

Stabilizers which are suitable for use include, *e.g.*, calcium saccharate and thymol.

Sugars are often used to impart a variety of desired characteristics to the pharmaceutical compositions of the present invention and in order to improve the results obtained, and include, *e.g.,* monosaccharides, disaccharides and polysaccharides such as glucose, xylose, fructose, reose, ribose, pentose, arabinose, allose, tallose, altrose, mannose, galactose, lactose, sucrose, erythrose, glyceraldehyde, or any combination thereof.

Surfactants are employed to provide stability for the multi-component pharmaceutical compositions of the present invention, enhance existing properties of those compositions, and bestow desirable new characteristics on said compositions. Surfactants are used as wetting agents, antifoam agents, for reducing the surface tension of water, and as emulsifiers, dispersing agents and penetrants, and include, *e.g.*, lapyrium chloride; laureth 4, *i.e.,* α-dodecyl-ω-hydroxy-poly(oxy-1,2-ethanediyl) or polyethylene glycol monododecyl ether; laureth 9, *i.e.,* a mixture of polyethylene glycol monododecyl ethers averaging about 9 ethylene oxide groups per molecule; monoethanolamine; nonoxynol 4, 9 and 10, *i.e.,* polyethylene glycol mono(*p*-nonylphenyl) ether; nonoxynol 15, *i.e.,* α-(*p*-nonylphenyl)-ω-hydroxypenta-deca (oxyethylene); nonoxynol 30, *i.e.,* α-(*p*-nonylphenyl)-ω-hydroxytriaconta(oxyethylene); poloxalene, *i.e.,* nonionic polymer of the polyethylene-polypropylene glycol type, MW = approx. 3000; poloxamer, referred to in the discussion of ointment bases further above; polyoxyl 8, 40 and 50 stearate, *i.e.,* poly(oxy-1,2-ethanediyl), α-hydro-ω-hydroxy-; octadecanoate; polyoxyl 10 oleyl ether, *i.e.,* poly(oxy-1,2-ethanediyl), α-[(Z)-9-octadecenyl-ω-hydroxy-; polysorbate 20, *i.e.,* sorbitan, monododecanoate, poly(oxy-1,2-ethanediyl); polysorbate 40, *i.e.,* sorbitan, monohexadecanoate, poly(oxy-1,2-ethanediyl); polysorbate 60, *i.e.,* sorbitan, monooctadecanoate, poly(oxy-1,2-ethanediyl); polysorbate 65, *i.e.,* sorbitan, trioctadecanoate, poly(oxy-1,2-ethanediyl); polysorbate 80, *i.e.,* sorbitan, mono-9-monodecenoate, poly(oxy-1,2-ethanediyl); polysorbate 85, *i.e.,* sorbitan, tri-9-octadecenoate, poly(oxy-1,2-ethanediyl); sodium lauryl sulfate; sorbitan monolaurate; sorbitan monooleate; sorbitan monopalmitate; sorbitan monostearate; sorbitan sesquioleate; sorbitan trioleate; and sorbitan tristearate.

The pharmaceutical compositions of the present invention may be prepared using methodology which is well understood by the artisan of ordinary skill. Where the pharmaceutical compositions of the present invention are simple aqueous and/or other solvent solutions, the various components of the overall composition are brought together in any practical order, which will be dictated largely by considerations of convenience. Those components having reduced water solubility, but sufficient solubility in the same co-solvent with water, may all be dissolved in said co-solvent, after which the co-solvent solution will be added to the water portion of the carrier whereupon the solutes therein will become dissolved in the water. To aid in this dispersion/solution process, a surfactant may be employed.

In the above description of pharmaceutical compositions containing a combination of active ingredients of the present invention, the equivalent expressions: "administration", "administration of", "administering", and "administering a" have been used with respect to said pharmaceutical compositions. As thus employed, these expressions are intended to mean providing to a patient in need of treatment a pharmaceutical composition of the present invention by the inhalation route of administration herein described, wherein the active ingredients are combinations of compounds of the present invention, or a prodrug, derivative, or metabolite thereof which is useful in treating an obstructive airways or other inflammatory disease, disorder, or condition in said patient. Accordingly, there is included within the scope of the present invention any other compound which, upon administration to a patient, is capable of directly or indirectly providing a component compound of the present invention. Such compounds are recognized as prodrugs, and a number of established procedures are available for preparing such prodrug forms of the component compounds of the present invention.

The dosage and dose rate of the component compounds of the present invention effective for treating or

preventing an obstructive airways or other inflammatory disease, disorder, or condition, will depend on a variety of factors, such as the nature of the component compound, the size of the patient, the goal of the treatment, the nature of the pathology to be treated, the specific pharmaceutical composition used, and the observations and conclusions of the treating physician.

For example, where the dosage form is topically administered to the bronchia and lungs, *e.g.,* by means of a powder inhaler, nebulizer, or other device known in the art, suitable dosage levels of the component compounds of the present invention will be between about 0.001 μg/kg and about 10.0 mg/kg of body weight per day, preferably between about 0.5 μg/kg and about 0.5 mg/kg of body weight per day, more preferably between about 1.0 μg/kg and about 0.1 mg/kg of body weight per day, and most preferably between about 2.0 μg/kg and about 0.05 mg/kg of body weight per day of the active ingredient.

Using representative body weights of 10 kg and 100 kg in order to illustrate the range of daily oral dosages which might be used as described above, suitable dosage levels of the component compounds of the present invention will be between about 1.0 - 10.0 μg and 500.0 - 5000.0 mg per day, preferably between about 50.0 - 500.0 μg and 50.0 - 500.0 mg per day, more preferably between about 100.0 - 1000.0 μg and 10.0 - 100.0 mg per day, and most perferably between about 200.0 - 2000.0 μg and about 5.0 - 50.0 mg per day of the active ingredient comprising a compound of Formula (1.0.0). These ranges of dosage amounts represent total dosage amounts of each active ingredient per day for a given patient. The number of times per day that a dose is administered will depend upon such pharmacological and pharmacokinetic factors as the half-life of each active ingredient, which reflects its rate of catabolism and clearance, as well as the minimal and optimal blood plasma or other body fluid levels of each said active ingredient attained in the patient which are required for therapeutic efficacy

Numerous other factors must also be considered in deciding upon the number of doses per day and the amount of each active ingredient per dose that will be administered. Not the least important of such other factors is the individual response of the patient being treated. Thus, for example, where the active ingredients are used to treat or prevent asthma, and are administered topically *via* aerosol inhalation into the lungs, from one to four doses consisting of acuations of a dispensing device, *i.e.,* "puffs" of an inhaler, will be administered each day, each dose containing from about 50.0 μg to about 10.0 mg of each said active ingredient.

A preferred delivery form of the pharmaceutical compositions of the present invention that are useful for inhalation administration of the combinations of compounds herein described is that of an aerosol. An aerosol is, in general terms, a colloid system in which the continuous phase, *i.e.,* the dispersion medium, is a gas. With reference to the pharmaceutical compositions herein described, an aerosol composition comprises a solution or suspension of a drug consisting of a combination of compounds of the present invention, which can be atomized into a fine mist for inhalation therapy. Thus, the aerosol composition comprises a liquid propellant and a particulate material.

Finely divided particles of drugs and suitable carriers therefor are widely used in the pharmaceutical industry and are especially important in the case of inhalation drugs where it is desired that the drug particles penetrate deep into the lung of a patient being treated. Effective use of an aerosol drug composition in the form of a suspension usually requires that the suspension comprise a uniform dispersion of the particulate matter in order to insure that an aerosol is produced that has the required components present in known amounts. A dispersion that is not homogeneous is usually the result of poor dispersibility of the particulate matter in the propellant and/or a tendency of the particulate matter to aggregate, sometimes to an extent that is irreversible.

The present invention is concerned with particulate-containing aerosol compositions consisting of inhaler suspensions used for the delivery of a particulate medicament comprising a combination of compounds of the present invention to the lungs or upper airway passages. The inhaler suspension is preferably held in a pressurized container fitted with a metering valve of fixed volume. Such a container is easy to use and portable, and assures that a known dose of the medicament is administered on each occasion of use. Containers of this type are referred to as metered dose inhalers.

It is essential that the inhaler suspension be consistently and homogeneously dispersed and that the performance of the metering valve be reproducible and effective throughout the life of the container. The inhaler suspension usually consists of the medicament particles dispersed in a liquefied gas which in use acts as a propellant. Once the valve stem of the metering valve is depressed, the propellant fraction of the metered dose rapidly vaporizes so as to aerosolize the suspended particulate medicament which is then inhaled by the user.

Heretofore, chlorofluorocarbons such as CFC-11, CFC-12 and CFC-14 have been employed as propellants in metered dose inhalers. It is important that a particulate medicament intended for pulmonary administration have a particle size with a median aerodynamic diameter between about 0.05 μm and about 11 μm. Larger particles will not necessarily or readily penetrate into the lungs and smaller sized particles are readily breathed out. On the other hand, particles between about 0.05 μm and about 11 μm can possess a high surface energy and therefore be difficult to disperse initially in the propellant, and once dispersed can exhibit a tendency to aggregate undesirably and rapidly, leading eventually to irreversible aggregation of the particles. Where CFC has been used as a propellant, this problem has been overcome by the addition of a surfactant soluble in the CFC, which coats the medicament particles and prevents their aggregation

by means of steric hindrance. The presence of such a surfactant is also believed to be an aid to valve performance. Accordingly, in practice, medicament particles have been homogenized in liquid CFC-11 with the inclusion of a propellant soluble surfactant such as lecithin, oleic acid or sorbitan trioleate. The resulting bulk suspension has been dispensed into individual metered dose inhalers and a high vapor pressure propellant such as liquefied gas CFC-12/CFC-114 has then been added. These compositions have proven to be satisfactory in use, although the added surfactant can adversely affect the perceived taste of the inhaler in use. Oleic acid, *e.g.*, can impart a bitter taste.

Propellant CFC-11 ($CC1_3F$) and/or propellant CFC-114 ($CF_2Cl[CF_2Cl]$) with propellant CFC-12 ($CC1_2F_2$), however, are now believed to provoke the degradation of stratospheric ozone and there is thus a need to provide aerosol formulations for medicaments which employ so called "ozone-friendly" propellants. The continued use of CFC propellants has therefore become unacceptable and has frequently been banned by local regulations. Alternative propellants which have been suggested for use in metered dose inhalers comprise fluorocarbons, hydrogen-containing fluorocarbons, notably HFA-134a and HFA-227, and hydrogen-containing chlorofluoro-carbons, and a number of medicinal aerosol formulations using such propellant systems have been disclosed in the art.

Problems have been encountered in attempting to formulate the hydrofluoroalkanes into an aerosol composition such as an inhaler suspension. For example, the acceptable surfactants which have been employed in CFC-based suspensions are not sufficiently soluble in hydrofluoroalkanes to prevent irreversible aggregation of the particulate medicament from occurring. Further, neither HFA-134a nor HFA-227 is a liquid at an acceptable temperature, so that bulk homogenization with particulate material prior to filling into individual pressurized containers is possible only if carried out under pressure. A number of proposals have, accordingly, been made in an attempt to employ hydrofluoroalkanes as the propellant in pressurized metered dose inhalers. For example, see WO 91/04011; W0 91/11495; WO 91/114422; WO 92/00107; WO 93/08446; WO 92/08477; WO 93/11743; W0 93/11744; and WO 93/11745. These published applications are all concerned with the preparation of pressurized aerosols for the administration of medicaments and seek to overcome the problems associated with the use of the new class of propellants, in particular the problems of stability associated with the pharmaceutical formulations prepared.

WO 92/06675 suggests the use of non-volatile co-solvents to modify the solvent characteristics of the hydrofluoroalkane propellant and thereby increase the solubility and hence permit the use of the surfactants traditionally employed in CFC-based metered dose inhalers. The co-solvent must be selected so that it does not result in less desirable aerosol properties or impart an unpleasant sharp taste to the formulation.

WO 91/11173 and WO 92/00061 suggest the use of alternative surfactants that are sufficiently soluble in HFA-134a and HFA-227, but such surfactants must be demonstrated to be free of any toxicity to humans.

WO 96/19968 suggests the use of a pharmaceutical formulation comprising a particulate medicament, at least one sugar, and a fluorocarbon or hydrogen-containing chlorofluorocarbon propellant. The particle size of the sugars employed in the formulations is said to be obtainable using conventional techniques such as milling and micronization, and the suspension stability of the aerosol formulations is said to be especially good.

WO 00/27363 discloses aqueous dispersions of nanoparticulate aerosol formulations, dry powder nanoparticulate aerosol formulations, propellant-based aerosol formulations, methods of using the formulations in aerosol delivery devices, and methods of making such aerosol formulations. The nanoparticles in said aqueous dispersions or dry powder aerosol formulations comprise insoluble drug particles having a surface modifier thereon; and there is demonstrated the ability to aerosolize a concentrated nanoparticulate dispersion in an ultrasonic nebulizer which incorporates a fine mesh screen into its design. A therapeutic quantity of a concentrated nanoparticulate beclomethasone dipropionate formulation can be aerosolized in less than two seconds.

WO 00/00181 discloses compositions containing corticosteroid compounds present in a dissolved state, formulated in a concentrated, essentially non-aqueous form for storage, or in a diluted, aqueous-based form for ready delivery. The corticosteroid compositions contain ethoxylated derivatives of vitamin E and/or a polyethyleneglycol fatty acid ester as the high HLB surfactant present in the formulation. For example, beclomethasone dipropionate monohydrate is dissolved in a 2:1 wt./wt. mixture of PEG-200 and a-tocopherol polyethylene glycol succinate and then diluted with water, 1:6.65 by volume.

WO 99/47196 discloses methods and devices for delivering active agent formulations in dry powder or nebulized form, or in admixture with a propellant, said formulations being delivered at an inspiratory flow rate of <17 L/min, preferably 5-10 L/min. Bioavailability of the active agent is increased due to increased deposition of the active agent in the lung. A flow restricter is used which comprises an aperture or set of apertures and a valving arrangement.

WO 99/16420 discloses stabilized dispersions that may be administered to the lung of a patient using a nebulizer, which comprise a stabilized colloidal system containing a perforated microstructure of the active agent dispersed in a fluorocarbon suspension medium. Density variations between the suspended particles and the suspension medium are minimized and the attractive forces between the microstructures are attenuated, so that the disclosed dispersions are particularly resistant to degradation, such as by settling or flocculation.

US 5,874,063 discloses finely divided particles of a pharmaceutical substance which, when exposed to water vapor, gives off heat of <1.2 J/g. Examples are given of salbutamol sulfate (25%) and lactose (75%) conditioned with

water at relative humidity 55-65%, of a non-conditioned micronized substance mixture (5-8 J/g), and of a conditioned micronized mixture (<0.5 J/g).

US 5,192,528 discloses pharmaceutical liposomes containing corticosteroids for the treatment of respiratory tract diseases. For example, a liposome suspension contains 95% egg phosphatidylcholine, 29.6 mg/mL; 95% egg phosphatidylglycerol, 0.9 mg/mL; beclomethasone dipropionate, 0.42 mg/mL; vitamin E, 0.172 mg/mL; $Na_2HPO_4$, 1.5 mg/mL; NaCl, 5.0 mg/mL; and water to 1.0 mL. The liposome suspension is aerosolized in a nebulizer at an air pressure of 10 psi to obtain aerosol particles with a mass median aerodynamic diameter of approximately 0.42 μm.

EP 338,670 discloses a solution of an inhalation drug packaged in a sealed disperser containing a pressurized gas and provided with a one-way outlet metering valve, that may be administered by nebulization. The dispenser may be prepared by introducing the solution and the pressurized gas into the dispenser under sterile conditions, or the dispenser may be sterilized after introduction of the solution and the pressurized gas. A preferred solution contains Na cromoglycate and chlorbutol for use in the treatment of obstructive airways diseases, and is prepared by dissolving chlorbutol in water at 20-60°C in a covered or sealed vessel, and admixing the resulting solution with solid Na cromoglycate.

US 4,908,382 discloses inhalation of a nebulized solution containing 10 mg furosemide and 7 mg NaCl with pH adjusted to 9 with a NaOH solution, which is effective in the treatment of asthmatic patients with exercise-induced bronchoconstriction.

GB 2,204,790 discloses mixtures of nedocromil Na with anti-cholinergic agents which are synergistic in the treatment of reversible obstructive airways diseases. An example of a nebulizer solution is one containing 0.5% (wt./vol.) nedocromil Na, 0.2% of atropine methonitrate, and water to 100%.

WO 87/00431 discloses treatment of bronchospastic disease characterized by airways hyper-reactivity by administration of gallopamil, a known Ca channel blocker. An example is a 3 mL nebulizer solution containing 1-20 mg gallopamil hydrochloride, 4% ethanol, and 4% propylene glycol in sterile saline, with pH adjusted to 6 with $NaHCO_3$.

EP 140,434 discloses pharmaceutical compositions with anticholinesterase, agonistic cholinergic, and antimuscarinic activity contained in a parasympathomimetic quaternary ammonium salt and a nasal carrier suitable for nasal administration. An example of a nebulizer solution is one containing neostigmine methylsulfate, 3 g; NaCl, 0.9 g; $KH_2PO_4$, 0.68 g; NaOH, 0.056 g; methyl *p*-hydroxybenzoate, 0.080 g; propyl *p*-hydroxybenzoate, 0.020 g; glycerin, 10 g; and water to 100 mL.

US 3,715,432 discloses aqueous aerosol compositions for inspiration into the alveoli in treatment of lung disorders, containing submicron (0.2-1 μ diameter) particles which are stable against evaporation; prepared by dispersing 100 mg to 5 g lecithin, *e.g.*, DL-dipalmitoyl-α-lecithin, in 100 mL water or isotonic saline solution; and nebulized by an ultrasonic generator at 25-75°C.

W0 95/01324 discloses a method and apparatus suitable for the formation of particulate drugs in a controlled manner utilizing a supercritical fluid particle formation system. The apparatus comprises a particle formation vessel with means for controlling the temperature and pressure of said vessel, together with means for the co-introduction into said vessel of a supercritical fluid and a vehicle containing at least one drug substance in solution or suspension, such that dispersion and extraction of the vehicle occur substantially simultaneously by the action of the supercritical fluid. The simultaneous co-introduction of the vehicle containing at least one drug substance in solution or suspension and the supercritical fluid, allows a high degree of control of parameters, *e.g.*, temperature, pressure and flow rate, of both vehicle fluid and supercritical fluid, at the exact point when they come into contact with one another. This gives a high degree of control over the conditions under which particles of the drug substance suspended or dissolved in the vehicle are formed, and thus of the resulting physical properties of said particles.

WO 95/31964 discloses a formulation suitable for nebulization comprising fluticasone propionate, substantially all of the particles of which have a particle size of <12 μm; one or more surfactants; one or more buffering agents; and water. An example of a nebulizer solution is one containing micronized fluticasone propionate, 0.525 mg; polyoxyethylene sorbitan monolaurate, 0.14 mg; sorbitan monolaurate, 0.018 mg; $NaH_2PO_4$, 18.80 mg; $Na_2HPO_4$, 3.50 mg; NaCl, 9.60 mg; and water to 2 mL.

WO 99/18971 discloses an aqueous nebulizer suspension containing water, mometasone furoate monohydrate, a nonionic surfactant, a soluble salt, and optionally a.pH buffer. The suspension is prepared by ultra-sonication or jet milling techniques. An-example of a nebulizer solution is one containing mometasone furoate, 500 mg; Polysorbate-80, 50 mg; citric acid monohydrate, 181 mg; sodium citrate dihydrate, 335 μg; sodium chloride, 9 mg; and water q.s. 1 mL. The suspension has a median particle size of 1.24 μm and a mean particle size of 1.34 μm.

WO 96/25919 discloses an aerosol comprising droplets of an aqueous dispersion of nanoparticles comprising beclomethasone particles having a surface modifier on the surface thereof. An example of a nebulizer solution is one containing a suspension of 2.5% beclomethasone dipropionate in an aqueous solution of polyvinyl alcohol as a surface modifier. The nanoparticles have a particle size distribution of 0.26 μm.

WO 96/22764 discloses pharmaceutical liposomes or dehydrated liposomes for use in the treatment of asthma by inhalation therapy. An example of a nebulizer solution is one containing 9α-chloro-6α-fluoro-11β-hydroxy-16α-methyl-3-oxo-17α-propionyloxyandrosta-1,4-diene-17β-carboxylate and one or more synthetic phospholipids, especially 1-*N*-hexadecanoyl-2-(9-*cis*-octadecenoyl)-3-sn-phosphatidylcholine, 700 mg; and Na 1,2-di(9-*cis*-octadecenoyl)-3-sn-phosthatidylserine, 300 mg dissolved in *tert*-BuOH, and the solution thereby obtained mixed with 100 mg of the above-recited 17β-carboxylate dissolved in 5 mL *tert*-BuOH. The resulting solution is added dropwise to 200 mL phosphate-buffered saline solution, and the aqueous liposome suspension is dialyzed against PBS and concentrated to 20 mL, filtered, and dispensed into vials for administration by nebulizer.

As already indicated, finely divided drug particles are prepared by conventional methods that involve micronization or grinding, although a number of other techniques are also available for their production. Micronization can produce particles which have regions of partially amorphous structure, but which are generally sufficiently stable for pharmaceutical use. However, these particles are liable to change their structure when kept in an adverse environment, such as during storage of a drug when conditions of high humidity that cause agglomeration may be encountered. Such adverse conditions can also be encountered during use of the drug by a patient. Drug particles produced by conventional methods often give off significant amounts of heat when exposed to water vapor. It is known in the art that this problem can be avoided by surface treatment of the particles without substantially altering their particle size. An added benefit of such treated particles is that they help to increase the respirable fraction of drugs in powder form when used in dry powder inhalation devices. Such particles have also been found to have a greater degree of crystallinity than more conventional fine particles. Preferably such particles give off less than 1.0 J/g, more preferably less than 0.5 J/g, and most preferably less than 0.1 J/g.

The particle size of drug substances in finely divided form, where it is desired that such particles enter deep into the lung of a patient being treated, should be <10 μm, and is preferably in the range of 0.1 to 10 μm. Where excipients in finely divided form are used as carriers for such particulate drug substances, they may be of a particle size of <10 μm, and preferably are in the range of 0.1 to 10 μm. In those cases when it is desired that said excipient does not enter the lung to any appreciable extent, the excipient particles may have a size of up to about 120 μm*, e.g.,* of from about 30 to about 120 μm. The size of a particle of either a drug substance or an excipient may be measured using a Malvem Master Sizer, a Coulter Counter, or a microscope. Such particles sizes are usually expressed as mass median diameters.

The total surface area of the particulate drug substances and their excipients which comprise the pharmaceutical compositions of the present invention is also an important criterion. Surface areas of said particles are determined by BET gas absorption, *e.g.*, as measured by a Flowsorb II 2300 or Gemini 2370, available from Micromeritics Co., USA, and should be from 3 to 12 $m^2$/g, and preferably of from 3 to 9 $m^2$/g.

The weight ratio of particulate drug substances to their excipients which are utilized in the pharmaceutical compositions of the present invention is preferably in the range of 1:1 to 1:1000, respectively, and more preferably in the range of 1:1 to 1:500, and most preferably in the range of 1:1 to 1:200.

Suitable excipients for use in the pharmaceutical compositions of the present invention are selected from those which are generally recognized as safe for inhalation use, and include, *e.g.,* carbohydrates, including sugars, *e.g.,* lactose, glucose, fructose, galactose, trehalose, sucrose, maltose, xylitol, mannitol, myoinositol, raffinose, maltitol, and melezitose. Other suitable excipients include amino acids, *e.g.,* alanine and betaine; and compounds which enhance the absorption of drug substances in the lung, such as surfactants, *e.g.,* alkali metal salts of fatty acids, including sodium tauro-dihydrofusidate, lecithins, sodium glycocholate, sodium taurocholate, and octylglucopyranoside. Other types of excipients useful in forming the pharmaceutical compositions of the present invention include antioxidants, *e.g.*, ascorbic acid; and buffer salts.

All of the substances which are components of the pharmaceutical compositions of the present invention can be used in the form of solvates, *e.g.*, hydrates; esters; or salts; or in the form of solvates or hydrates of such salts or esters.

In certain embodiments of the present invention, the method disclosed in above-mentioned W0 95/01324 is used, including an apparatus suitable for the formation of particulate drugs in a controlled manner utilizing a supercritical fluid particle formation system. An aerosol pharmaceutical formulation prepared in accordance with this method comprises a combination of compounds of the present invention having a controlled particle size, shape and morphology, together with a fluorocarbon, hydrogen-containing fluorocarbon or hydrogen-containing chlorofluorocarbon propellant. In particular, use of particulate crystalline forms of said component compounds can provide benefits consisting of a reduction in the rates of agglomeration and deposition of drug substance onto aerosol can walls, actuator and valve components. Use of such particulate crystalline forms may also permit the formation of stable dispersions using little or no additional components such as surfactants or co-solvents. It is also possible to reduce the adsorption of drug substances into the rubber components of the valve and/or actuator parts of the delivery device. A further benefit of minimizing or eliminating the use of formulation excipients such as surfactants and co-solvents is a formulation that may be substantially taste and odor free, less irritating and less toxic than conventional formulations. Preferably the propellant is 1,1,1,2-tetrafluoroethane (HFA 134a), in which formulations the weight ratio of drug to propellant is preferably between 0.025:75 and 0.1:75, for example 0.05:75.

Preparation of particles using the supercritical fluid particle formation method also permits control over the quality of the crystalline and polymorphic phases of those particles. Many of the compound components of the combinations of the present invention exist in two or more polymophic forms, and it is desirable to provide the best particulate forms for these polymorphs as well. It is possible to achieve such quality control because the particles will experience the same stable conditions of temperature and pressure when formed. This method also affords the potential for enhanced purity of the particulate final product, which is a result of the high selectivity of supercritical fluids under different working conditions, that in turn enables the extraction of one or more impurities that may be present from the vehicle containing the drug substance of interest.

Co-introduction of the vehicle and supercritical fluid, leading to simultaneous dispersion and particle formation, allows particle formation to be carried out at temperatures at or above the boiling point of the vehicle, enabling operation of the process in temperature and pressure domains which allow the formation of particulate products not otherwise achievable. Thus, control of parameters such as size and shape in the particulate product will depend upon the operating conditions used when carrying out the supercritical fluid method. Variables include the flow rates of the supercritical fluid and/or of the vehicle containing the drug substance, the concentration of the drug substance in the vehicle, and the temperature and pressure inside the particle formation vessel.

Aerosol pharmaceutical formulations containing compound combinations of the present invention are prepared in a form having a dynamic bulk density of <0.1g/cm$^{-3}$, preferably in a range of between 0.01 and 0.1 g/cm$^{-3}$ and, more preferably, in the range of between 0.01 and 0.075 g/cm$^{-3}$, together with a fluorocarbon, hydrogen-containing fluorocarbon or hydrogen-containing chlorofluorocarbon propellant. The dynamic bulk density (W) is indicative of a substance's fluidisability and is defined as:

$$W = \frac{(P - A)C}{100} + A$$

where P is the packed bulk density (g/cm$^{-3}$), A is the aerated bulk density (g/cm$^{-3}$), and C is the compressibility (%) where C is calculated by the equation:

$$C = \frac{P - A}{P} \times 100$$

In those cases where the value of W is low, there is a correspondingly high degree of fluidisability.

When crystallized compound components of the present invention prepared by other conventional methods are compared to those prepared by the above-described supercritical fluid particle formation method, both before and after micronisation, said component compounds exhibit a significantly lower dynamic bulk density. It will be appreciated that in the case of an inhaled pharmaceutical, it is particularly desirable to produce a drug substance which is readily fluidisable, thereby potentially improving its inhalation properties. Thus, the component compounds used in the formulations of the present invention are observed to have improved handling and fluidising characteristics compared with said compounds crystallized by other conventional methods.

Preferably, the of the present invention are within a particle size range suitable for pharmaceutical dosage forms to be delivered by inhalation or insufflation. A suitable particle size range for this use is I to 10 $\mu$m, preferably 1 to 5 $\mu$m. Said particles also generally have a uniform particle size distribution, as measured by a uniformity coefficient of from 1 to 100, typically 1 to 20, *e.g.,* 5 to 20.

The drug substances employed in the pharmaceutical formulations of the present invention typically have a low cohesivity, for example of 0 to 20%, preferably 0 to 5%, as established by methods of measurement based on those described by R. L. Carr in *Chemical Engineering,* 163-168, 1965.

Conventionally crystallized component compounds used in the present invention may also be studied by differential scanning calorimetry (DSC) in order to show any transition between two or more polymorphic forms that may exist. Use of the above-described supercritical fluid particle formation method allows the preparation of substantially pure polymorphs or controlled mixtures of the polymorphic forms. The thus prepared polymorphs are also stable, meaning that there is no transition from one polymorph to another observed under DSC conditions. By "substantially pure" polymorph is meant a composition containing a first polymorph, but essentially none of the other polymorph(s); and by "essentially none" is meant less than 0.5% w/w based upon said first polymorph, *e.g.*, 0.1 % or less.

A component compound of the present invention prepared by the above-described supercritical fluid particle formation method may be used to prepare a pharmaceutical composition which further comprises a pharmaceutically acceptable carrier. Preferred carriers for this purpose include polymers, *e.g.*, starch and hydroxypropylcellulose; silicon dioxide; sorbitol; mannitol; and lactose, *e.g.*, lactose monohydrate. Using the above-described supercritical fluid particle

formation method, a component compound and a carrier may be co-crystallized together to form multi-component particles comprising both said component compound and said carrier. Pharmaceutical formulations of the present invention comprise said multi-component particles together with a fluorocarbon, hydrogen-containing fluorocarbon, or hydrogen-containing chlorofluorocarbon propellant. Preferred embodiments of the present invention include a pharmaceutical composition comprising a component compound together with lactose in the form of multi-component particles.

For further details concerning the use of supercritical fluids, see J. W. Tom and P.G. Debendetti, "Particle Formation with Supercritical Fluids - A Review", *J. Aerosol. Sci.,* 22 (5), 555-584 (1991). A supercritical fluid can be defined as a fluid existing simultaneously at or above its critical pressure ($P_C$) and its critical temperature ($T_C$). Supercritical fluids are characterized by high diffusivity, low viscosity, and low surface tension compared with other non-supercritical liquids. The significant compressibility of supercritical fluids compared with that of the ideal gas implies large changes in fluid density in response to slight changes in pressure, which in turn means highly controllable solvation power. Supercritical fluid densities typically range from 0.1-0.9 g/mL under normal working conditions. Consequently, selective extraction with one supercritical fluid is possible.

Many supercritical fluids are normally gases under ambient conditions, thereby eliminating the evaporation/concentration step needed with conventional liquid extraction. Further, most of the commonly used supercritical fluids create non-oxidizing or non-degrading atmospheres due to their inertness and the moderate temperatures which may be employed during routine working, thus providing a protective environment for sensitive and thermolabile compounds. Carbon dioxide is the most extensively used supercritical fluid due to its cheapness, non-toxicity, non-flammability and low critical temperature.

As a result of the above-described characteristics of supercritical fluids, several techniques of extraction and particle formation have been developed which utilize supercritical fluids, in addition to that described in the above-mentioned W0 95/01324.

As used herein, the term "supercritical fluid" means a fluid at or above its critical pressure ($P_c$) and critical temperature ($T_c$) simultaneously. In practice, the pressure of the fluid is likely to be in the range of from 1.01 $P_c$ - 7.0 $P_c$, and its temperature in the range of from 1.01 $T_c$, - 4.0 $T_c$. The term "vehicle" as used herein means a fluid which dissolves a solid or solids, to form a solution, or which forms a suspension of a solid or solids which do not dissolve, or else have a low solubility in said fluid. Said vehicle can be composed of one or more fluids.

As used herein, the term "supercritical solution" means a supercritical fluid which has extracted and dissolved said vehicle. The term "dispersion" as used herein means the formation of droplets of said vehicle containing at least one drug substance in solution or suspension. The term "particulate product" as used herein includes products in a single-component or multi-component form, *e.g.*, as an intimate mixture of one component in a matrix of another component.

Supercritical fluids for use as described herein include carbon dioxide, nitrous oxide, sulphur hexafluoride, xenon, ethylene, chlorotrifluoro methane, ethane, and trifluoromethane. Carbon dioxide is an especially preferred choice as supercritical fluid. The supercritical fluid may optionally contain one or more modifiers, *e.g.,* methanol, ethanol, isopropanol or acetone. When used, the modifier preferably constitutes not more than 20%, and more particularly constitutes between 1 and 10%, of the supercritical fluid. The term "modifier" as used herein is well known to those persons skilled in the art. Accordingly, a modifier (or co-solvent) may be described as a substance which, when added to a supercritical fluid, changes the intrinsic properties of said supercritical fluid at or about the critical point. It will be appreciated that the precise conditions of operation of the process described herein will be dependent upon the choice of supercritical fluid and whether or not any modifiers are present.

It is preferred to maintain the pressure inside the particle formation vessel substantially in excess of the Pc, *e.g.*, 100-300 bar for carbon dioxide, while the temperature is maintained slightly above the Tc, *e.g.*, 40-600°C for carbon dioxide. The flow rates of the supercritical fluid and/or the vehicle may also be controlled so as to achieve a desired particle size, shape and/or form. Typically, the ratio of the vehicle flow rate to the supercritical fluid flow rate is between 0.001 and 0.1, preferably between 0.01 and 0.07, and more preferably around 0.03. The method preferably additionally involves collecting the particulate product following its formation, and may also involve recovering the supercritical solution formed, separating the components of the solution, and recycling one or more of those components for future use. It will be appreciated that the choice of a suitable combination of supercritical fluid, modifier, if any, and vehicle is well within the capabilities of a person of ordinary skill in the art.

Use of an automated back-pressure regulator such as model number 880-81 produced by Jasco Inc. can eliminate pressure fluctuation across the particle formation vessel and ensure a more uniform dispersion by the supercritical fluid of the vehicle containing the drug substance, with narrow droplet size distribution, during the particle formation process. The dispersed droplets are unlikely to reunite to form larger droplets, since the dispersion occurs by the action of the supercritical fluid, which also ensures thorough mixing with the vehicle and rapidly removes the vehicle from the drug substance, leading to particle formation. The means for co-introduction of the supercritical fluid and the vehicle into the particle formation vessel should allow for concurrent directions of flow, preferably by means of a coaxial nozzle. This procedure ensures no contact between the formed particles and the vehicle fluid around the nozzle tip area. Such contact

reduces control of the final product size and shape.

Further control over droplet size in addition to that provided by the above-described nozzle design, is achieved by managing the flow rates of the supercritical fluid and the vehicle fluid. Also, retaining the particles in the particle formation vessel eliminates the potential of contact with the vehicle fluid that might otherwise take place on depressurizing of the supercritical solution. Such contact would alter the shape and size, and potentially the yield, of the particulate product. Another advantage of the above-described method is that it can allow particle formation to occur in a completely closed environment in which the apparatus is sealed from the atmosphere. This facilitates the maintenance of sterile operating conditions and the elimination of oxygen, moisture, or other contaminants. It also reduces the risk of environmental pollution.

The final aerosol pharmaceutical formulation of the present invention desirably contains 0.03-0.13% w/w, preferably 0.07% w/w, of medicament relative to the total weight of said formulation.

Suitable propellants for use in the pharmaceutical compositions of the present invention comprise any fluorocarbon, hydrogen-containing fluorocarbon, or hydrogen-containing chlorofluorocarbon or mixtures thereof having a sufficient vapor pressure to render them effective as propellants. Preferably, said propellant will be a non-solvent for the medicament involved. Suitable propellants include ($C_1$-$C_4$) hydrogen-containing chlorofluorocarbons, *e.g.*, $CH_2ClF$, $CClF_2CHClF$, $CF_3CHClF$, $CHF_2CClF_2$, $CHClFCHF_2$, $CF_3CH_2Cl$, and $CClF_2CH_3$; ($C_1$-$C_4$) hydrogen-containing fluorocarbons, *e.g.,* $CHF_2CHF_2$, $CF_3CH_2F$, $CHF_2CH_3$, and $CF_3CHFCF_3$; and perfluorocarbons, *e.g.*, $CF_3CF_3$ and $CF_3CF_2CF_3$.

Where mixtures of fluorocarbon, hydrogen-containing fluorocarbon, or hydrogen-containing chlorofluorocarbon propellants are employed, they may be mixtures of the above-identified propellant compounds, or they may be mixtures, preferably binary mixtures, with other fluorocarbon, hydrogen-containing fluorocarbon, or hydrogen-containing chlorofluorocarbon propellants, *e.g.*, $CHClF_2$, $CH_2F_2$, and $CF_3CH_3$. Preferably, a single fluorocarbon, hydrogen-containing fluorocarbon, or hydrogen-containing chlorofluorocarbon is employed as the propellant. Particularly preferred as propellants are ($C_1$-$C_4$) hydrogen-containing fluorocarbons, *e.g.,* 1,1,1,2-tetrafluoroethane, $CF_3CH_2F$; and 1,1,1,2,3,3,3-heptafluoro-*n*-propane, $CF_3CHFCF_3$, especially 1,1,1,2-tetrafluoroethane. It is preferred, but not required, that propellants are used which do not degrade stratospheric ozone. Accordingly, it is preferred that the pharmaceutical formulations of the present invention be substantially free of chlorofluorocarbons, *e.g.,* $CC1_3F$, $CC1_2F_2$, and $CF_3CC1_3$.

The propellant used in preparing the pharmaceutical compositions of the present invention may additionally contain a volatile adjuvant such as a saturated hydrocarbon, *e.g.,* propane, *n*-butane, *iso*-butane, pentane, and *iso*-pentane; or a dialkyl ether, *e.g.,* dimethyl ether. Up to 50% w/w of the propellant which is being used may comprise a volatile hydrocarbon, *e.g.,* 1-30% w/w. Preferably, however, pharmaceutical formulations of the present invention are substantially free of volatile adjuvant.

It is not required that the pharmaceutical compositions of the present invention contain a surfactant or a co-solvent, and it is not necessary to pre-treat the medicament prior to dispersal in the propellant. However, certain pharmaceutical formulations of the present invention may include liquid components of higher polarity than the propellant employed. Such polarity may be determined by the method described in EP 327,777. Where such components of higher polarity are included, alcohols, *e.g.*, ethanol, are preferable. Such higher polarity liquid components are preferably included at relatively low concentrations, *e.g.*, <5%, preferably <1% w/w, based on the total weight of fluorocarbon or hydrogen-containing chlorofluorocarbon present. Preferred pharmaceutical formulations of the present invention contain essentially no higher polarity liquid components, *i.e.,* <0.1 % w/w, based on total weight of propellant, *e.g.,* 0.0001 % or less.

Where a surfactant is employed in the pharmaceutical compositions of the present invention, it is selected from those which are physiologically acceptable upon administration by inhalation, *e.g.*, oleic acid; sorbitan trioleate (Span® 85); sorbitan monooleate; sorbitan monolaurate; polyoxyethylene (20) sorbitan monolaurate; polyoxyethylene (20) sorbitan monooleate; natural lecithin; fluorinated and perfluorinated surfactants including fluorinated lecithins; fluorinated phosphatidylcholines; oleyl polyoxyethylene (2) ether; stearyl polyoxyethylene (2) ether; lauryl polyoxyethylene (4) ether; block copolymers of oxyethylene and oxypropylene; synthetic lecithin; diethylene glycol dioleate; tetrahydrofurfuryl oleate; ethyl oleate; isopropyl myristate; glyceryl monooleate; glyceryl monostearate; glyceryl mono-ricinoteate; cetyl alcohol; stearyl alcohol; polyethylene glycol 400; cetyl pyridinium chloride; benzalkonium chloride; olive oil; glyceryl monolaurate; corn oil; cotton seed oil; and sunflower seed oil.

Embodiments of the present invention comprising a pharmaceutical formulation in which the particulate medicament is pre-coated with surfactant, preferably contain substantially a non-ionic surfactant having reasonable solubility in substantially nonpolar solvents, since it facilitates coating of the medicament particles when using solvents in which the medicament has limited or minimal solubility. The particulate drug substance with its dry coating of surfactant may then be suspended in propellant, optionally with a co-solvent such as ethanol. These types of pharmaceutical formulations are well known in the art and are described in W0 92/08446 and W0 92/08447.

The pharmaceutical compositions of the present invention may be prepared by dispersal of the combination of particulate drug substances and the pharmaceutically acceptable carrier in the selected propellant in an appropriate container with the aid, *e.g.*, of sonication. This preparation process is preferably carried out under anhydrous condition

in order to prevent any adverse effects on suspension stability from moisture. Chemical and physical stability and the pharmaceutical acceptability of the aerosol formulations of the present invention may be determined using techniques that are well known in the art. For example, chemical stability of the components may be determined by HPLC assay of the overall formulation after storage for a prolonged period of time. Physical stability data may be obtained from analytical techniques, *e.g.,* leak testing, valve delivery assay based on average shot weights per actuation, dose reproducibility assay based on active ingredient per actuation, and spray distribution analysis.

The particle size distribution of the aerosol formulations of the present invention may be measured by conventional techniques, *e.g.,* by cascade impaction, or by twin impinger analysis as described in *British Pharmacopoeia,* A204-207, Appendix XVII C, 1988. Using this technique, the "respirable fraction" may be calculated, which, as used herein, means the amount of active ingredient collected in the lower impingement chamber per actuation, expressed as a percentage of the total amount of active ingredient delivered per actuation. The pharmaceutical formulations of the present invention containing the combination of compounds as described herein of mean particle size between 1 and 10 μm, preferably have a respirable fraction of 30% or more by weight of the medicaments, more preferably 30-70% by weight, *e.g.,* 30-50% by weight, based on the total weight of said medicaments.

The pharmaceutical formulations of the present invention may be filled into canisters suitable for delivering pharmaceutical aerosol formulations. Such canisters generally comprise a container capable of withstanding the vapor pressure of the propellant employed, *e.g.,* a plastic or plastic-coated glass bottle, or preferably a metal can, *e.g.*, an aluminum can that is optionally anodized, lacquer-coated, and/or plastic-coated, said container being closed with a metering valve. Canisters lined with a fluorocarbon polymer, especially polytetrafluoroethylene, PTFE, in combinatiion with a non-fluorocarbon polymer, especially pllyethersulfone, PES, are preferred. Typical metering valves are designed to deliver a metered amount of the pharmaceutical formulation per actuation, and usually incorporate a gasket to prevent leakage of propellant through or around said valve. Said gasket may comprise any suitable elastomeric material, *e.g.,* low density polyethylene; chlorobutyl rubber; black and white butadiene-acrylonitrile rubbers; butyl rubber; and neoprene. Suitable valves are available from a number of different manufacturers.

Conventional bulk manufacturing methods and machinery well known in the art may be employed in the preparation of large scale batches for the commercial production of filled canisters. For example, in one bulk manufacturing method, a metering valve is crimped onto an aluminum can to form an empty canister. The particulate medicament is thereafter added to a charge vessel and liquified propellant is pressure filled through the charge vessel into a manufacturing vessel. The particulate medicament suspension is mixed before recirculation to a filling machine, and an aliquot of the medicament suspension is then filled through the metering valve into the canister. Each filled canister is check-weighed, coded with a batch number, and packed into a tray for storage prior to release testing.

Each filled canister is conveniently fitted into a suitable channeling device to form a metered dose inhaler for administration of the medicament into the lungs or nasal cavity of a patient. Channeling devices comprise, *e.g.,* a valve actuator and a cylindrical or cone-like passage through which said medicament may be delivered from said filled canister *via* said metering valve to the nose or mouth of a patient. Metered dose inhalers are typically designed to deliver a fixed unit dosage of medicament per actuation, *e.g.*, in the range of 10-500 μg of medicament per puff. However, the actual amount of medicament administered per day to a patient will depend upon the age and condition of that patient, the particular medicaments being administered, and the frequency of administration of said medicaments. When combinations of medicaments are employed as in the case of the present invention, the dose of each component of the combination will generally be that employed for each component when used alone. Typically, administration may be one or more times, *e.g.*, 1-8 times per day, with 1-4 puffs being inhaled during each individual administration. Each filled canister for use in a metered dose inhaler contains anywhere from about 60 to about 240 doses or puffs of medicament.

## Preparations and Working Examples

There follows a description of numerous Examples showing preparation of pharmaceutical compositions containing a combination of therapeutic agents in accordance with the present invention. These Examples are intended to further illustrate the combinations of therapeutic agents of the present invention, pharmaceutical compositions contaiing them, and processes in accordance with which said pharmaceutical compositions may be readily prepared by the artisan. The artisan will be aware of many other suitable processes and pharmaceutically acceptable carriers that are also available, as well as acceptable variations in the procedures and ingredients described below.

## EXAMPLE 1

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 9-cyclopentyl-5,6-dihydro-7-ethyl-3-(2-thienyl)-9*H*-pyrazolo[3,4-c]-1,2,4-triazolo [4,3-a]pyridine | dichlorotetrafluoroethane |
| tiotropium bromide | trichloromonofluoromethane |
| dichlorodifluoromethane | soya lecithin |

**EXAMPLE 2**

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 9-cyclopentyl-5,6-dihydro-7-ethyl-3-(*tert*-butyl)-9*H*-pyrazolo[3,4-c]-1,2,4-triazolo [4,3-a]pyridine | dichlorotetrafluoroethane |
| tiotropium bromide | trichloromonofluoromethane |
| dichlorodifluoromethane | soya lecithin |

**EXAMPLE 3**

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 9-cyclopentyl-5,6-dihydro-7-ethyl-3-phenyl-9*H*-pyrazolo[3,4-*c*]-1,2,4-triazolo[4,3-α] pyridine | dichlorotetrafluoroethane |
| tiotropium bromide | trichloromonofluoromethane |
| dichlorodifluoromethane | soya lecithin |

**EXAMPLE 4**

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 9-cyclopentyl-5,6-dihydro-7-ethyl-3-(4-pyridyl)-9*H*-pyrazolo[3,4-*c*]-1,2,4-triazolo [4,3-α]pyridine | dichlorotetrafluoroethane |
| tiotropium bromide | trichloromonofluoromethane |
| dichlorodifluoromethane | soya lecithin |

**EXAMPLE 5**

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 9-cyclopentyl-5,6-dihydro-7-ethyl-3-(3-thienyl)-9*H*-pyrazolo[3,4-*c*]-1,2,4-triazolo[4,3-α] pyridine | dichlorotetrafluoroethane |
| tiotropium bromide | ethanol |
| dichlorodifluoromethane | ascorbic acid |

**EXAMPLE 6**

A package in the form of a non-pressurized glass vial is prepared which may be used for administration of the active ingredients as an aerosol mist by hand-bulb nebulizer, compressed air or oxygen operated nebulizer, or by an

intermittent positive pressure breathing (IPPB) device. The contents of each said vial are as follows:

| | |
|---|---|
| 3-benzyl-9-cyclopentyl-5,6-dihydro-7-ethyl-9*H*-sodium pyrazolo[3,4-*c*]-1,2,4-triazolo [4,3-α]pyridine | metabisulfite |
| tiotropium bromide | glycerin or saccharin sodium |
| chlorobutanol | citric acid or sodium citrate |
| purified water | sodium chloride |

## EXAMPLE 7

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 9-cyclopentyl-5,6-dihydro-7-ethyl-3-propyl-9*H*-pyrazolo[3,4-*c*]-1,2,4-triazolo[4,3-α] pyridine | trichloromonofluoromethane |
| tiotropium bromide | sorbitan trioleate |
| dichlorodifluoromethane | |

## EXAMPLE 8

A package in the form of a pressurized, tetrafluoroethylene-coated aluminum canister for use in a metered dose inhaler is prepared which is sufficient to provide about 200 actuations of the inhaler, each actuation providing about 20 μg of each active ingredient. The contents of each said canister are as follows:

| | |
|---|---|
| 3,9-dicyclopentyl-5,6-dihydro-7-ethyl-9*H*-pyrazolo[3,4-*c*]-1,2,4-triazolo[4,3-α] pyridine | trichloromonofluoromethane |
| tiotropium bromide | oleic acid |
| dichlorodifluoromethane | |

## EXAMPLE 9

A package in the form of a non-pressurized glass vial is prepared which may be used for administration of the active ingredients as an aerosol mist by hand-bulb nebulizer, compressed air or oxygen operated nebulizer, or by an intermittent positive pressure breathing (IPPB) device. The contents of each said vial are as follows:

| | |
|---|---|
| 9-cyclopentyl-5,6-dihydro-7-ethyl-3-(1-methyl-cyclohex-1-yl)-9*H*-pyrazolo[3,4-*c*]-1,2,4-triazolo-[4,3-α]pyridine | sulfuric acid |
| tiotropium bromide | sodium chloride |
| benzalkonium chloride | purified water |

## EXAMPLE 10

A package in the form of a double-foil blister strip in which each blister contains a powder formulation is prepared. Said package is designed for use with a device that opens each said blister when said device is actuated. The active ingredients are dispersed from said blister into the air stream created when the patient inhales through the mouthpiece of said device. The dry powder contents of each said blister are as follows:

| | |
|---|---|
| 3-(*tert*-butyl)-9-cyclopentyl-5,6-dihydro-7-ethyl-9*H*-pyrazolo[3,4-*c*]-1,2,4-triazolo[4,3-α]pyridine | lactose |
| tiotropium bromide monohydrate | |

## Claims

**1.** A combination of therapeutic agents useful in the treatment of obstructive airways and other inflammatory diseases

comprising *(I)* a PDE4 inhibitor that is therapeutically effective in the treatment of said diseases when administered by inhalation; together with *(II)* an anti-cholinergic agent comprising a member selected from the group consisting of tiotropium and derivatives thereof that is therapeutically effective in the treatment of said diseases when administered by inhalation,
wherein the PDE4 inhibitor comprises a member selected from the group consisting of the following:

9-cyclopentyl-5,6-dihydro-7-ethyl-3-phenyl-9*H*-pyrazolo[3,4-*c*]-1,2,4-triazoto[4,3-α]pyridine;
9-cyclopenyl-5,6-dihydro-7-ethyl-3-(furan-2-yl)-9*H*-pyrazolo[3,4-*c*]-1,2,4-triazolo[4,3-α]pyridine;
9-cyclopentyl-5,6-dihydro-7-ethyl-3-(2-pyridyl)-9*H*-pyrazolo[3,4-*c*]-1,2,4-triazolo[4,3-α]pyridine;
9-cyclopentyl-5,6-dihydro-7-ethyl-3-(4-pyridyl)-9*H*-pyrazolo[3,4-*c*]-1,2,4-triazolo[4,3-α]pyridine;
9-cyclopentyl-5,6-dihydro-7-ethyl-3-(3-thienyl)-9*H*-pyrazolo[3,4-*c*]-1,2,4-triazolo[4,3-α]pyridine;
3-benzyl-9-cyclopentyl-5,6-dihydro-7-ethyl-9*H*-pyrazolo[3,4-*c*]-1,2,4-triazolo[4,3-α]pyridine;
9-cyclopentyl-5,6-dihydro-7-ethyl-3-propyl-9*H*-pyrazolo[3,4-*c*]-1,2,4-triazolo[4,3-α]pyridine;
3,9-dicyclopentyl-5,6-dihydro-7-ethyl-9*H*-pyrazolo[3,4-*c*]-1,2,4-triazolo[4,3-α]pyridine;
9-cyclopentyl-5,6-dihydro-7-ethyl-3-(1-methylcyclohex-1-yl)-9*H*-pyrazolo[3,4-*c*]-1,2,4-triazolo[4,3-α]pyridine;
3-(*tert*-butyl)-9-cyclopentyl-5,6-dihydro-7-ethyl-9*H*-pyrazolo[3,4-*c*]-1,2,4-triazolo[4,3-α]pyridine;
9-cyclopentyl-5,6-dihydro-7-ethyl-3-(2-methylphenyl)-9*H*-pyrazolo[3,4-*c*]-1,2,4-triazolo[4,3-α]pyridine;
9-cyclopentyl-5,6-dihydro-7-ethyl-3-(2-methoxyphenyl)-9*H*-pyrazolo[3,4-*c*]-1,2,4-triazolo[4,3-α]pyridine;
9-cyclopentyl-5,6-dihydro-7-ethyl-3-(thien-2-yl)-9*H*-pyrazolo[3,4-*c*]1,2,4-triazolo[4,3-α]pyridine;
3-(2-chlorophenyl)-9-cyclopentyl-5,6-dihydro-7-ethyl-9*H*-pyrazolo[3,4-*c*]-1,2,4-triazolo[4,3-α]pyridine;
9-cyclopentyl-5,6-dihydro-7-ethyl-3-(2-iodophenyl)-9*H*-pyrazolo[3,4-*c*]-1,2,4-triazolo[4,3-α]pyridine;
9-cyclopentyl-5,6-dihydro-7-ethyl-3-(2-trifluoromethylphenyl)-9*H*-pyrazolo[3,4-*c*]-1,2,4-triazolo[4,3-α]pyridine;
and
5,6-dihydro-7-ethyl-9-(4-fluorophenyl)-3-(1-methylcyclohex-1-yl)-9*H*-pyrazolo[3,4-*c*]-1,2,4-triazolo[4,3-α]pyridine.

**2.** A combination according to Claim 1 wherein said obstructive airways disease is asthma, COPD, or other obstructive airways disease exacerbated by bronchial hyper-reactivity and bronchospasm.

**3.** A combination according to Claim 1 or 2 wherein said PDE4 inhibitor is 9-cyclopentyl-5,6-dihydro-7-ethyl-3-(2-thienyl)-9*H*-pyrazolo[3,4-*c*]-1,2,4-triazolo[4,3-a]pyridine; or 9-cyclopentyl-5,6-dihydro-7-ethyt-3-(tert-butyl)-9*H*-pyrazolo[3,4-*c*]-1,2,4-triazolo(4,3-a]pyridine.

**4.** A combination according to Claim 1, 2 or 3 wherein said anti-cholinergic agent comprising a member selected from the group consisting of tiotropium and derivatives thereof is a compound of Formula (2.1.1):

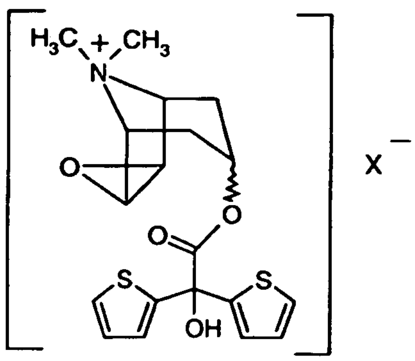

**(2.1.1)**

wherein X is a physiologically acceptable anion.

**5.** A combination according to Claim 4 wherein said physiologically acceptable anion, $X^-$, is a member selected from the group consisting of fluoride, $F^-$; chloride, $Cl^-$; bromide, $Br^-$; iodide, $I^-$; methanesulfonate, $CH_3S(=O)_2O^-$; ethanesulfonate, $CH_3CH_2S(=O)_2O^-$; methylsulfate, $CH_3OS(=O)_2O^-$; benzene sulfonate, $C_6H_5S(=O)_2O^-$; *p*-toluenesulfonate, and $4\text{-}CH_3\text{-}C_6H_5S(=O)_2O^-$.

6. A combination according to Claim 5 wherein said physiologically acceptable anion, $X^-$, is bromide, $Br^-$.

7. A combination according to Claim 4 wherein said anti-cholinergic agent comprising a member selected from the group consisting of tiotropium and derivatives thereof is a 3-α compound.

8. Use of a combination according to one of claims 1 to 7 for the manufacture of a medicament for the treatment of obstructive airways and other inflammatory diseases in a mammal.

9. Use according to Claim 8 wherein said obstructive airways disease is asthma, COPD, or other obstructive airways disease exacerbated by bronchial hyper-reactivity and bronchospasm.

10. Use according to Claim 8 or 9 wherein said mammal is a human being.

11. A pharmaceutical composition suitable for administration by inhalation in the treatment of obstructive airways and other inflammatory diseases in a mammal in need of such treatment, comprising a pharmaceutically acceptable carrier together with a combination of therapeutic agents according to one of claims 1 to 7.

12. A pharmaceutical composition according to Claim 11 wherein said administration by inhalation comprises simultaneous or sequential delivery of said pharmaceutical composition in the form of an aerosol or dry powder dispersion.

**Patentansprüche**

1. Kombination von therapeutischen Mitteln, verwendbar bei der Behandlung obstruktiver Atemwegs- und anderer entzündlicher Erkrankungen, umfassend (I) einen PDE4-Inhibitor, der bei der Behandlung dieser Krankheiten therapeutisch wirksam ist, wenn er durch Inhalation verabreicht wird; zusammen mit (II) einem anti-cholinergen Mittel, umfassend ein Mitglied, ausgewählt aus der Gruppe, bestehend aus Tiotropium und Derivaten hiervon, das bei der Behandlung dieser Krankheiten therapeutisch wirksam ist, wenn es durch Inhalation verabreicht wird,
worin der PDE4-Inhibitor ein Mitglied umfasst, ausgewählt aus der Gruppe, bestehend aus den Folgenden:

   9-Cyclopentyl-5,6-dihydro-7-ethyl-3-phenyl-9H-pyrazolo-[3,4-c]-1,2,4-triazolo-[4,3-α]-pyridin;
   9-Cyclopentyl-5,6-dihydro-7-ethyl-3-(furan-2-yl)-9H-pyrazolo-[3,4-c]-1,2,4-triazolo-[4,3-α]-pyridin;
   9-Cyclopentyl-5,6-dihydro-7-ethyl-3-(2-pyridyl)-9H-pyrazolo-[3,4-c]-1,2,4-triazolo-[4,3-α]-pyridin;
   9-Cyclopentyl-5,6-dihydro-7-ethyl-3-(4-pyridyl)-9H-pyrazolo-[3,4-c]-1,2,4-triazolo-[4,3-α]-pyridin;
   9-Cyclopentyl-5,6-dihydro-7-ethyl-3-(3-thienyl)-9H-pyrazolo-[3,4-c]-1,2,4-triazolo-[4,3-α]-pyridin;
   3-Benzyl-9-cyclopentyl-5,6-dihydro-7-ethyl-9H-pyrazolo-[3,4-c]-1,2,4-triazolo-[4,3-α]-pyridin;
   9-Cyclopentyl-5,6-dihydro-7-ethyl-3-propyl-9H-pyrazolo-[3,4-c]-1,2,4-triazolo-[4,3-α]-pyridin;
   3,9-Dicyclopentyl-5,6-dihydro-7-ethyl-9*H*-pyrazolo-[3,4-c]-1,2,4-triazolo-[4,3-α]-pyridin;
   9-Cyclopentyl-5,6-dihydro-7-ethyl-3-(1-methylcyclohex-1-yl)-9H-pyrazolo-[3,4-c]-1,2,4-triazolo-[4,3-α]-pyridin;
   3-(tert-Butyl)-9-cyclopentyl-5,6-dihydro-7-ethyl-9H-pyrazolo-[3,4-c]-1,2,4-triazolo-[4,3-α]-pyridin;
   9-Cyclopentyl-5,6-dihydro-7-ethyl-3-(2-methylphenyl)-9H-pyrazolo-[3,4-c]-1,2,4-triazolo-[4,3-α]-pyridin;
   9-Cyclopentyl-5,6-dihydro-7-ethyl-3-(2-methoxyphenyl)-9H-pyrazolo-[3,4-c]-1,2,4-triazolo-[4,3-α]-pyridin;
   9-Cyclopentyl-5,6-dihydro-7-ethyl-3-(thien-2-yl)-9H-pyrazolo-[3,4-c]-1,2,4-triazolo-[4,3-α]-pyridin;
   3-(2-Chlorophenyl)-9-cyclopentyl-5,6-dihydro-7-ethyl-9H-pyrazolo-[3,4-c]-1,2,4-triazolo-[4,3-α]-pyridin;
   9-Cyclopentyl-5,6-dihydro-7-ethyl-3-(2-iodophenyl)-9H-pyrazolo-[3,4-c]-1,2,4-triazolo-[4,3-α]-pyridin;
   9-Cyclopentyl-5,6-dihydro-7-ethyl-3-(2-trifluoromethylphenyl)-9H-pyrazolo-[3,4-c]-1,2,4-triazolo-[4,3-α]-pyridin; und
   5,6-Dihydro-7-ethyl-9-(4-fluorophenyl)-3-(1-methylcyclohex-1-yl)-9H-pyrazolo-[3,4-c]-1,2,4-triazolo-[4,3-α]-pyridin.

2. Kombination nach Anspruch 1, worin die obstruktive Atemwegserkrankung Asthma, COPD oder eine andere obstruktive Atemwegserkrankung, die durch bronchiale Hyperraktivität und Bronchialspasmen verschlimmert wird, darstellt.

3. Kombination nach Anspruch 1 oder 2, worin der PDE4-Inhibitor 9-Cyclopentyl-5,6-dihydro-7-ethyl-3-(2-thienyl)-9H-pyrazolo-[3,4-c]-1,2,4-triazolo-[4,3-α]-pyridin; oder 9-Cyclopentyl-5,6-dihydro-7-ethyl-3-(tert-butyl)-9H-pyrazolo-[3,4-c]-1,2,4-triazolo-[4,3-α]-pyridin darstellt.

4. Kombination nach Anspruch 1, 2 oder 3, worin das anti-cholergene Mittel, umfassend ein Mitglied, ausgewählt aus der Gruppe, bestehend aus Tiotropium und Derivaten hiervon, eine Verbindung der Formel (2.1.1) darstellt:

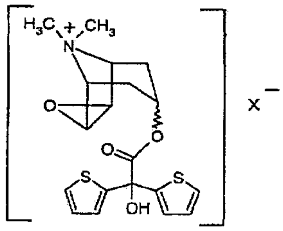

worin $X^-$ ein physiologisch akzeptables Anion darstellt.

5. Kombination nach Anspruch 4, worin das physiologisch akzeptable Anion $X^-$ ein Mitglied ist, ausgewählt aus der Gruppe, bestehend aus Fluorid, $F^-$; Chlorid, $Cl^-$; Bromid, $Br^-$; Iodid, $I^-$; Methansulfonat, $CH_3S(=O)_2O^-$; Ethansulfonat, $CH_3CH_2S(=O)_2O^-$; Methylsulfat, $CH_3OS(=O)_2O^-$; Benzolsulfonat, $C_6H_5S(=O)_2O^-$; p-Toluolsulfonat und 4-$CH_3$-$C_6H_5S(=O)_2O^-$.

6. Kombination nach Anspruch 5, worin das physiologisch akzeptable Anion $X^-$ Bromid $Br^-$ darstellt.

7. Kombination nach Anspruch 4, worin das anti-cholergene Mittel, umfassend ein Mitglied, ausgewählt aus der Gruppe, bestehend aus Tiotropium und Derivaten hiervon, eine 3-α-Verbindung darstellt.

8. Verwendung einer Kombination nach irgendeinem der Ansprüche 1 bis 7 zur Herstellung eines Medikaments zur Behandlung obstruktiver Atemwegs- und anderer entzündlicher Erkrankungen bei einem Säuger.

9. Verwendung nach Anspruch 8, worin die obstruktive Atemwegserkrankung Asthma, COPD oder eine andere obstruktive Atemwegserkrankung, die durch bronchiale Hyperreaktivität und Bronchialspasmen verschlimmert wird, darstellt.

10. Verwendung nach Anspruch 8 oder 9, worin der Säuger einen Menschen darstellt.

11. Pharmazeutische Zusammensetzung, geeignet für die Verabreichung durch Inhalation bei der Behandlung von obstruktiven Atemwegs- und anderen entzündlichen Erkrankungen bei einem Säuger, der eine solche Behandlung benötigt, umfassend einen pharmazeutisch akzeptablen Träger, zusammen mit einer Kombination therapeutischer Mittel nach irgendeinem der Ansprüche 1 bis 7.

12. Pharmazeutische Zusammensetzung nach Anspruch 11, worin die Verabreichung durch Inhalation die gleichzeitige oder sequentielle zur Verfügungstellung der pharmazeutischen Zusammensetzung in Form eines Aerosols oder einer trockenen Pulverdispersion umfasst.

## Revendications

1. Combinaison d'agents thérapeutiques utile dans le traitement des maladies des voies respiratoires obstructives et d'autres maladies inflammatoires comprenant *(I)* un inhibiteur de PDE4 qui est thérapeutiquement efficace dans le traitement desdites maladies quand il est administré par inhalation; en même temps que *(II)* un agent anti-cholinergique comprenant un membre choisi dans le groupe consistant en le tiotropium et ses dérivés qui est thérapeutiquement efficace dans le traitement desdites maladies quand il est administré par inhalation,
où l'inhibiteur de PDE4 comprend un membre choisi dans le groupe consistant en ce qui suit :

la 9-cyclopentyl-5,6-dihydro-7-éthyl-3-phényl-9*H*-pyrazolo[3,4-c]-1,2,4-triazolo[4,3-α]pyridine ;
la 9-cyclopentyl-5,6-dihydro-7-éthyl-3-(furan-2-yl)-9*H*-pyrazolo[3,4-c]-1,2,4-triazolo[4,3-α]pyridine ;
la 9-cyclopentyl-5,6-dihydro-7-éthyl-3-(2-pyridyl)-9*H*-pyrazolo[3,4-c]-1,2,4-triazolo[4,3-α]pyridine ;
la 9-cyclopentyl-5,6-dihydro-7-éthyl-3-(4-pyridyl)-9*H*-pyrazolo[3,4-c]-1,2,4-triazolo[4,3-α]pyridine ;
la 9-cyclopentyl-5,6-dihydro-7-éthyl-3-(3-thiényl)-9*H*-pyrazolo[3,4-c]-1,2,4-triazolo[4,3-α]pyridine ;

la 3-benzyl-9-cyclopentyl-5,6-dihydro-7-éthyl-9*H*-pyrazolo[3,4-c]-1,2,4-triazolo[4,3-α]pyridine ;
la 9-cyclopentyl-5,6-dihydro-7-éthyl-3-propyl-9*H*-pyrazolo[3,4-c]-1,2,4-triazolo[4,3-α]pyridine ;
la 3,9-dicyclopentyl-5,6-dihydro-7-éthyl-9*H*-pyrazolo[3,4-c]-1,2,4-triazolo[4,3-α]pyridine ;
la 9-cyclopentyl-5,6-dihydro-7-éthyl-3-(1-méthylcyclohex-1-yl)-9*H*-pyrazolo[3,4-c]-1,2,4-triazolo[4,3-α]pyridine ;
la 3-(*tert*-butyl)-9-cyclopentyl-5,6-dihydro-7-éthyl-9*H*-pyrazolo[3,4-c]-1,2,4-triazolo[4,3-α]pyridine ;
la 9-cyclopentyl-5,6-dihydro-7-éthyl-3-(2-méthylphényl)-9*H*-pyrazolo[3,4-c]-1,2,4-triazolo[4,3-α]pyridine ;
la 9-cyclopentyl-5,6-dihydro-7-éthyl-3-(2-méthoxyphényl)-9*H*-pyrazolo[3,4-c]-1,2,4-triazolo[4,3-α]pyridine ;
la 9-cyclopentyl-5,6-dihydro-7-éthyl-3-(thién-2-yl)-9*H*-pyrazolo[3,4-c]-1,2,4-triazolo[4,3-α]pyridine ;
la 3-(2-chlorophényl)-9-cyclopentyl-5,6-dihydro-7-éthyl-9*H* pyrazolo[3,4-c]-1,2,4-triazolo[4,3-α]pyridine ;
la 9-cyclopentyl-5,6-dihydro-7-éthyl-3-(2-iodophényl)-9*H* pyrazolo[3,4-c]-1,2,4-triazolo[4,3-α]pyridine ;
la 9-cyclopentyl-5,6-dihydro-7-éthyl-3-(2-trifluorométhylphényl)-9*H* pyrazolo[3,4-c]-1,2,4-triazolo[4,3-α]pyridine ; et
la 5,6-dihydro-7-éthyl-9-(4-fluorophényl)-3-(1-méthylcyclohex-1-yl)-9*H*-pyrazolo[3,4-c]-1,2,4-triazolo[4,3-α]pyridine.

**2.** Combinaison selon la revendication 1 où ladite maladie des voies respiratoires obstructive est l'asthme, la COPD ou une autre maladie des voies respiratoires obstructive exacerbée par une hyper-réactivité bronchique et un bronchospasme.

**3.** Combinaison selon la revendication 1 ou 2 où ledit inhibiteur de PDE4 est la 9-cyclopentyl-5,6-dihydro-7-éthyl-3-(2-thiényl)-9*H*-pyrazolo[3,4-c]-1,2,4-triazolo[4,3-α]pyridine ; ou la 9-cyclopentyl-5,6-dihydro-7-éthyl-3-(*tert*-butyl)-9*H*-pyrazolo[3,4-c]-1,2,4-triazolo[4,3-α]pyridine.

**4.** Combinaison selon la revendication 1, 2 ou 3 où ledit agent anti-cholinergique comprenant un membre choisi dans le groupe consistant en le tiotropium et ses dérivés est un composé de formule (2.1.1) :

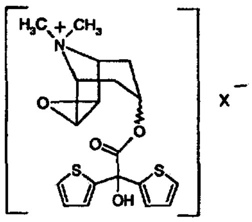

où $X^-$ est un anion physiologiquement acceptable.

**5.** Combinaison selon la revendication 4 où ledit anion physiologiquement acceptable, $X^-$, est un membre choisi dans le groupe consistant en fluorure, $F^-$ ; chlorure, $Cl^-$ ; bromure, $Br^-$ ; iodure, $I^-$ ; méthanesulfonate, $CH_3S(=O)_2O^-$ ; éthanesulfonate, $CH_3CH_2S(=O)_2O^-$ ; méthylsulfate, $CH_3OS(=O)_2O^-$; benzènesulfonate, $C_6H_5S(=O)_2O^-$; p-toluènesulfonate, et $4\text{-}CH_3\text{-}C_6H_5S(=O)_2O^-$.

**6.** Combinaison selon la revendication 5 où ledit anion physiologiquement acceptable, $X^-$, est bromure, $Br^-$.

**7.** Combinaison selon la revendication 4 où ledit agent anti-cholinergique comprenant un membre choisi dans le groupe consistant en le tiotropium et ses dérivés est un composé 3-α.

**8.** Utilisation d'une combinaison selon l'une des revendications 1 à 7 pour la fabrication d'un médicament pour le traitement des maladies des voies respiratoires obstructives et d'autres maladies inflammatoires chez un mammifère.

**9.** Utilisation selon la revendication 8 où ladite maladie des voies respiratoires obstructive est l'asthme, la COPD ou une autre maladie des voies respiratoires obstructive exacerbée par une hyper-réactivité bronchique et un bronchospasme.

**10.** Utilisation selon la revendication 8 ou 9 où ledit mammifère est un être humain.

**11.** Composition pharmaceutique appropriée à l'administration par inhalation dans le traitement des maladies des voies

respiratoires obstructives et d'autres maladies inflammatoires chez un mammifère nécessitant un tel traitement, comprenant un support pharmaceutiquement acceptable ainsi qu'une combinaison d'agents thérapeutiques selon l'une des revendications 1 à 7.

**12.** Composition pharmaceutique selon la revendication 11 où ladite administration par inhalation comprend la délivrance simultanée ou successive de ladite composition pharmaceutique sous forme d'un aérosol ou d'une dispersion de poudre sèche.